(19)

**Europäisches Patentamt**
**European Patent Office**
**Office européen des brevets**

(11)  **EP 3 663 758 A1**

(12)  **EUROPEAN PATENT APPLICATION**

(43) Date of publication:
**10.06.2020   Bulletin 2020/24**

(51) Int Cl.:
**G01N 33/48** *(2006.01)*      **G01N 33/68** *(2006.01)*
**G01N 30/88** *(2006.01)*      **G16B 20/00** *(2019.01)*
**G16H 50/30** *(2018.01)*

(21) Application number: **20152640.7**

(22) Date of filing: **18.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **18.03.2012   JP 2012061305**

(62) Document number(s) of the earlier application(s) in accordance with Art. 76 EPC:
**13764636.0 / 2 829 877**

(71) Applicant: **Shiseido Company, Ltd.**
**Chuo-ku**
**Tokyo 104-0061 (JP)**

(72) Inventors:
• **HAMASE, Kenji**
**Fukuoka, Fukuoka 819-0395 (JP)**

• **TOUJO, Yousuke**
**Tokyo, Tokyo 1040061 (JP)**
• **MITA, Masashi**
**Tokyo, Tokyo 1040061 (JP)**
• **MIZUMOTO, Chieko**
**Tokyo, Tokyo 1040061 (JP)**

(74) Representative: **Engelhard, Markus**
**Boehmert & Boehmert**
**Anwaltspartnerschaft mbB**
**Pettenkoferstrasse 22**
**80336 München (DE)**

Remarks:
This application was filed on 20-01-2020 as a divisional application to the application mentioned under INID code 62.

(54)  **APPARATUS, SYSTEM AND METHOD FOR ANALYZING DISEASE SAMPLE**

(57)      [Problem] To elucidate a correlation between a disease and the amount of amino acid stereoisomers or the change in the amount by carrying out a total analysis of amino acid stereoisomers, leading to development of a novel method for diagnosing a disease, and a novel apparatus for diagnosing a disease in which the method for diagnosing a disease is executed.

[Means for Resolution] The apparatus for analyzing a disease sample according to the present invention includes: a member for separating and quantitatively determining an amino acid stereoisomer in a biological material from a subject; a member for obtaining a disease state index value through a calculation by substituting the amount of the amino acid stereoisomer into a discriminant equation; and a member for outputting disease state information on the subject on the basis of the disease state index value. The discriminant equation may be: disease state index value = (a measurement value for an amino acid stereoisomer that correlates with the disease from among the measurement values in a biological material from the subject)/ (in a biological material from a reference value from a healthy individual), or the like. The method for analyzing a disease sample according to the present invention includes: a step of measuring the amount of amino acid stereoisomers in a biological material from a subject; a step of obtaining a disease state index value through a calculation by substituting the amount of the amino acid stereoisomers into a discriminant equation; and the like.

EP 3 663 758 A1

**Description**

TECHNICAL FIELD

The present invention relates to an apparatus, a system and a method for analyzing a disease sample, on the basis of a quantitative determination method that discriminates stereoisomers of an amino acid. Specifically, the present invention relates to an apparatus for analyzing a disease sample, the apparatus including: a member for separating and quantitatively determining amino acid stereoisomers in a biological material from a subject; a member for obtaining a disease state index value through a calculation by substituting a quantitatively determined value of the amino acid stereoisomer into a discriminant equation; and a member for outputting disease state information on the subject on the basis of the disease state index value. Further, the present invention also relates to a system for analyzing a disease sample, the system including: a quantitative determination analysis unit for separating and quantitatively determining an amino acid stereoisomer in a biological material from a subject; a disease state index value computation unit for obtaining a disease state index value through a calculation by substituting a quantitatively determined value of the amino acid stereoisomer into a discriminant equation; and a disease state information output unit for outputting disease state information on the subject on the basis of the disease state index value. Additionally, the present invention further relates to a method for analyzing a disease sample, the method including: a step of separating and quantitatively determining an amino acid stereoisomer in a biological material from a subject; a step of obtaining a disease state index value through a calculation by substituting a quantitatively determined value of the amino acid stereoisomer into a discriminant equation; and a step of obtaining disease state information on the subject on the basis of the disease state index value.

BACKGROUND ART

There are two types of stereoisomers, i.e., D-form and L-form, of all amino acids other than glycine. L-amino acids are constitutive elements of proteins in organisms, and thus amino acids contained in proteins are L-amino acids in principle. In contrast, D-amino acids are contained in a part of bioactive peptides in lower organisms, and many of these are biosynthesized via a posttranslational modification process. Accordingly, amino acids that constitute proteins or peptides are predominantly L-amino acids, whereas D-amino acids are exceptionally present.

D-amino acids are one of constitutive components of peptide glycan of bacterial cell walls. In addition, with regard to free D-amino acids that do not constitute peptides, the presence thereof in lower animals such as aquatic animals and insects has been conventionally reported. However, it was believed in some past times that amino acids are present in their L-form in higher animals, without an involvement of D-forms in the physiological activities (Nonpatent Document 1).

However, the presence and roles of D-amino acids in mammals including humans have been managed to be elucidated in accordance with improvements of a resolution ability and sensitivity owing to the advance of analytical techniques in recent years (Nonpatent Document 2). D-aspartic acid was proven to be localized in prolactin-producing cells in rat pituitary gland by means of a double staining process and the like carried out using an anti-D-aspartic acid antibody. Also, addition of D-aspartic acid to cells that synthesize and secrete prolactin, a cell strain derived from rat pituitary gland, resulted in an increase of the secretion of prolactin in a dose-dependent manner. From these findings, it is believed that D-aspartic acid controls the secretion of prolactin in prolactin-producing cells (Nonpatent Document 3).

On the other hand, it was reported that D-aspartic acid is detected in rat testicular vein at a constantly higher concentration than that in other venous blood, and further that addition of D-aspartic acid to Leydig cells isolated and purified from rat testis promotes the synthesis and secretion of testosterone in a dose-dependent manner (Nonpatent Document 4).

It is reported that D-serine selectively stimulates a glycine binding site of an NMDA type glutamic acid receptor which is presumed to involve in schizophrenia, and promotes neurotransmission through enhancing an action of glutamic acid that is operated via this receptor (Nonpatent Document 5). In fact, it was reported that administration with D-serine ameliorates schizophrenia, and that the concentration of D-serine in sera from schizophrenia patients is lower than that from healthy individuals. Moreover, it was also reported recently that D-serine involves in degeneration of motor nerve in amyotrophic lateral sclerosis (ALS) (Nonpatent Document 6).

Zaitsu et al., developed a simultaneous and highly sensitive analysis system of D- and L-amino acids (Patent Document 1, Nonpatent Documents 7 to 9) to put a simultaneous and highly sensitive analytical technique of amino acid stereoisomers into a practical application. Accordingly, it has become possible to make comprehensive separation and quantitative determination of D-amino acids present in a trace amount and L-amino acids present in a comparatively large amount both contained in a human biological material from an identical sample.

In connection with the present invention, it was found from results of quantitative determination analyses on amino acid stereoisomers from various types of disease patients, that the amounts of D-amino acids and L-amino acids in biological materials from healthy individuals are maintained at a constant balance, with small individual differences, and that there is a disturbance in balance, in some amino acids, that cannot be ascertained unless D-amino acids and L-amino acids in biological materials from patients are separated and quantitatively determined. The present invention was accomplished in view of such unexpected findings.

PRIOR ART DOCUMENTS

PATENT DOCUMENTS

Patent Document 1: Japanese Patent No. 4291628

NONPATENT DOCUMENTS

Nonpatent Document 1: Corrigan J.J., Science 164: 142 (1969)
Nonpatent Document 2: Hamase K, Morikawa A, and Zaitsu K., J Chromatogr. B 781: 73 (2002)
Nonpatent Document 3: D'Aniello A et el., FASEB J 14: 699 (2000)
Nonpatent Document 4: Nagata Y et el., FEBS Lett. 444: 160 (1999)
Nonpatent Document 5: Nishikawa T, Biol. Pharm. Bull. 28: 1561 (2005)
Nonpatent Document 6: Sasabe, J., et el., Proc. Natl. Acad. Sci. 109: 627 (2012)
Nonpatent Document 7: Hamase K., et el., J. Chromatogr. A, 1143: 105 (2007)
Nonpatent Document 8: Hamase K., et el., J. Chromatogr. A, 1217: 1056 (2010)
Nonpatent Document 9: Miyoshi Y, et el., J. Chromatogr. B, 879: 3184 (2011)

DISCLOSURE OF THE INVENTION

Problems to be Solved by the Invention

There is a need for a development of a novel method for diagnosing a disease by a total analysis of amino acid stereoisomers to elucidate a correlation between a disease and the amount of an amino acid stereoisomer or the change in the amount thereof, as well as a novel apparatus for diagnosing a disease in which the method for diagnosing a disease is executed.

Means for Solving the Problems

According to an aspect of the present invention, an apparatus for analyzing a disease sample is provided. The apparatus for analyzing a disease sample according to the aspect of the present invention includes a member for separating and quantitatively determining an amino acid stereoisomer in a biological material from a subject, a member for obtaining a disease state index value through a calculation by substituting the amount of the amino acid stereoisomer into a discriminant equation, and a member for outputting disease state information on the subject on the basis of the disease state index value.

In the apparatus for analyzing a disease sample according to the aspect of the present invention, the discriminant equation may be either:

$$\text{disease state index value} = (\text{a measurement value for an amino acid stereoisomer that correlates with the disease from among the measurement values in a biological material from the subject})/ (\text{a reference value for the amino acid stereoisomer that correlates with the disease in a biological material from a healthy individual});$$

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

In the apparatus for analyzing a disease sample according to the aspect of the present invention, the member for outputting disease state information on the subject on the basis of the disease state index value may be a member for outputting disease state information on the subject that the subject is suffering from the disease when the disease state index value is 2.0 or greater.

In the apparatus for analyzing a disease sample according to the aspect of the present invention, the amino acid stereoisomer that correlates with the disease may be: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine in the case of DAO deficiency; D-asparagine, D-aspartic acid and D-arginine in the case of DDO deficiency; L-phenylalanine in the case of phenylketonuria; L-valine, L-allo-isoleucine, D-isoleucine, L-isoleucine and L-leucine in the case of a maple syrup urine disease; L-glutamic acid, L-glutamine and L-cysteine in the case of articular rheumatism; D-serine and D-alanine in the case of kidney cancer; D-alanine in the case of lung cancer; L-arginine and L-glutamic acid in the case of a cardiovascular disease; D-serine and L-cysteine in the case of multiple sclerosis; L-cysteine in the case of acute myeloid leukemia; L-cysteine in the case of lymphoma; L-glutamic acid and L-cysteine in the case of acute lymphocytic leukemia; L-arginine and L-cysteine in the case of psoriasis; or D-alanine, L-cysteine and L-glutamic acid in the case of diabetes.

According to another aspect of the present invention, a system for analyzing a disease sample is provided. The system for analyzing a disease sample according to the another aspect of the present invention includes: a quantitative determination analysis unit for separating and quantitatively determining an amino acid stereoisomer in a biological material from a subject; a disease state index value computation unit for obtaining a disease state index value through a calculation by substituting the amount of the amino acid stereoisomer into a discriminant equation; and a disease state information output unit for outputting disease state information on the subject on the basis of the disease state index value.

In the system for analyzing a disease sample according to the another aspect of the present invention, the discriminant equation may be either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

In the system for analyzing a disease sample according to the another aspect of the present invention, the disease state information output unit may output disease state information on the subject that the subject is suffering from the disease when the disease state index value is 2.0 or greater.

In the system for analyzing a disease sample according to the another aspect of the present invention, the amino acid stereoisomer that correlates with the disease may be: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine in the case of DAO deficiency; D-asparagine, D-aspartic acid and D-arginine in the case of DDO deficiency; L-phenylalanine in the case of phenylketonuria; L-valine, L-allo-isoleucine, D-isoleucine, L-isoleucine and L-leucine in the case of a maple syrup urine disease; L-glutamic acid, L-glutamine and L-cysteine in the case of articular rheumatism; D-serine and D-alanine in the case of kidney cancer; D-alanine in the case of lung cancer; L-arginine and L-glutamic acid in the case of a cardiovascular disease; D-serine and L-cysteine in the case of multiple sclerosis; L-cysteine in the case of acute myeloid leukemia; L-cysteine in the case of lymphoma; L-glutamic acid and L-cysteine in the case of acute lymphocytic leukemia; L-arginine and L-cysteine in the case of psoriasis; or D-alanine, L-cysteine and L-glutamic acid in the case of diabetes.

According to still another aspect of the present invention, a method for analyzing a disease sample is provided. The method for analyzing a disease sample according to the still another aspect of the present invention includes: a step of measuring the amount of an amino acid stereoisomer in a biological material from a subject; a step of obtaining a disease state index value through a calculation by substituting the amount of the amino acid stereoisomer into a discriminant equation; and a step of outputting disease state information on the subject on the basis of the disease state index value.

In the method for analyzing a disease sample according to the still another aspect of the present invention, the discriminant equation may be either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

In the method for analyzing a disease sample according to the still another aspect of the present invention, the step of outputting disease state information on the subject on the basis of the disease state index value may be a step of outputting disease state information on the subject that the subject is suffering from the disease when the disease state index value is 2.0 or greater.

In the method for analyzing a disease sample according to the still another aspect of the present invention, the amino acid stereoisomer that correlates with the disease may be: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine in the case of DAO deficiency; D-asparagine, D-aspartic acid and D-arginine in the case of DDO deficiency; L-phenylalanine in the case of phenylketonuria; L-valine, L-allo-isoleucine, D-isoleucine, L-isoleucine and L-leucine in the case of a maple syrup urine disease; L-glutamic acid, L-glutamine and L-cysteine in the case of articular rheumatism; D-serine and D-alanine in the case of kidney cancer; D-alanine in the case of lung cancer; L-arginine and L-glutamic acid in the case of a cardiovascular disease; D-serine and L-cysteine in the case of multiple sclerosis; L-cysteine in the case of acute myeloid leukemia; L-cysteine in the case of lymphoma; L-glutamic acid and L-cysteine in the case of acute lymphocytic leukemia; L-arginine and L-cysteine in the case of psoriasis; or D-alanine, L-cysteine and L-glutamic acid in the case of diabetes.

According to yet another aspect of the present invention, a method for diagnosing a disease is provided. The method for diagnosing a disease according to the yet another aspect of the present invention includes: a step of measuring the amount of an amino acid stereoisomer in a biological material from a subject; and a step of diagnosing the disease on the basis of a measurement value of the amount of the amino acid stereoisomer and a reference value from a healthy individual.

According to yet another aspect of the present invention, a method for diagnosing a disease is provided. The method for diagnosing a disease according to the yet another aspect of the present invention includes: a step of separating and quantitatively determining an amino acid stereoisomer in a biological material from a subject; a step of obtaining a disease state index value through a calculation by substituting the amount of the amino acid stereoisomer into a discriminant equation; and a step of diagnosing the subject on the basis of the disease state index value.

In the method for diagnosing a disease according to the yet another aspect of the present invention, the discriminant equation may be either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

In the method for diagnosing a disease according to the yet another aspect of the present invention, the step of outputting disease state information on the subject on the basis of the disease state index value may be a step of diagnosing that the subject is suffering from the disease when the disease state index value is 2.0 or greater.

In the method for diagnosing a disease according to the yet another aspect of the present invention, the amino acid stereoisomer that correlates with the disease may be: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; or D-asparagine when the disease is osteoporosis.

The amino acid stereoisomer as referred to herein involves: 20 types of amino acids used in translation of proteins; 19 types of D-amino acids that are optical isomers of 19 types of L-amino acids, which are amino acids other than glycine; and allo-L-threonine, allo-D-threonine and allo-D-isoleucine.

The apparatus for analyzing a disease sample according to the aspect of the present invention includes: a member for measuring the amount of an amino acid stereoisomer in a biological material; a member for obtaining a disease state index value through a calculation by substituting the amount of the amino acid stereoisomer into a discriminant equation; and a member for outputting disease state information on the subject on the basis of the disease state index value. The member for measuring the amount of an amino acid stereoisomer in the biological material includes an automatic sample fractionation unit, and an HPLC separation and peak detection unit by way of a reverse phase column or the like. The member for obtaining an disease state index value through a calculation by substituting the amount of the amino acid stereoisomer into a discriminant equation includes: a memory unit for storing data such as the discriminant equation, reference values from healthy individuals for each of the disease, etc., and a computation unit for executing the calculation with the discriminant equation on the basis of the data. The member for outputting disease state information on the subject on the basis of the disease state index value includes: a disease state information selection unit; and a disease state information output unit. In addition thereto, the apparatus for analyzing a disease sample according to the aspect of the present invention may include: a control unit such as CPU for totally controlling the entire apparatus; an input and output interface unit for connecting an input device and an output device, and a communication interface unit for communicably connecting to a network.

The biological material as referred to herein involves: body fluids such as blood, plasma, serum, ascites, amniotic fluid, lymph fluid, saliva, seminal fluid and urine; excrement such as faeces, sweat and nasal discharge; and body tissues such as body hair, nail, skin tissues and visceral organ tissues, but not limited thereto.

The discriminant equation as referred to herein may involve a formula that calculates as to what times the measurement value of the amount of the amino acid stereoisomer in the subject is with respect to the reference value predetermined on the basis of a measurement value from a healthy individual. Also, the discriminant equation may involve a formula that calculates a proportion or a percentage of the amount of the amino acid stereoisomer with respect to a sum of the amount of the amino acid stereoisomer and the amount of enantiomer of the isomer. Furthermore, the discriminant equation may involve a formula that calculates a disease state index value from a combination of amounts of a plurality of types of amino acid stereoisomers. The plurality of amino acid stereoisomers may be a group of amino acids having a common feature of serving as a substrate for D-amino acid oxidase or D-aspartic acid oxidase, and the like. For example, among amino acid stereoisomers that serve as substrates of an identical enzyme, the amount of the amino acids correlating to a disease may be normalized with the amount of the amino acids not correlating to the disease.

In the discriminant equation according to the aspect of the present invention, a reference value for the amino acid stereoisomer that correlates with a disease in a biological material from a healthy individual is determined from an average or a median of the amounts of the amino acid stereoisomer that correlates with the disease in either one or both of biological materials from among a biological material from a healthy individual, and a biological material from other disease patient not correlating with the amino acid stereoisomer. Although the reference value may be predetermined, a case in which the reference value is a measurement value, or an average or a median thereof from a biological material prepared for and concomitantly tested on a control experiment in putting the present invention into practice is also acceptable.

As the disease state information according to the aspect of the present invention, outputting that "the subject is a healthy individual" is executed when the disease state index value calculated by the discriminant equation according to the aspect of the present invention is 1.0 or approximate thereto. When the disease state index value is 2.0 or greater, outputting that "the subject is highly probable of being suffering from the disease" may be executed. However, even when the disease state index value is less than 2.0, outputting that "the subject is probable of being suffering from the disease" may be executed.

The data on quantitative determination of the amino acid stereoisomer in a biological material from a subject obtained by the apparatus for analyzing a disease sample, the analysis system and the analysis method according to the aspects of the present invention can be used as an indicator for diagnoses and prophylaxes of a variety of diseases. In addition, the quantitative data can be used as an indicator for progression of the medical condition of the disease. Furthermore, the quantitative data may be used as an indicator for deciding the effectiveness of a medical drug for a treatment and/or prophylaxis of the disease. In addition, the quantitative data may be used as an indicator for deciding an influence of medical drugs, quasi-drugs, cosmetics, food and other chemical substances on living bodies, as well as influence of other physical and/or biological environmental factors on living bodies.

The entire contents of all documents referred to herein are incorporated herein by reference in their entirety.

BRIEF DESCRIPTION OF THE DRAWINGS

FIG. 1 shows a Table summarizing analysis results of D-amino acids on each initial sample from a healthy individual and various types of disease patients;
FIG. 2-1 shows a distribution chart presenting serum concentrations of D-serine in each disease sample;
FIG. 2-2 shows a distribution chart presenting serum concentrations of L-serine in each disease sample;

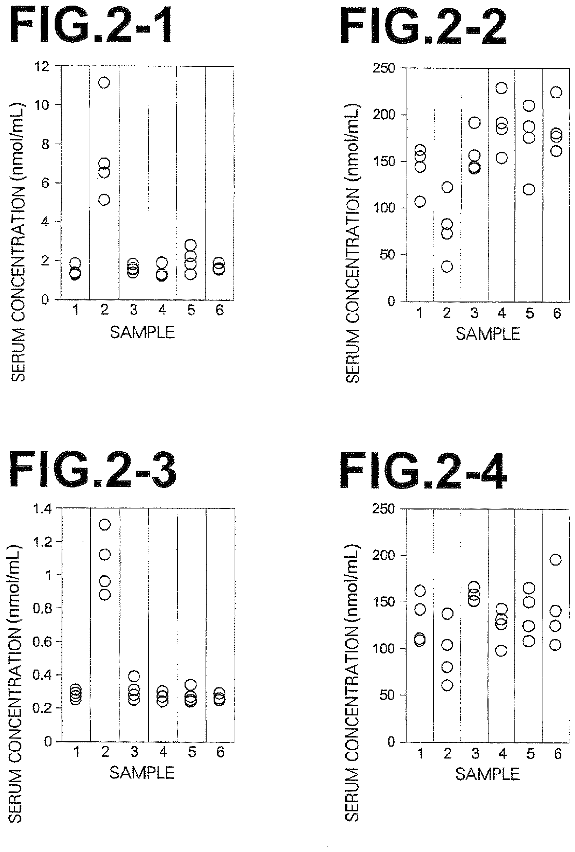

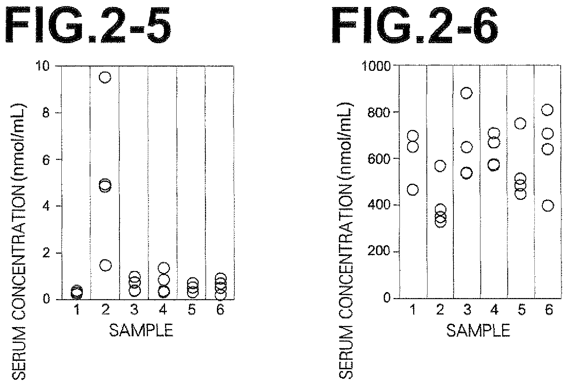

FIG. 2-3 shows a distribution chart presenting serum concentrations of D-threonine in each disease sample;
FIG. 2-4 shows a distribution chart presenting serum concentrations of L-threonine in each disease sample;
FIG. 2-5 shows a distribution chart presenting serum concentrations of D-alanine in each disease sample;
FIG. 2-6 shows a distribution chart presenting serum concentrations of L-alanine in each disease sample;

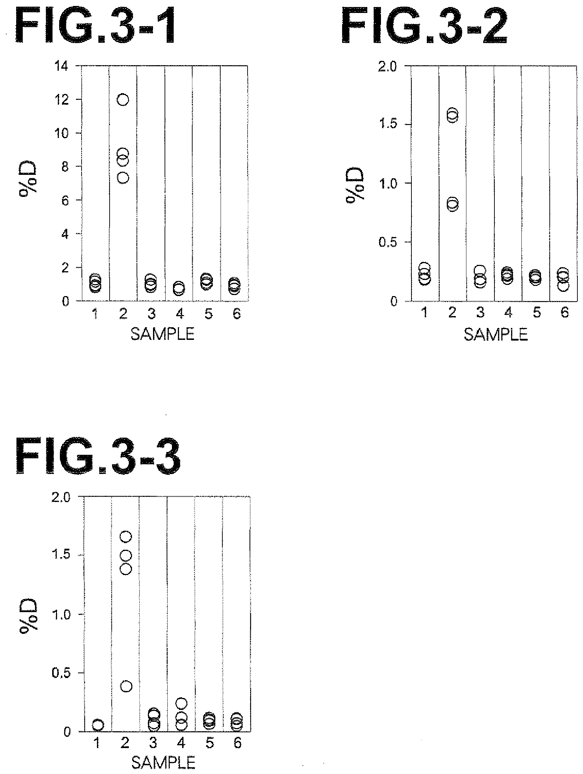

FIG. 3-1 shows a distribution chart presenting percentages of the amount of the D-form of serine (%D) with respect to the sum of the amounts of the D-form and L-form of serine in samples;
FIG. 3-2 shows a distribution chart presenting percentages of the amount of the D-form of threonine (%D) with respect to the sum of the amounts of the D-form and L-form of threonine in samples;
FIG. 3-3 shows a distribution chart presenting percentages of the amount of the D-form of alanine (%D) with respect to the sum of the amounts of the D-form and L-form of alanine in samples;

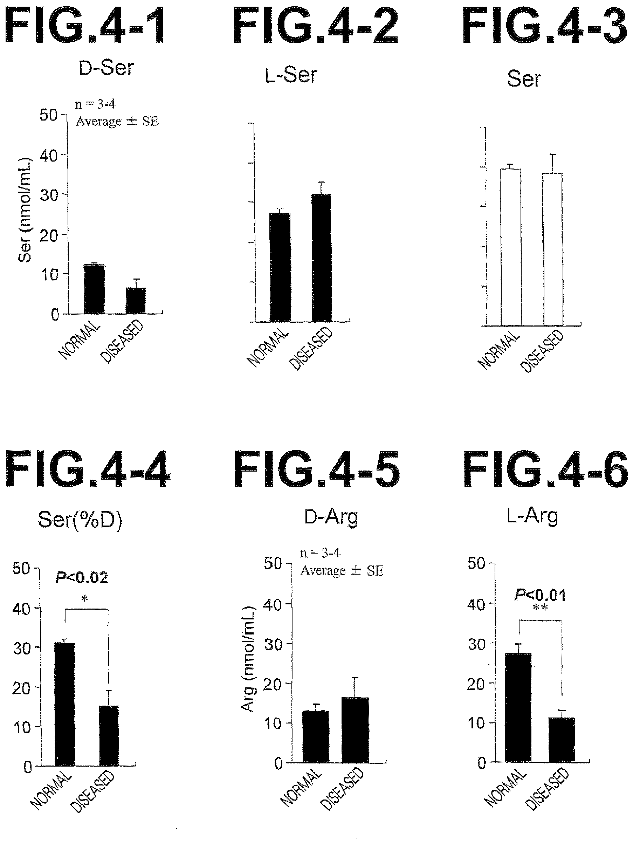

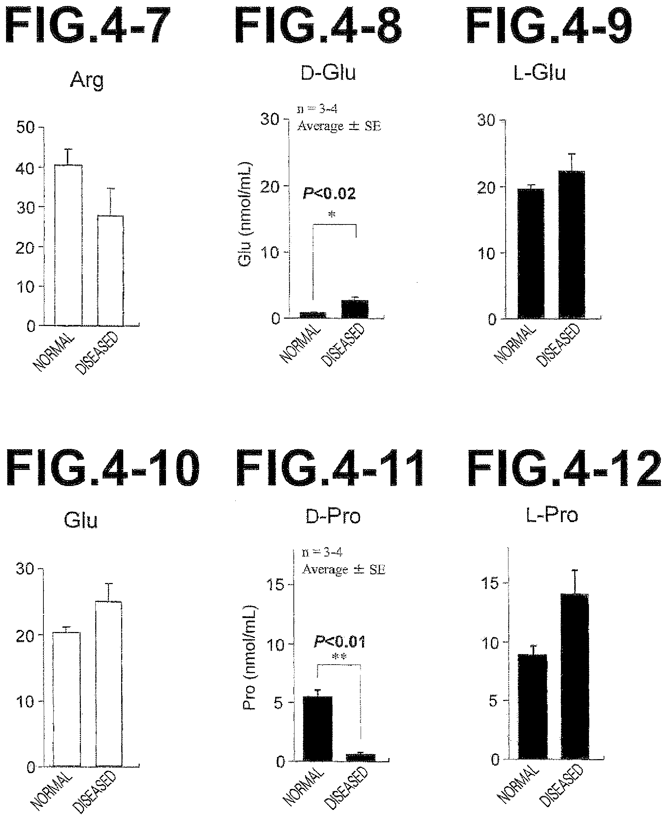

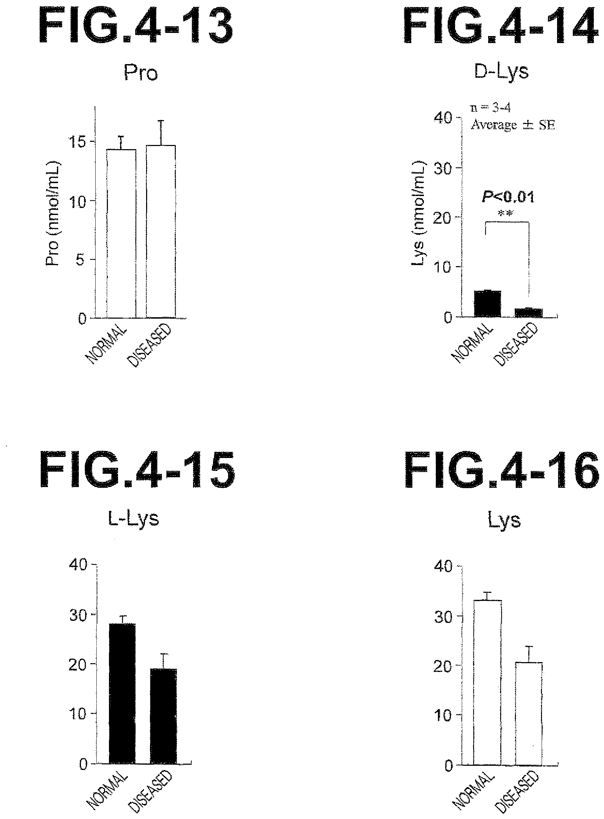

FIG. 4-1 shows a bar chart presenting averages and standard errors of D-serine concentrations in the urine from three dilated cardiomyopathy model mice (MLP-KO mouse, hereinafter, may be referred to as "diseased") and four control normal mice (hereinafter, may be referred to as "normal"). The ordinate indicates the D-serine concentration (nanomol/mL);
FIG. 4-2 shows a bar chart presenting averages and standard errors of L-serine concentrations in the urine from three dilated cardiomyopathy model mice (hereinafter, may be referred to as "diseased") and four control normal mice (hereinafter, may be referred to as "normal"). The ordinate indicates the L-serine concentration (nanomol/mL);

FIG. 4-3 shows a bar chart presenting averages and standard errors of total serine concentrations (the sums of D-serine concentration and L-serine concentration) in the urine from three dilated cardiomyopathy model mice (hereinafter, may be referred to as "diseased") and four control normal mice (hereinafter, may be referred to as "normal"). The ordinate indicates the total serine concentration (nanomol/mL);

FIG. 4-4 shows a bar chart presenting averages and standard errors of the percentages of D-serine concentration (%D) with respect to the total serine concentration in the urine from three dilated cardiomyopathy model mice (hereinafter, may be referred to as "diseased") and four control normal mice (hereinafter, may be referred to as "normal"). The ordinate indicates the %D. In regard to the significant difference between the normal and the diseased, $P$ was less than 0.02 according to a Student's t-test;

FIG. 4-5 shows a bar chart presenting averages and standard errors of D-arginine concentrations in the urine from three dilated cardiomyopathy model mice (hereinafter, may be referred to as "diseased") and four control normal mice (hereinafter, may be referred to as "normal"). The ordinate indicates the D-arginine concentration (nanomol/mL);

FIG. 4-6 shows a bar chart presenting averages and standard errors of L-arginine concentrations in the urine from three dilated cardiomyopathy model mice (hereinafter, may be referred to as "diseased") and four control normal mice (hereinafter, may be referred to as "normal"). The ordinate indicates the L-arginine concentration (nanomol/mL). In regard to the significant difference between the normal and the diseased, $P$ was less than 0.01 according to a Student's t-test;

FIG. 4-7 shows a bar chart presenting averages and standard errors of total arginine concentrations (the sums of D-arginine concentration and L-arginine concentration) in the urine from three dilated cardiomyopathy model mice (hereinafter, may be referred to as "diseased") and four control normal mice (hereinafter, may be referred to as "normal"). The ordinate indicates the total arginine concentration (nanomol/mL);

FIG. 4-8 shows a bar chart presenting averages and standard errors of D-glutamic acid concentrations in the urine from three dilated cardiomyopathy model mice (hereinafter, may be referred to as "diseased") and four control normal mice (hereinafter, may be referred to as "normal"). The ordinate indicates the D-glutamic acid concentration (nanomol/mL). In regard to the significant difference between the normal and the diseased, $P$ was less than 0.02 according to a Student's t-test;

FIG. 4-9 shows a bar chart presenting averages and standard errors of the L-glutamic acid concentrations in the urine from three dilated cardiomyopathy model mice (hereinafter, may be referred to as "diseased") and four control normal mice (hereinafter, may be referred to as "normal"). The ordinate indicates the L-glutamic acid concentration (nanomol/mL);

FIG. 4-10 shows a bar chart presenting averages and standard errors of total glutamic acid concentrations (the sums of D-glutamic acid concentration and L-glutamic acid concentration) in the urine from three dilated cardiomyopathy model mice (hereinafter, may be referred to as "diseased") and four control normal mice (hereinafter, may be referred to as "normal"). The ordinate indicates the total glutamic acid concentration (nanomol/mL);

FIG. 4-11 shows a bar chart presenting averages and standard errors of D-proline concentrations in the urine from three dilated cardiomyopathy model mice (hereinafter, may be referred to as "diseased") and four control normal mice (hereinafter, may be referred to as "normal"). The ordinate indicates the D-proline concentration (nanomol/mL). In regard to the significant difference between the normal and the diseased, $P$ was less than 0.01 according to a Student's t-test;

FIG. 4-12 shows a bar chart presenting averages and standard errors of L-proline concentrations in the urine from three dilated cardiomyopathy model mice (hereinafter, may be referred to as "diseased") and four control normal mice (hereinafter, may be referred to as "normal"). The ordinate indicates the L-proline concentration (nanomol/mL);

FIG. 4-13 shows a bar chart presenting averages and standard errors of total proline concentrations (the sums of D-proline concentration and L-proline concentration) in the urine from three dilated cardiomyopathy model mice (hereinafter, may be referred to as "diseased") and four control normal mice (hereinafter, may be referred to as "normal"). The ordinate indicates the total proline concentration (nanomol/mL);

FIG. 4-14 shows a bar chart presenting averages and standard errors of D-lysine concentrations in the urine from three dilated cardiomyopathy model mice (hereinafter, may be referred to as "diseased") and four control normal mice (hereinafter, may be referred to as "normal"). The ordinate indicates the D-lysine concentration (nanomol/mL). In regard to the significant difference between the normal and the diseased, $P$ was less than 0.01 according to a Student's t-test;

FIG. 4-15 shows a bar chart presenting averages and standard errors of L-lysine concentrations in the urine from three dilated cardiomyopathy model mice (hereinafter, may be referred to as "diseased") and four control normal mice (hereinafter, may be referred to as "normal"). The ordinate indicates the L-lysine concentration (nanomol/mL);

FIG. 4-16 shows a bar chart presenting averages and standard errors of total lysine concentrations (the sums of D-lysine concentration and L-lysine concentration) in the urine from three dilated cardiomyopathy model mice (hereinafter, may be referred to as "diseased") and four control normal mice (hereinafter, may be referred to as "normal"). The ordinate indicates the total lysine concentration (nanomol/mL);

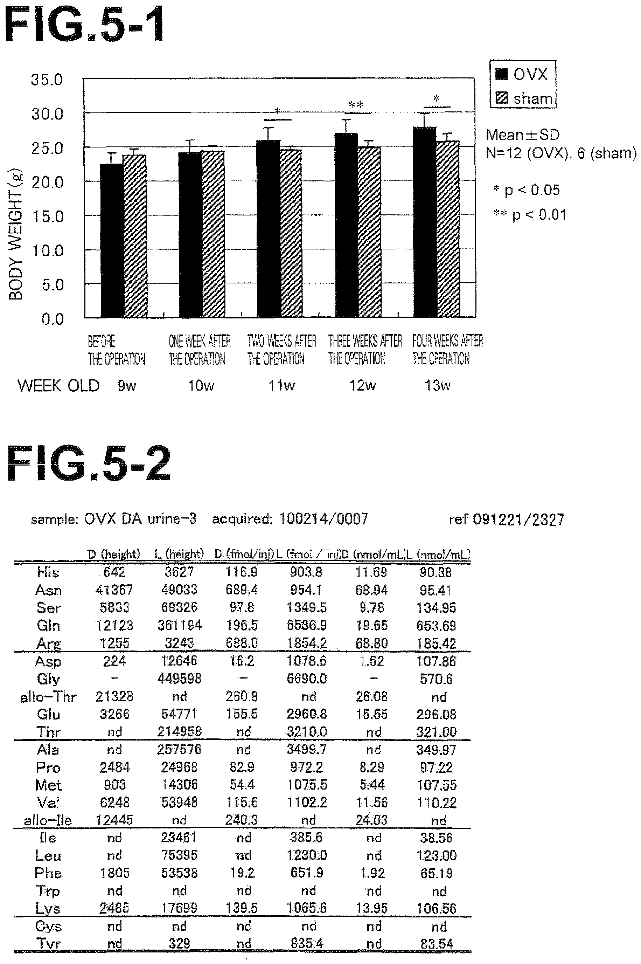

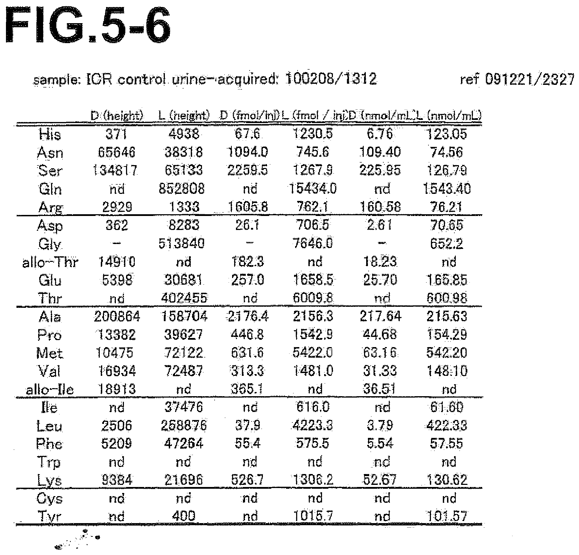

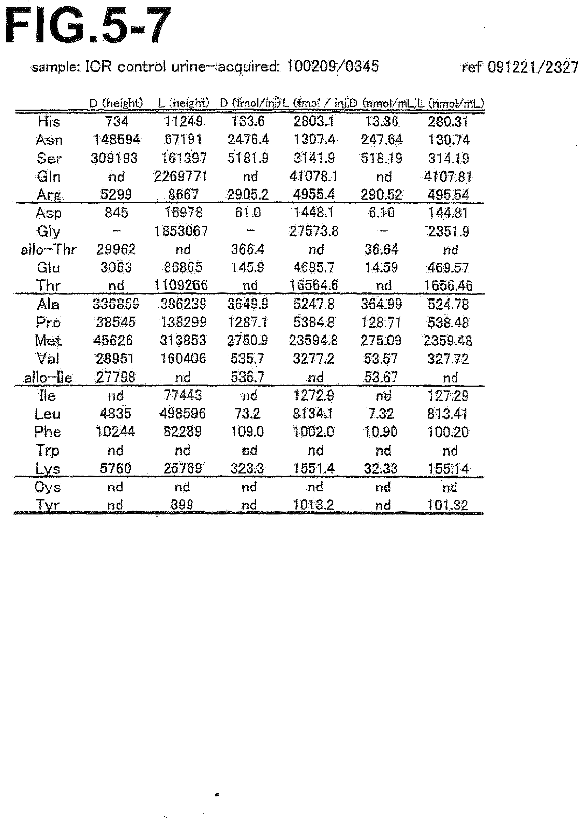

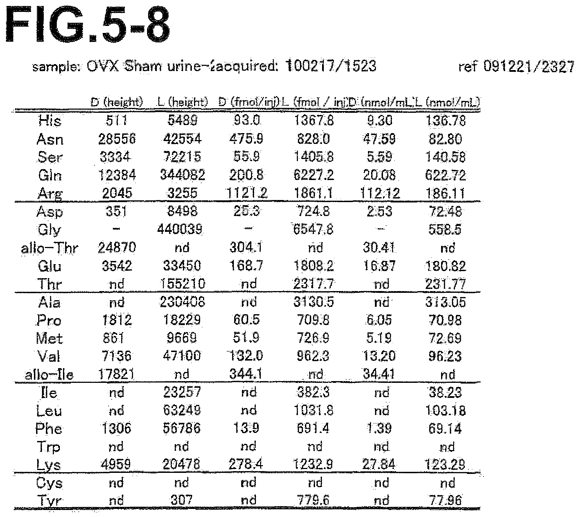

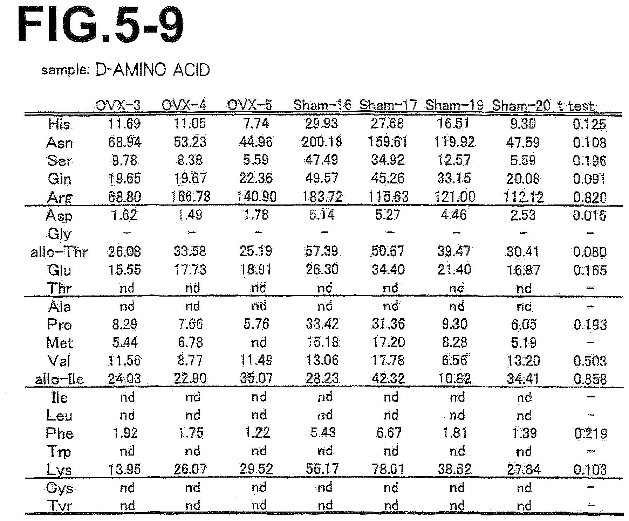

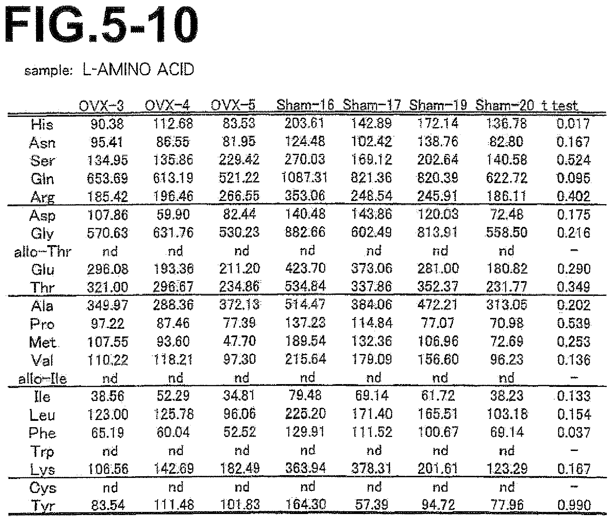

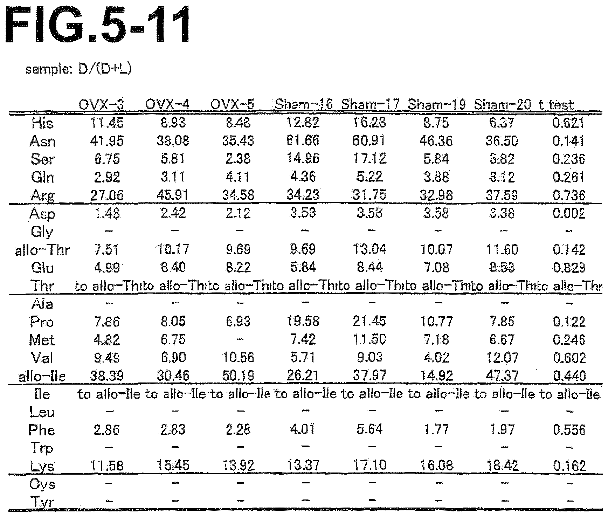

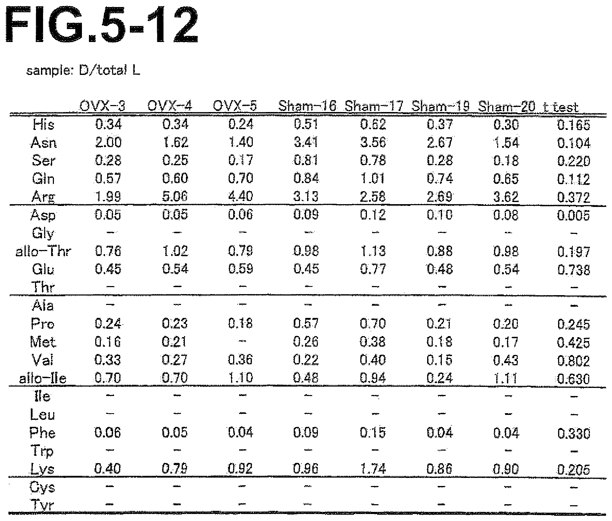

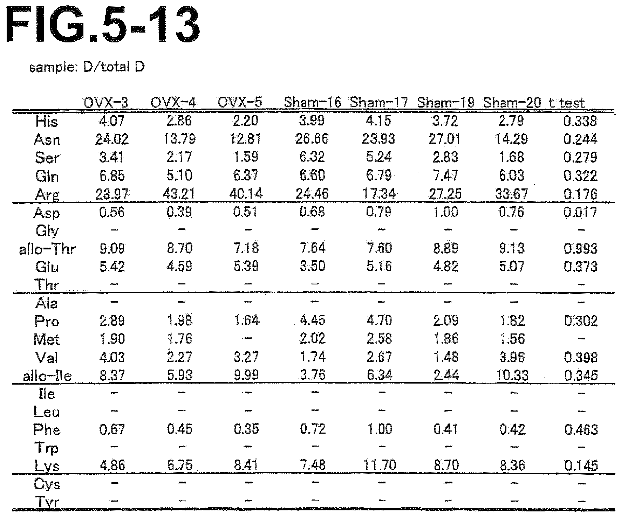

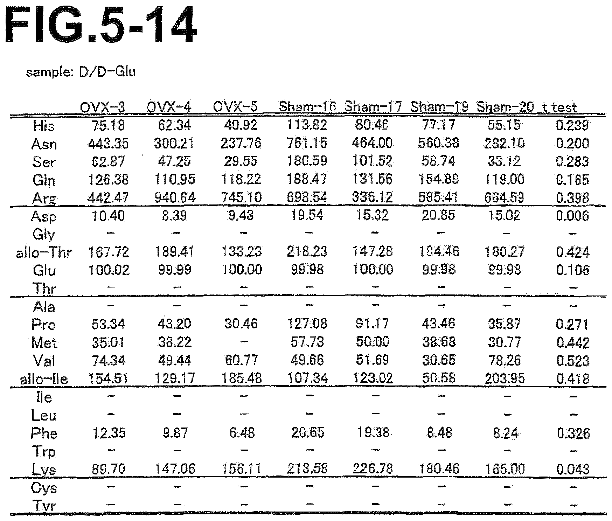

FIG. 5-1 shows a graph presenting the change of averages and standard deviations of the body weights of 12 climacterium model mice (hereinafter, may be referred to as "OVX") obtained by extirpation of the ovary from 9 weeks old HR-1 mice, and six control mice (hereinafter, may be referred to as "sham") subjected to only skin incision and suture without carrying out the extirpation of the ovary from female mice of the same weeks old. The ordinate indicates the body weight (gram) of the mice. The black solid bars and the diagonally hatched bars show the average and standard deviation of the body weights of the mice before the operation (9 weeks old) and one week (10 weeks old), two weeks (11 weeks old), three weeks (12 weeks old) and four weeks (13 weeks old) after the operation of both climacterium model mice (OVX) and control mice (sham), respectively. In regard to the significant difference of the body weights between the climacterium model mice (OVX) and the control mice (sham), P was less than 0.05 (*) or less than 0.01 (**) according to a Student's t-test. In climacterium model mice obtained by extirpation of the ovary, the body weight significantly increased compared with the control mice from two weeks after the operation, suggesting that the climacterium model mice were successfully produced;

FIG. 5-2 shows a Table presenting results of the total analysis of amino acid optical isomer contents in the urine from the first mouse (OVX-3) among three climacterium model mice (OVX). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

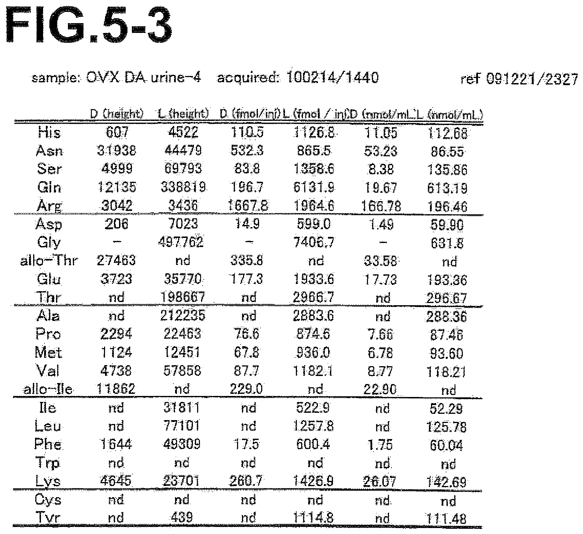

FIG. 5-3 shows a Table presenting results of the total analysis of amino acid optical isomer contents in the urine from the second mouse (OVX-4) among three climacterium model mice (OVX). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

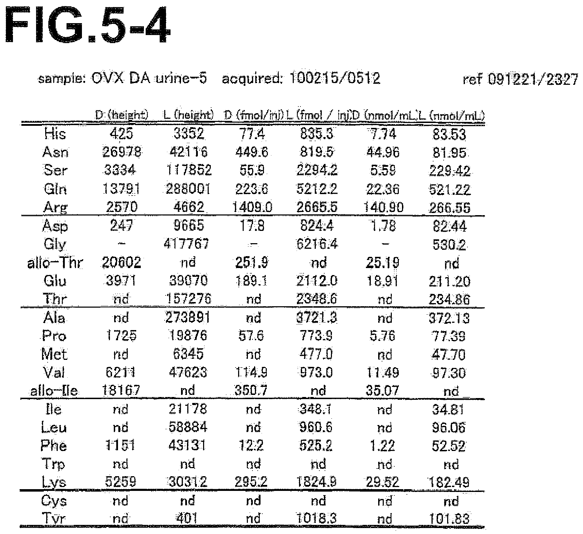

FIG. 5-4 shows a Table presenting results of the total analysis of amino acid optical isomer contents in the urine from the third mouse (OVX-5) among three climacterium model mice (OVX). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

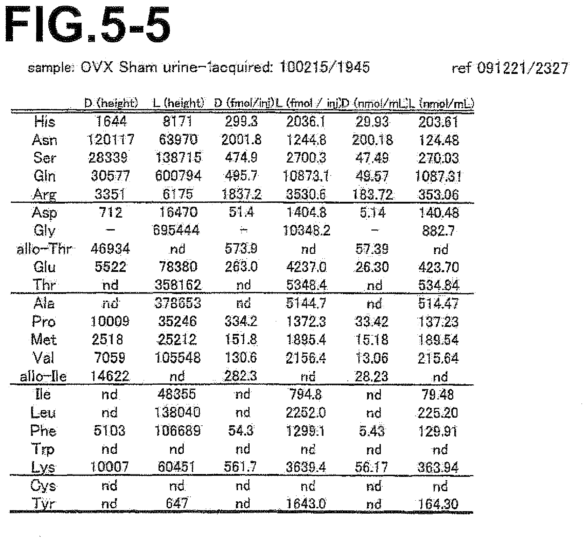

FIG. 5-5 shows a Table presenting results of the total analysis of amino acid optical isomer contents in the urine from the first mouse (Sham-16) among four control mice (sham). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

FIG. 5-6 shows a Table presenting results of the total analysis of amino acid optical isomer contents in the urine from the second mouse (Sham-17) among four control mice (sham). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

FIG. 5-7 shows a Table presenting results of the total analysis of amino acid optical isomer contents in the urine from the third mouse (Sham-19) among four control mice (sham). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

FIG. 5-8 shows a Table presenting results of the total analysis of amino acid optical isomer contents in the urine from the fourth mouse (Sham-20) among four control mice (sham). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

FIG. 5-9 shows a Table comparing D-amino acid contents in the urine from three climacterium model mice (OVX) and four control mice (sham). The content of D-aspartic acid was lower in the control mice (sham) than in the climacterium model mice (OVX), and in regard to the significant difference, P was 0.015 according to a Student's t-test;

FIG. 5-10 shows a Table comparing L-amino acid contents in the urine from three climacterium model mice (OVX) and four control mice (sham). The contents of L-histidine and L-phenylalanine were lower in the climacterium model mice (OVX) than in the control mice (sham), and in regard to the significant difference, P was 0.017 and 0.037, respectively, according to a Student's t-test;

FIG. 5-11 shows a Table comparing percentages of the D-form concentration (%D) with respect to the sum of the D-form concentration and the L-form concentration of each amino acid in the urine from three climacterium model mice (OVX) and four control mice (sham). Since D-forms of threonine and isoleucine turn to be allo-forms in a living body, the %D was evaluated in terms of the percentage of the D-allo-form concentration with respect to the sum of the D-allo-form concentration and the L-form concentration. The percentage of D-aspartic acid was lower in the climacterium model mice (OVX) than in the control mice (sham), and in regard to the significant difference, P was 0.002 according to a Student's t-test;

FIG. 5-12 shows a Table comparing percentages of each D-amino acid concentration (%D/total L) with respect to the sum of the concentrations of all the L-amino acids in the urine from three climacterium model mice (OVX) and four control mice (sham). The percentage of D-aspartic acid was lower in the climacterium model mice (OVX) than in the control mice (sham), and in regard to the significant difference, P was 0.005 according to a Student's t-test;

FIG. 5-13 shows a Table comparing percentages of each D-amino acid concentration (%D/total D) with respect to the sum of the concentrations of all the D-amino acids in the urine from three climacterium model mice (OVX) and four control mice (sham). The percentage of D-aspartic acid was lower in the climacterium model mice (OVX) than in the control mice (sham), and in regard to the significant difference, P was 0.017 according to a Student's t-test;

FIG. 5-14 shows a Table comparing percentages of each D-amino acid concentration with respect to the D-glutamic acid concentration (%D/D-Glu) in the urine from three climacterium model mice (OVX) and four control mice (sham). Since D-aspartic acid is an acidic D-amino acid, an evaluation was made after a correction with the concentration of D-glutamic acid which is considered to be similarly metabolized. Also in the case of %D/D-Glu, the percentage of D-aspartic acid was lower in the climacterium model mice (OVX) than in the control mice (sham), and in regard to the significant difference, P was 0.006 according to a Student's t-test;

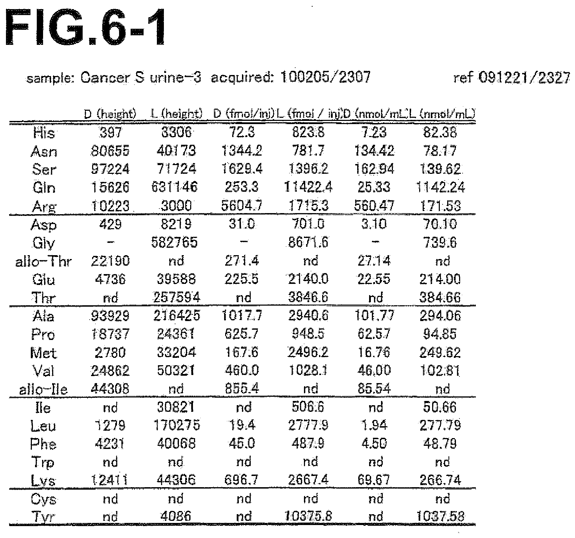

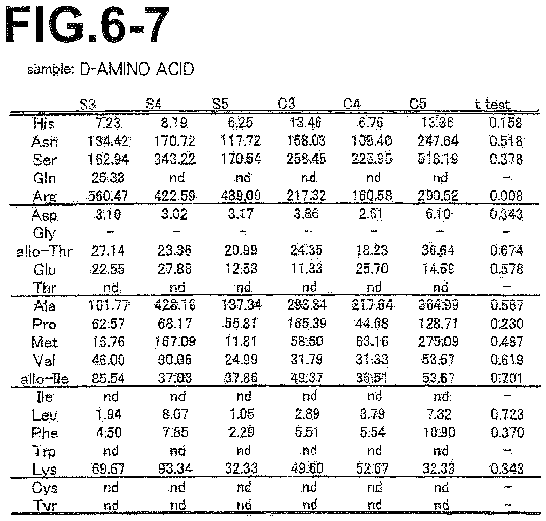

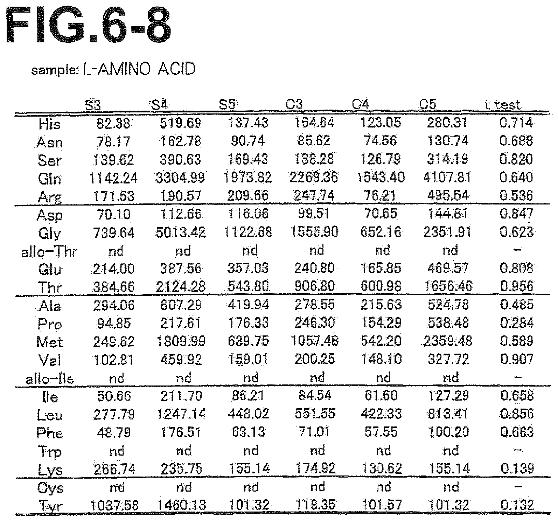

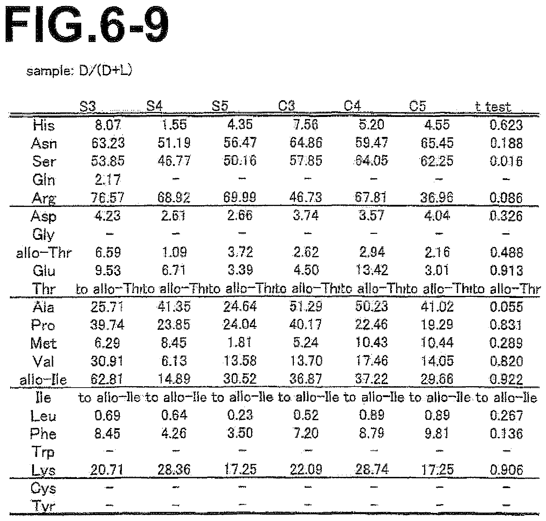

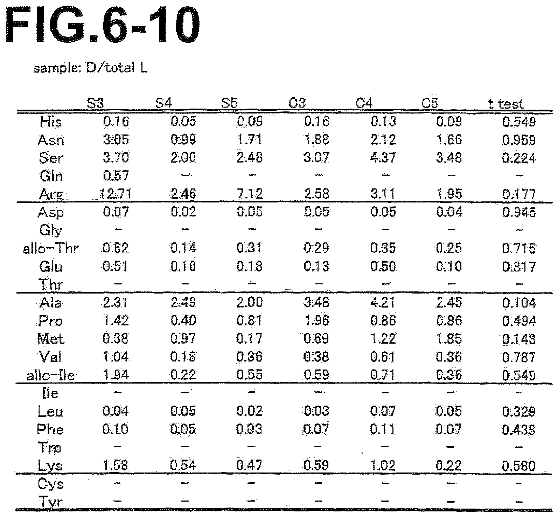

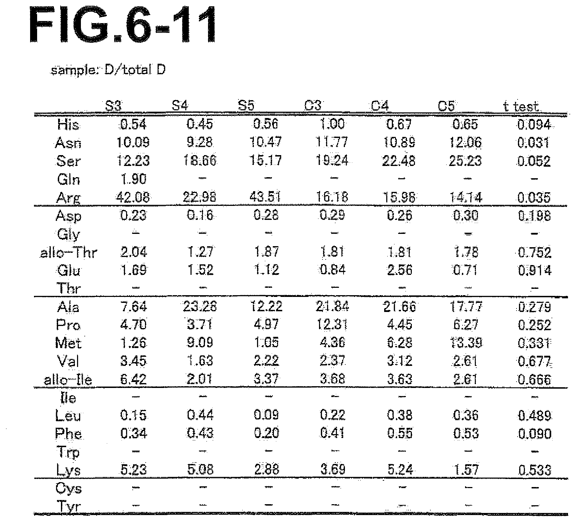

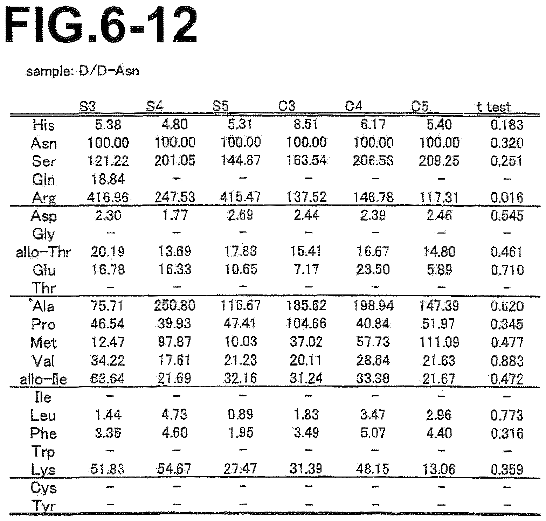

FIG. 6-1 shows a Table presenting results of the total analysis of amino acid optical isomer contents in the urine from the first mouse (S3) among three 6 weeks old male ICR mice transplanted with $2 \times 10^7$ sarcoma cells, in which growth of the explanted tumor was verified three weeks after the transplantation. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

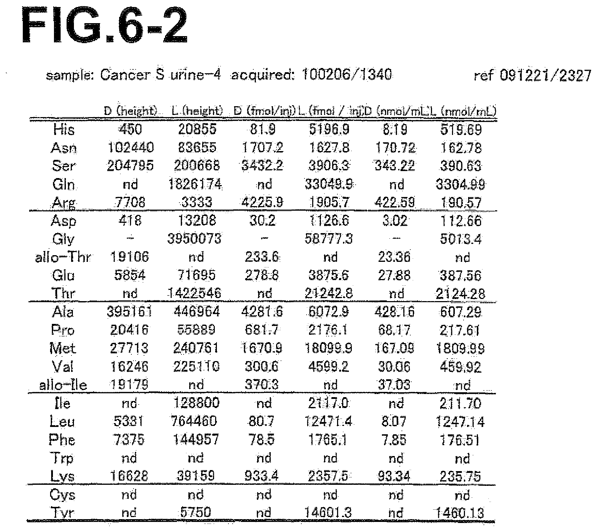

FIG. 6-2 shows a Table presenting results of the total analysis of amino acid optical isomer contents in the urine from the second mouse (S4) among three 6 weeks old male ICR mice transplanted with $2 \times 10^7$ sarcoma cells, in which growth of the explanted tumor was verified three weeks after the transplantation. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

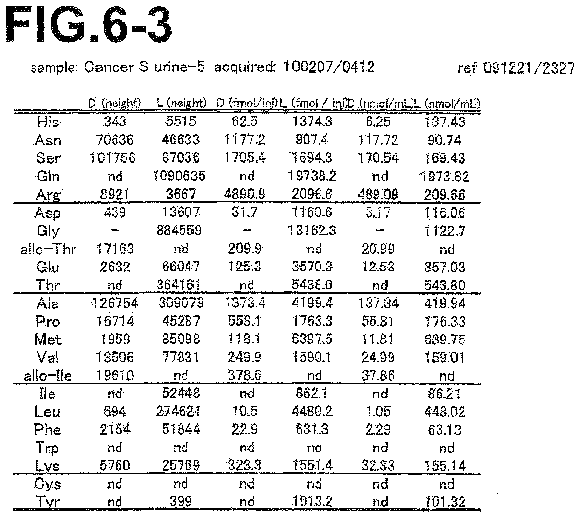

FIG. 6-3 shows a Table presenting results of the total analysis of amino acid optical isomer contents in the urine from the third mouse (S5) among three 6 weeks old male ICR mice transplanted with $2 \times 10^7$ sarcoma cells, in which growth of the explanted tumor was verified three weeks after the transplantation. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

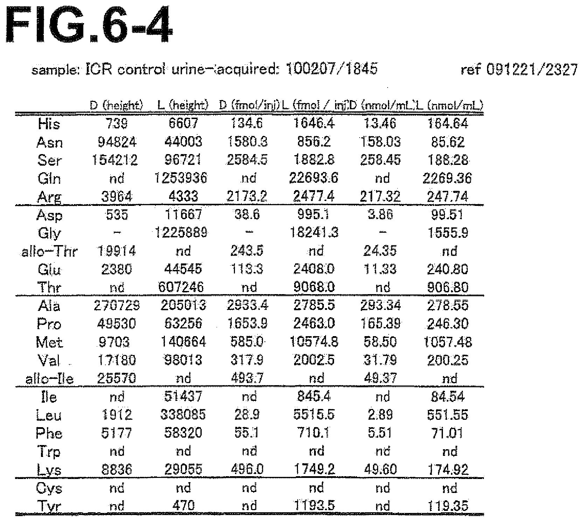

FIG. 6-4 shows a Table presenting results of the total analysis of amino acid optical isomer contents in the urine from the first mouse (C3) among three 9 weeks old male ICR mice fed for a control experiment in the same environment as that of the sarcoma-transplanted mice. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

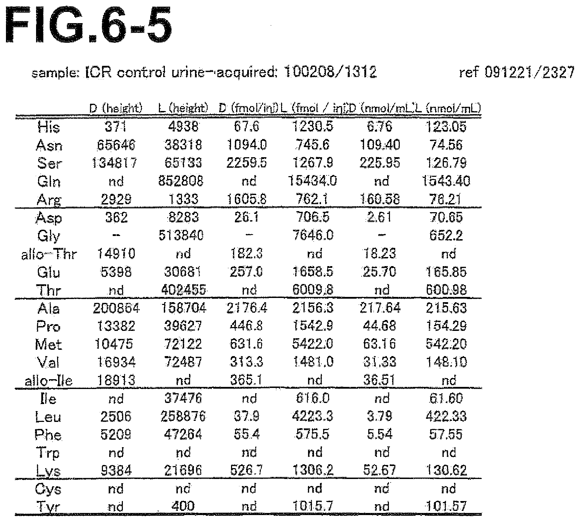

FIG. 6-5 shows a Table presenting results of the total analysis of amino acid optical isomer contents in the urine from the second mouse (C4) among three 9 weeks old male ICR mice fed for a control experiment in the same environment as that of the sarcoma-transplanted mice. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

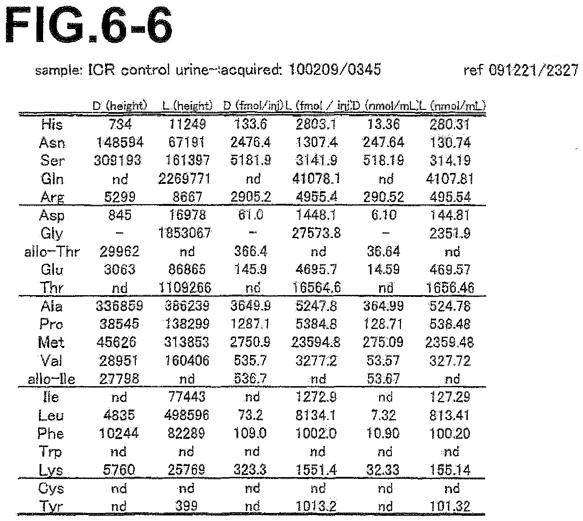

FIG. 6-6 shows a Table presenting results of the total analysis of amino acid optical isomer contents in the urine from the third mouse (C5) among three 9 weeks old male ICR mice fed for a control experiment in the same environment as that of the sarcoma-transplanted mice. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

FIG. 6-7 shows a Table comparing D-amino acid contents in the urine from three sarcoma-transplanted mice (S3, S4 and S5) and three control mice (C3, C4 and C5). The content of D-arginine was higher in the sarcoma-transplanted mice than in the control mice, and in regard to the significant difference, P was 0.008 according to a Student's t-test;

FIG. 6-8 shows a Table comparing L-amino acid contents in the urine from three sarcoma-transplanted mice (S3, S4 and S5) and three control mice (C3, C4 and C5);

FIG. 6-9 shows a Table comparing percentages of the D-form concentration (%D) with respect to the sum of the D-form concentration and the L-form concentration of each amino acid in the urine from three sarcoma-transplanted mice (S3, S4 and S5) and three control mice (C3, C4 and C5). The %D of serine was lower in the sarcoma-transplanted mice than in the control mice, and in regard to the significant difference, P was 0.016 according to a Student's t-test;

FIG. 6-10 shows a Table comparing percentages of each D-amino acid concentration (%D/total L) with respect to the sum of the concentrations of all the L-amino acids in the urine from three sarcoma-transplanted mice (S3, S4 and S5) and three control mice (C3, C4 and C5);

FIG. 6-11 shows a Table comparing percentages of each D-amino acid concentration (%D/total D) with respect to the sum of the concentrations of all the D-amino acids in the urine from three sarcoma-transplanted mice (S3, S4 and S5) and three control mice (C3, C4 and C5). The percentages of D-asparagine and D-arginine tended to be higher in the sarcoma-transplanted mice than in the control mice, and in regard to the significant difference of D-arginine, P was 0.035 according to a Student's t-test;

FIG. 6-12 shows a Table comparing percentages of each D-amino acid concentration with respect to the D-asparagine concentration (%D/D-Asn) in the urine from three sarcoma-transplanted mice (S3, S4 and S5) and three control mice (C3, C4 and C5). D-asparagine was used as a basis for a correction of the concentration in the urine since D-asparagine was present in mammalian urine at a comparatively high concentration, and the proportion with respect to the total D-amino acid concentration was most stable. The percentage of D-arginine tended to be higher in the sarcoma-transplanted mice than in the control mice, and in regard to the significant difference, P was 0.016 according to a Student's t-test;

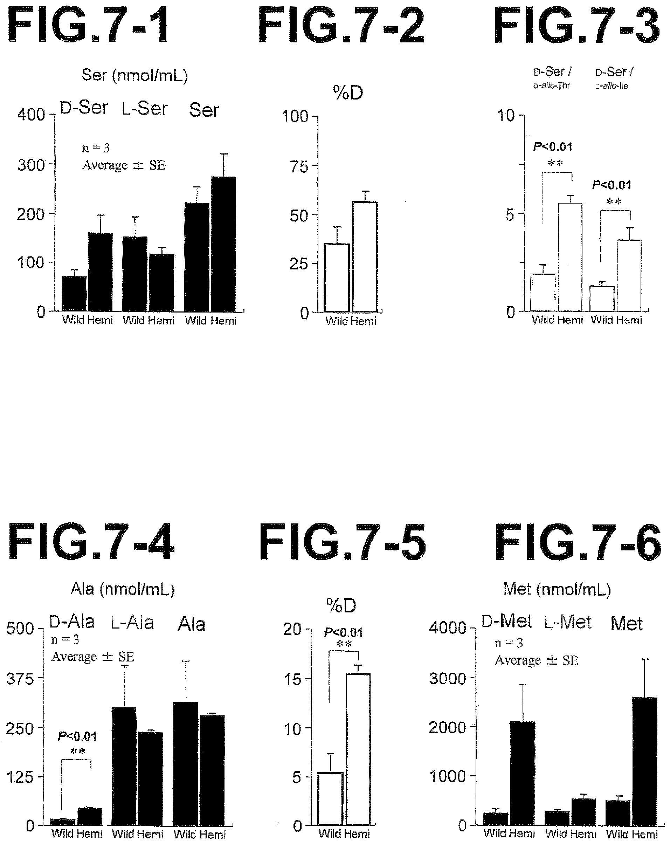

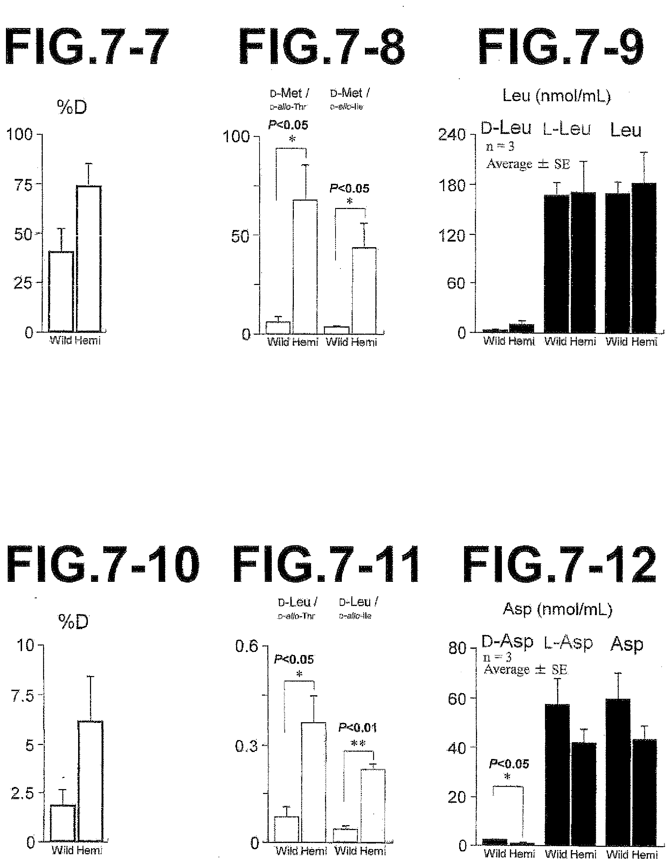

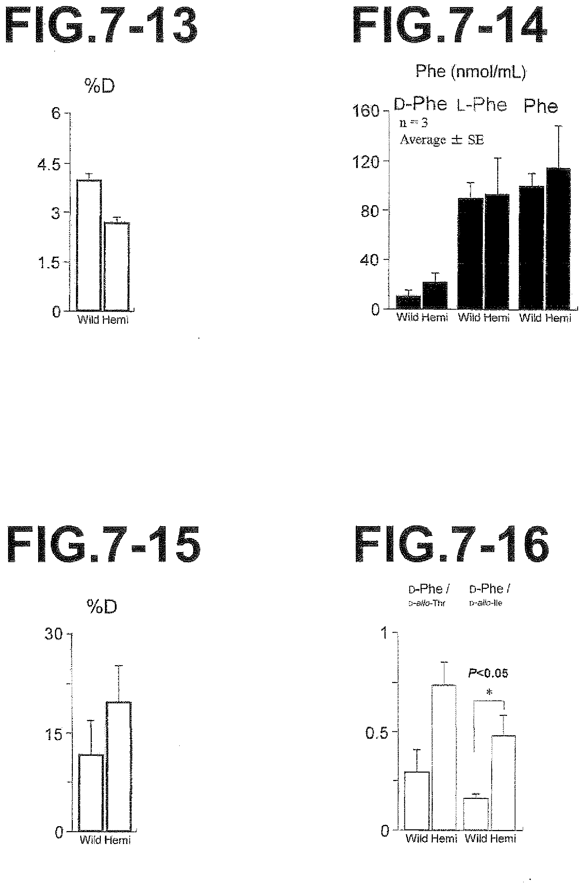

FIG. 7-1 shows a bar chart presenting averages and standard errors of D-serine concentrations (D-Ser), L-serine concentrations (L-Ser) and the sum of both concentrations (Ser) in the urine from three Alzheimer's disease model mice (8 weeks old male hemizygote mice of an amyloid β precursor protein-highly expressing transgenic mouse Tg2576, hereinafter, may be referred to as "hemi") and three control normal mice (C57BL, hereinafter, may be referred to as "Wild"). The ordinate indicates the concentration (nanomol/mL);

FIG. 7-2 shows a bar chart presenting averages and standard errors of the percentages of D-serine concentration (%D) with respect to the total serine concentration in the urine from three Alzheimer's disease model mice (hemi) and three control normal mice (Wild). The ordinate indicates the %D;

FIG. 7-3 shows a graph presenting a relative ratio of the D-serine concentration to the D-allo-threonine concentration (D-Ser/D-allo-Thr) or a relative ratio of the D-serine concentration to the D-allo-isoleucine concentration (D-Ser/D-allo-Ile) in the urine from three Alzheimer's disease model mice (hemi) and three control normal mice (Wild). Both the relative ratio of the D-serine concentration to the D-allo-threonine concentration in the urine, and the relative ratio of the D-serine concentration to the D-allo-isoleucine concentration in the urine were higher in the Alzheimer's disease model mice than in the control mice, and in regard to the significant difference, P was less than 0.01 according to a Student's t-test in both cases;

FIG. 7-4 shows a bar chart presenting averages and standard errors of D-alanine concentrations (D-Ala), L-alanine concentrations (L-Ala) and the sum of both concentrations (Ala) in the urine from three Alzheimer's disease model mice (hemi) and three control normal mice (Wild). The ordinate indicates the concentration (nanomol/mL). The D-alanine concentration in the urine was higher in the Alzheimer's disease model mice than in the control normal mice, and in regard to the significant difference, P was less than 0.01 according to a Student's t-test;

FIG. 7-5 shows a bar chart presenting averages and standard errors of the percentages of D-alanine concentration (%D) with respect to the total alanine concentration in the urine from three Alzheimer's disease model mice (hemi) and three control normal mice (Wild). The ordinate indicates the %D. The percentage of the D-alanine concentration (%D) with respect to the total alanine concentration in the urine was higher in the Alzheimer's disease model mice than in the control normal mice, and in regard to the significant difference, P was less than 0.01 according to a Student's t-test;

FIG. 7-6 shows a bar chart presenting averages and standard errors of D-methionine concentrations (D-Met), L-methionine concentrations (L-Met) and the sum of both concentrations (Met) in the urine from three Alzheimer's disease model mice (hemi) and three control normal mice (Wild). The ordinate indicates the concentration (nanomol/mL). The D-Met concentration in the urine tended to be higher in the Alzheimer's disease model mice than in the control normal mice;

FIG. 7-7 shows a bar chart presenting averages and standard errors of the percentages of D-methionine concentration (%D) with respect to the total serine concentration in the urine from three Alzheimer's disease model mice (hemi) and three control normal mice (Wild). The ordinate indicates the %D;

FIG. 7-8 shows a graph presenting a relative ratio of the D-methionine concentration to the D-allo-threonine concentration (D-Met/D-allo-Thr) or a relative ratio of the D-methionine concentration to the D-allo-isoleucine concentration (D-Met/D-allo-Ile) in the urine from three Alzheimer's disease model mice (hemi) and three control normal mice (Wild). Both the relative ratio of the D-methionine concentration to the D-allo-threonine concentration in the urine, and the relative ratio of the D-methionine concentration to the D-allo-isoleucine concentration in the urine were higher in the Alzheimer's disease model mice than in the control mice, and in regard to the significant difference, P was less than 0.05 according to a Student's t-test in both cases;

FIG. 7-9 shows a bar chart presenting averages and standard errors of D-leucine concentrations (D-Leu), L-leucine concentrations (L-Leu) and the sum of both concentrations (Leu) in the urine from three Alzheimer's disease model mice (hemi) and three control normal mice (Wild). The ordinate indicates the concentration (nanomol/mL);

FIG. 7-10 shows a bar chart presenting averages and standard errors of the percentages of D-leucine concentration (%D) with respect to the total serine concentration in the urine from three Alzheimer's disease model mice (hemi) and three control normal mice (Wild). The ordinate indicates the %D;

FIG. 7-11 shows a graph presenting a relative ratio of the D-leucine concentration to the D-allo-threonine concentration (D-Leu/D-allo-Thr) or a relative ratio of the D-leucine concentration to the D-allo-isoleucine concentration (D-Leu/D-allo-Ile) in the urine from three Alzheimer's disease model mice (hemi) and three control normal mice (Wild). Both the relative ratio of the D-leucine concentration to the D-allo-threonine concentration in the urine, and the relative ratio of the D-leucine concentration to the D-allo-isoleucine concentration in the urine were higher in the Alzheimer's disease model mice than in the control mice, and in regard to the significant difference, P was less than 0.05 and less than 0.01, respectively, according to a Student's t-test;

FIG. 7-12 shows a bar chart presenting averages and standard errors of D-aspartic acid concentrations (D-Asp), L-aspartic acid concentrations (L-Asp) and the sum of both concentrations (Asp) in the urine from three Alzheimer's disease model mice (hemi) and three control normal mice (Wild). The ordinate indicates the concentration (nanomol/mL). The D-aspartic acid concentration in the urine was lower in the Alzheimer's disease model mice than in the control normal mice, and in regard to the significant difference, P was less than 0.05 according to a Student's t-test;

FIG. 7-13 shows a bar chart presenting averages and standard errors of the percentages of the D-aspartic acid concentration (%D) with respect to the total aspartic acid concentration in the urine from three Alzheimer's disease model mice (hemi) and three control normal mice (Wild). The ordinate indicates the %D;

FIG. 7-14 shows a bar chart presenting averages and standard errors of D-phenylalanine concentrations (D-Phe), L-phenylalanine concentrations (L-Phe) and the sum of both concentrations (Phe) in the urine from three Alzheimer's disease model mice (hemi) and three control normal mice (Wild). The ordinate indicates the concentration (nanomol/mL);

FIG. 7-15 shows a bar chart presenting averages and standard errors of the percentages of D-phenylalanine concentration (%D) with respect to the total phenylalanine concentration in the urine from three Alzheimer's disease model mice (hemi) and three control normal mice (Wild). The ordinate indicates the %D;

FIG. 7-16 shows a graph presenting a relative ratio of the D-phenylalanine concentration to the D-allo-threonine concentration (D-Phe/D-allo-Thr) or a relative ratio of the D-phenylalanine concentration to the D-allo-isoleucine concentration (D-Phe/D-allo-Ile) in the urine from three Alzheimer's disease model mice (hemi) and three control normal mice (Wild). The relative ratio of the D-phenylalanine concentration to the D-allo-isoleucine concentration in the urine was higher in the Alzheimer's disease model mice than in the control mice, and in regard to the significant difference, P was less than 0.05 according to a Student's t-test;

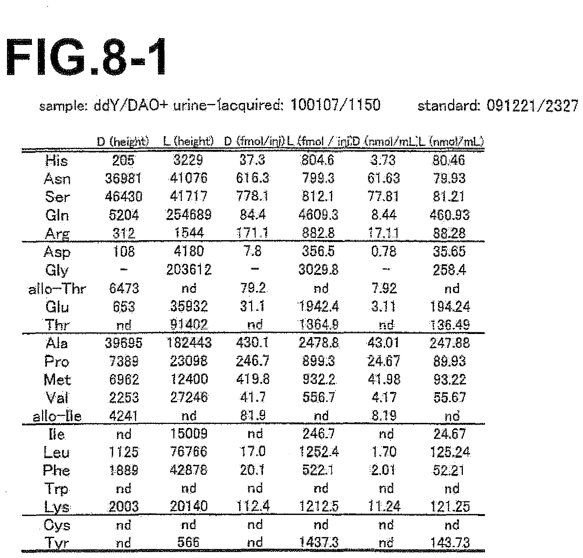

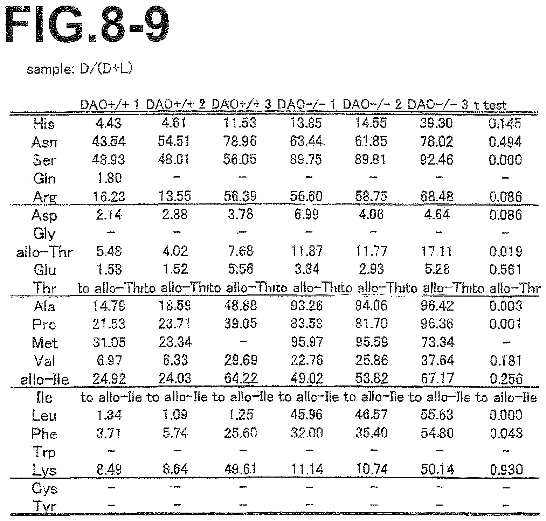

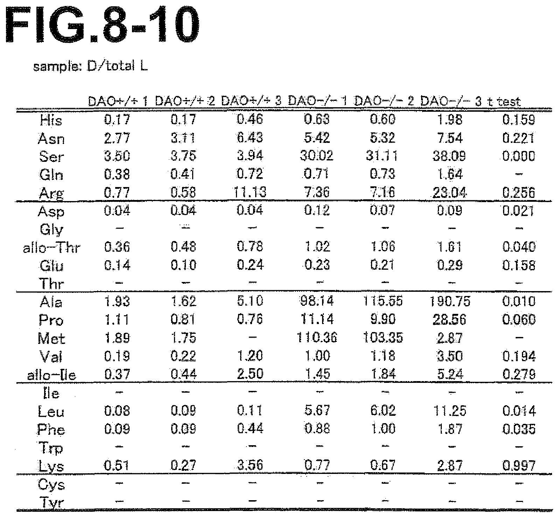

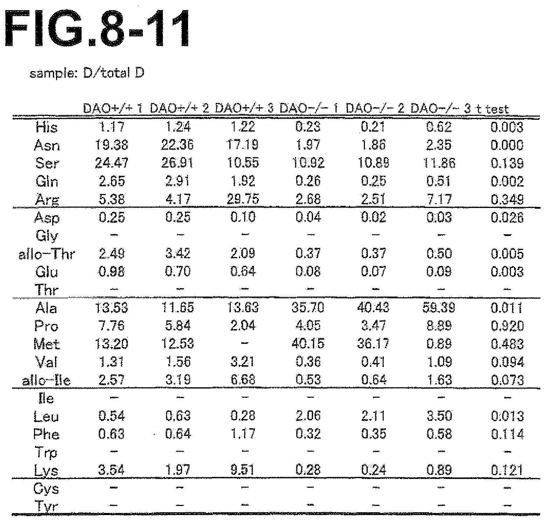

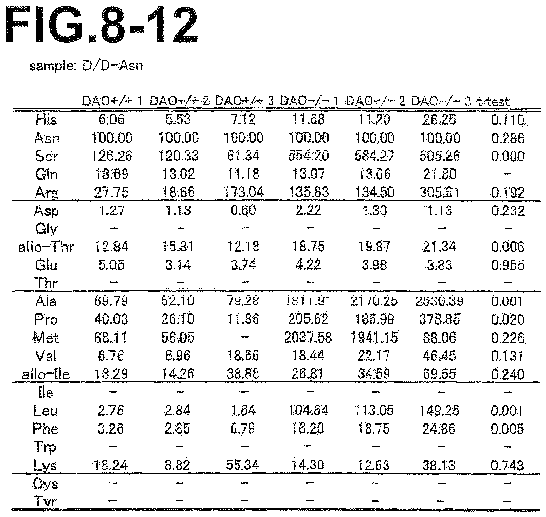

FIG. 8-1 shows a Table presenting results of the total analysis of amino acid optical isomer contents in the urine from the first mouse (DAO+/+ 1) among three D-amino acid oxidase (DAO) wild type mice (ddY/DAO+, which may be referred to as "DAO+/+"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

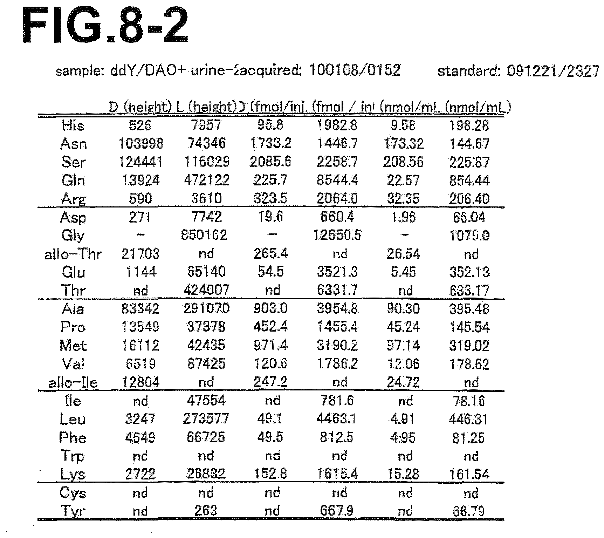

FIG. 8-2 shows a Table presenting results of the total analysis of amino acid optical isomer contents in the urine from the second mouse (DAO+/+ 2) among three D-amino acid oxidase (DAO) wild type mice (ddY/DAO+, which may be referred to as "DAO+/+"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

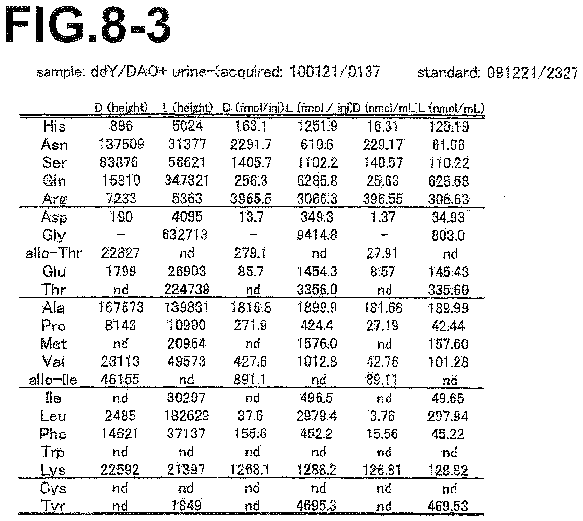

FIG. 8-3 shows a Table presenting results of the total analysis of amino acid optical isomer contents in the urine from the third mouse (DAO+/+ 3) among three D-amino acid oxidase (DAO) wild type mice (ddY/DAO+, which may be referred to as "DAO+/+"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

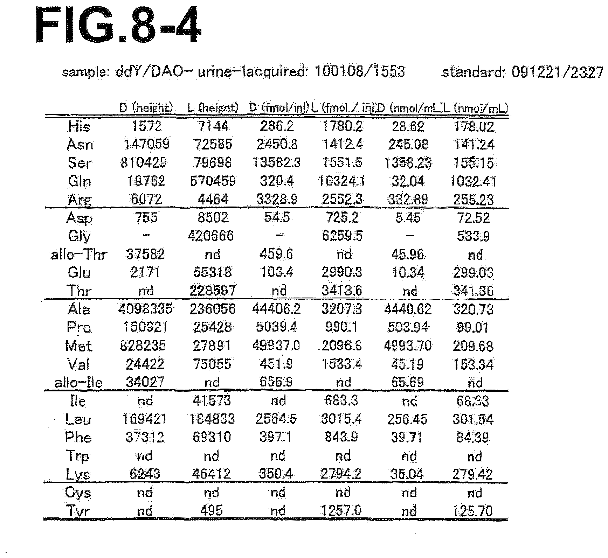

FIG. 8-4 shows a Table presenting results of the total analysis of amino acid optical isomer contents in the urine from the first mouse (DAO-/- 1) among three D-amino acid oxidase (DAO) deficient mice (ddY/DAO-, which may be referred to as "DAO-/-"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

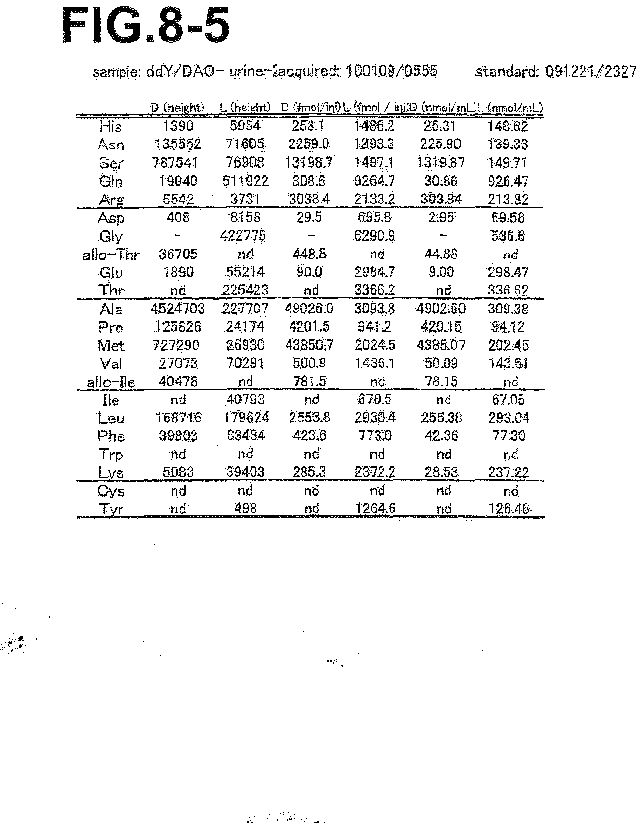

FIG. 8-5 shows a Table presenting results of the total analysis of amino acid optical isomer contents in the urine from the second mouse (DAO-/- 2) among three D-amino acid oxidase (DAO) deficient mice (ddY/DAO-, which may be referred to as "DAO-/-"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

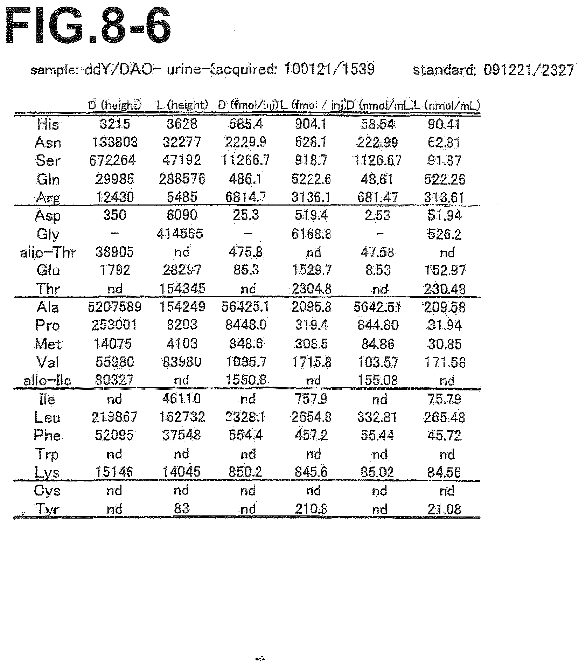

FIG. 8-6 shows a Table presenting results of the total analysis of amino acid optical isomer contents in the urine from the third mouse (DAO-/- 3) among three D-amino acid oxidase (DAO) deficient mice (ddY/DAO-, which may be referred to as "DAO-/-"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis;

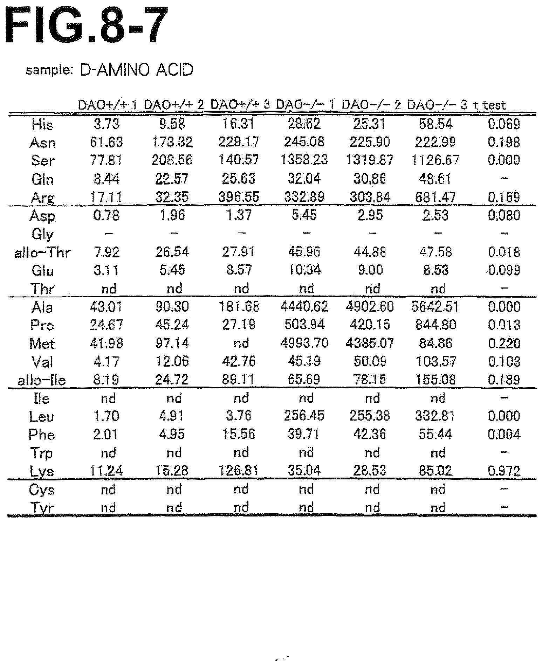

FIG. 8-7 shows a Table comparing D-amino acid contents in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The contents of D-serine, D-allo-threonine, D-alanine, D-proline, D-leucine and D-phenylalanine were significantly higher in the D-amino acid oxidase (DAO) enzyme deficient mice than in the DAO wild type mice;

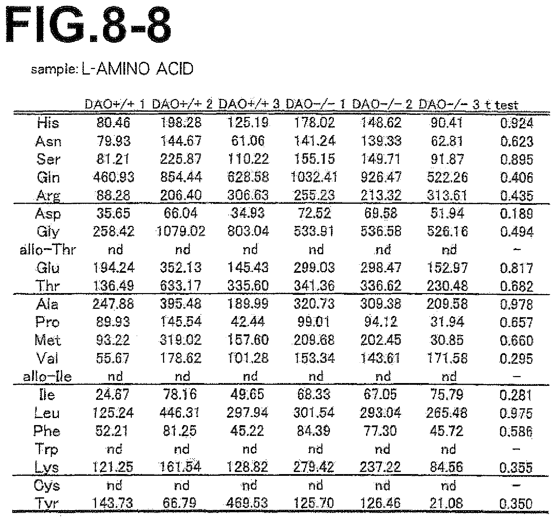

FIG. 8-8 shows a Table comparing L-amino acid contents in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3);

FIG. 8-9 shows a Table comparing percentages of the D-form concentration (%D) with respect to the sum of the D-form concentration and the L-form concentration of each amino acid in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-proline, D-leucine and D-phenylalanine were significantly higher in the D-amino acid oxidase (DAO) enzyme deficient mice than in the DAO wild type mice;

FIG. 8-10 shows a Table comparing percentages of each D-amino acid concentration (%D/total L) with respect to the sum of the concentrations of all the L-amino acids in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-leucine and D-phenylalanine were significantly higher in the D-amino acid oxidase (DAO) enzyme deficient mice than in the DAO wild type mice;

FIG. 8-11 shows a Table comparing percentages of each D-amino acid concentration (%D/total D) with respect to the sum of the concentrations of all the D-amino acids in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3);

FIG. 8-12 shows a Table comparing percentages of each D-amino acid concentration (%D/D-Asn) with respect to the D-asparagine concentration in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-proline, D-leucine and D-phenylalanine were significantly higher in the D-amino acid oxidase (DAO) enzyme deficient mice than in the DAO wild type mice;

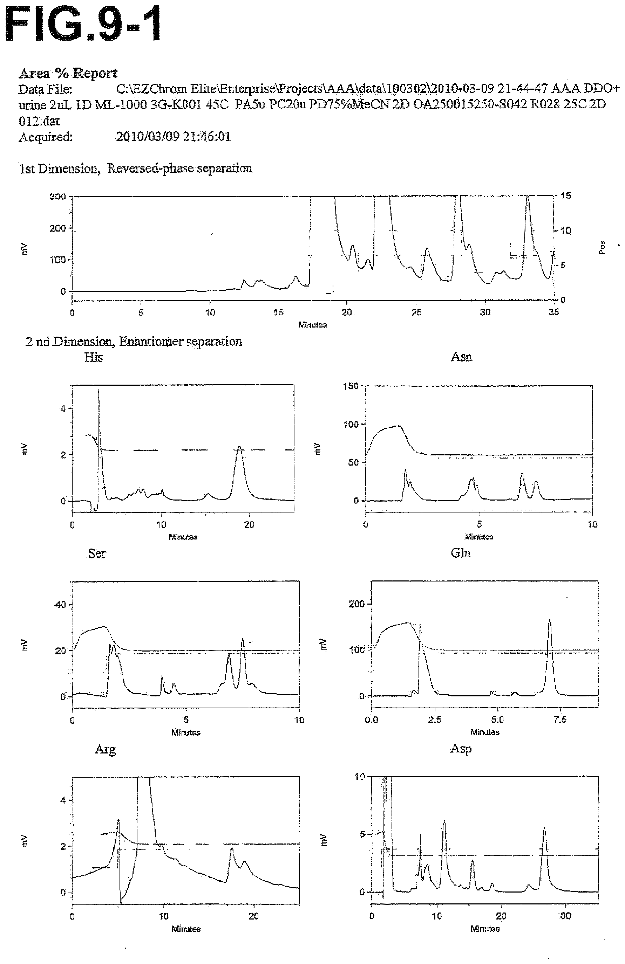

FIG. 9-1 shows a wave form chart presenting results of the total analysis of amino acid optical isomer contents in the urine from the first mouse among four D-aspartate oxidase (DDO) wild type mice (DDO+);

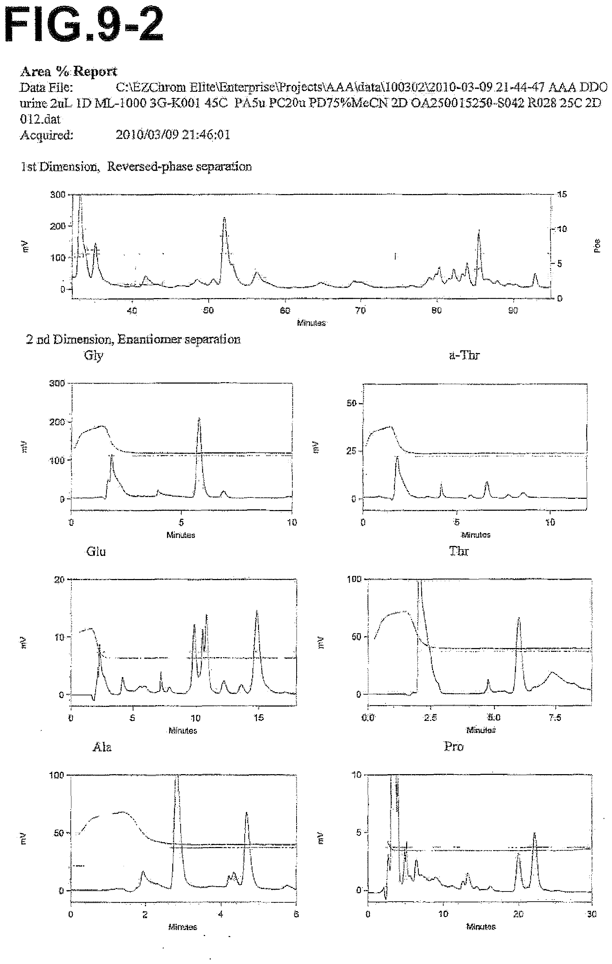

FIG. 9-2 shows a wave form chart presenting results of the total analysis of amino acid optical isomer contents in the urine from the second mouse among four D-aspartate oxidase (DDO) wild type mice (DDO+);

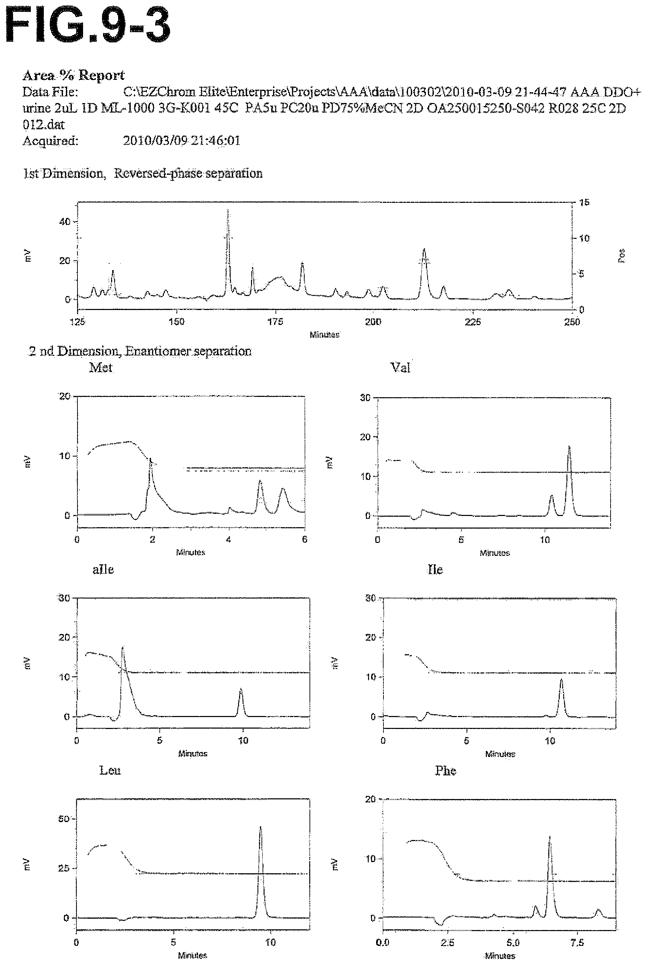

FIG. 9-3 shows a wave form chart presenting results of the total analysis of amino acid optical isomer contents in the urine from the third mouse among four D-aspartate oxidase (DDO) wild type mice (DDO+);

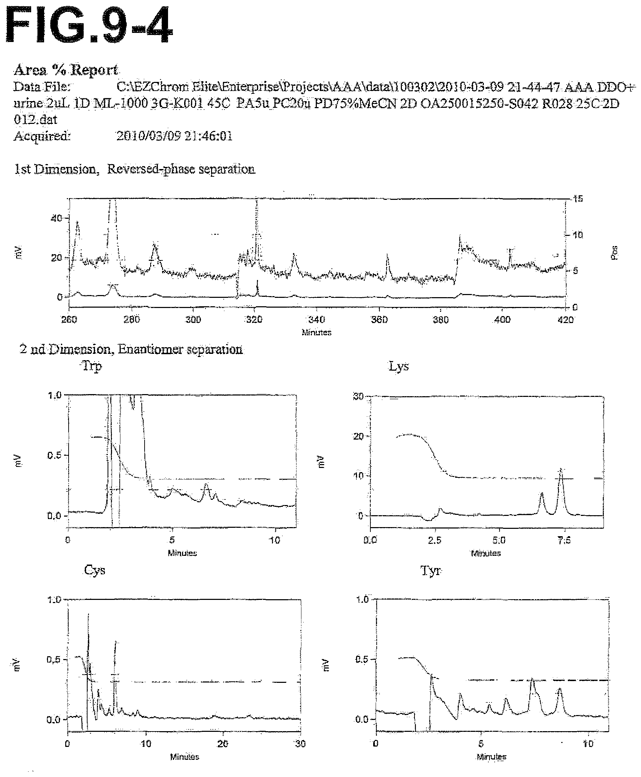

FIG. 9-4 shows a wave form chart presenting results of the total analysis of amino acid optical isomer contents in the urine from the fourth mouse among four D-aspartate oxidase (DDO) wild type mice (DDO+);

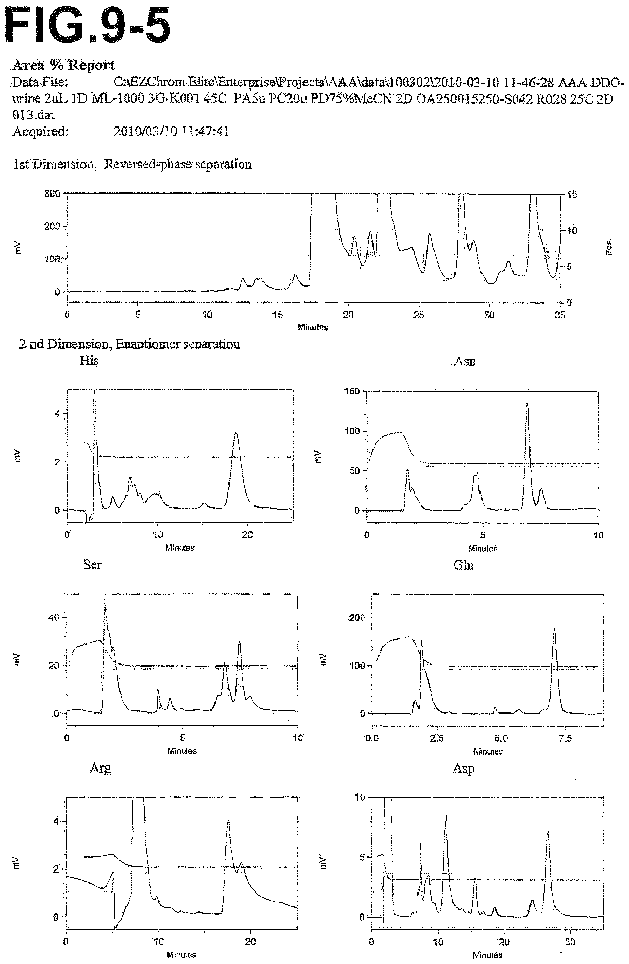

FIG. 9-5 shows a wave form chart presenting results of the total analysis of amino acid optical isomer contents in the urine from the first mouse among four D-aspartate oxidase (DDO) deficient mice (DDO-);

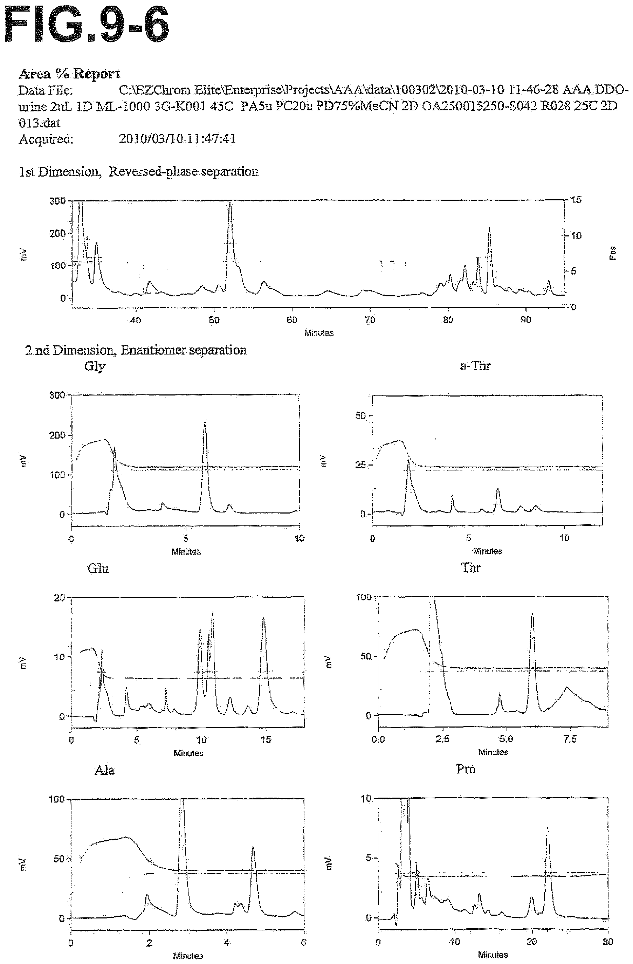

FIG. 9-6 shows a wave form chart presenting results of the total analysis of amino acid optical isomer contents in the urine from the second mouse among four D-aspartate oxidase (DDO) deficient mice (DDO-);

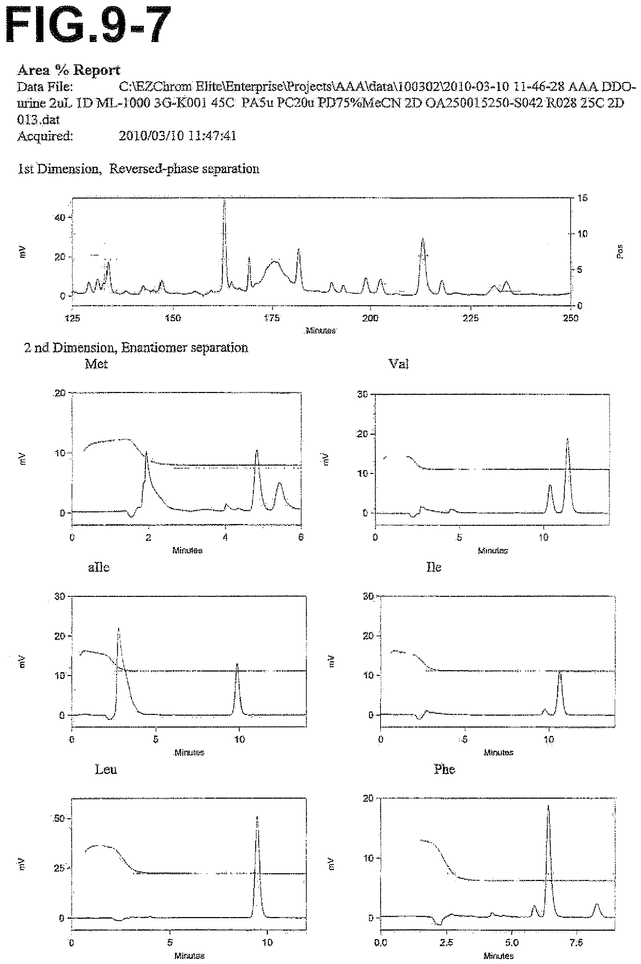

FIG. 9-7 shows a wave form chart presenting results of the total analysis of amino acid optical isomer contents in the urine from the third mouse among four D-aspartate oxidase (DDO) deficient mice (DDO-);

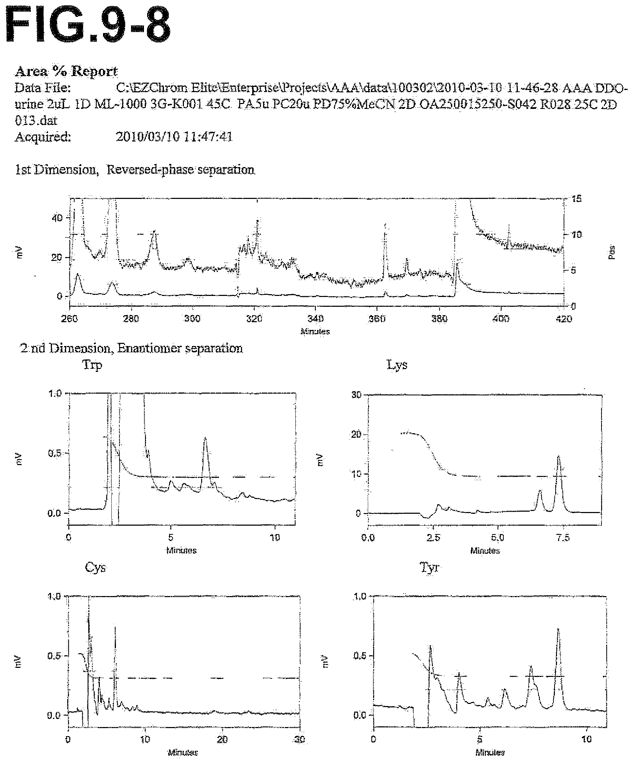

FIG. 9-8 shows a wave form chart presenting results of the total analysis of amino acid optical isomer contents in the urine from the fourth mouse among four D-aspartate oxidase (DDO) deficient mice (DDO-);

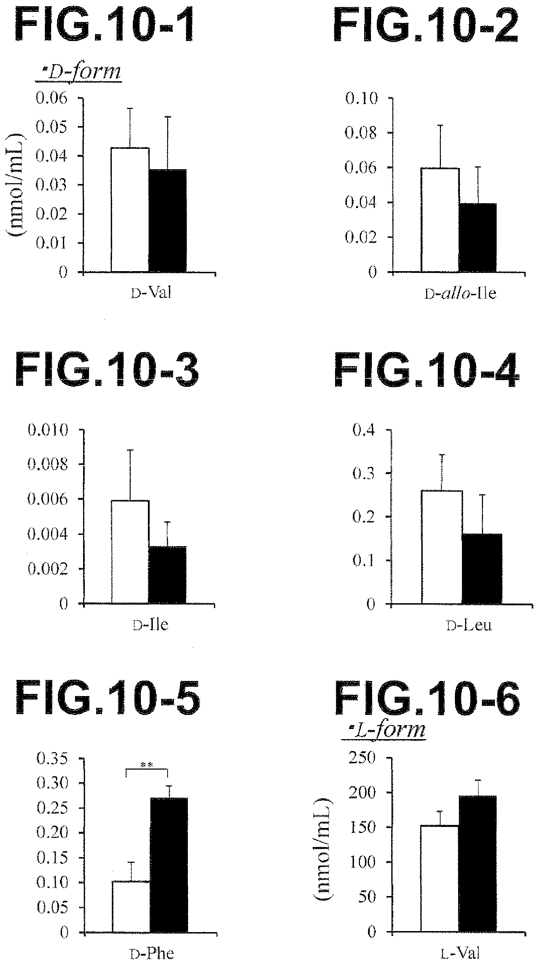

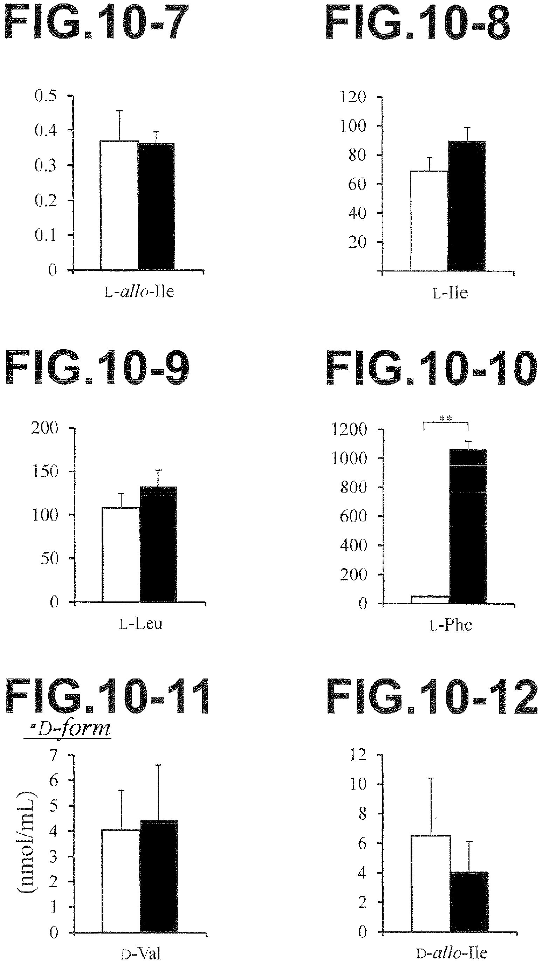

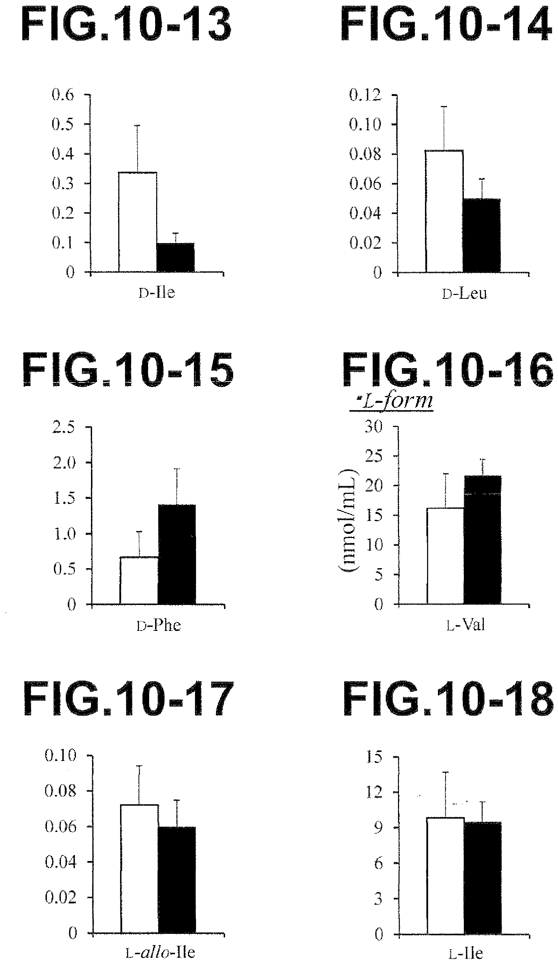

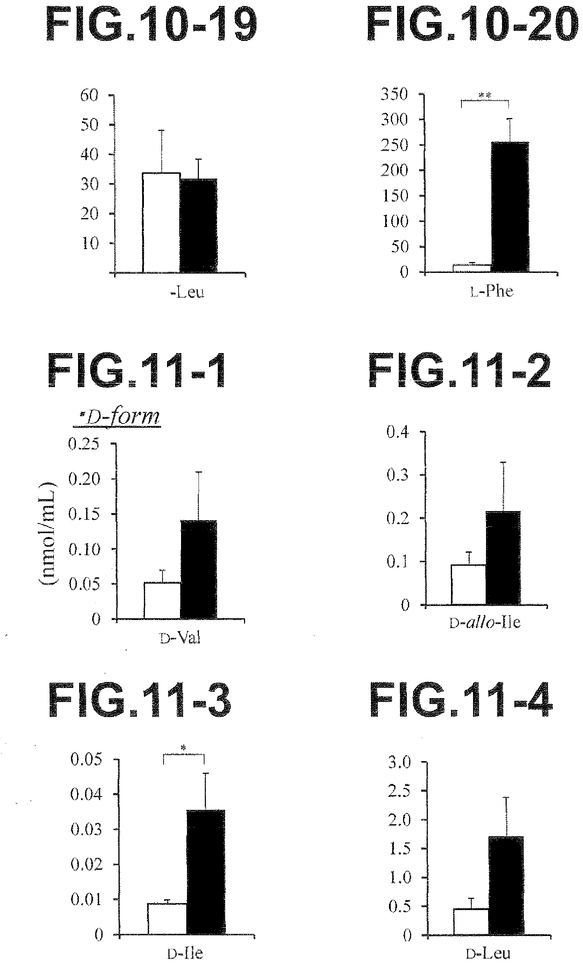

FIG. 10-1 shows a bar chart presenting D-valine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-2 shows a bar chart presenting D-allo-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-3 shows a bar chart presenting D-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-4 shows a bar chart presenting D-leucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-5 shows a bar chart presenting D-phenylalanine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). In regard to the significant difference, p is less than 0.01;

FIG. 10-6 shows a bar chart presenting L-valine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-7 shows a bar chart presenting L-allo-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-8 shows a bar chart presenting L-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-9 shows a bar chart presenting L-leucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid);

FIG. 10-10 shows a bar chart presenting L-phenylalanine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). In regard to the significant difference, p is less than 0.01;

FIG. 10-11 shows a bar chart presenting D-valine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-12 shows a bar chart presenting D-allo-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-13 shows a bar chart presenting D-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-14 shows a bar chart presenting D-leucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-15 shows a bar chart presenting D-phenylalanine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-16 shows a bar chart presenting L-valine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-17 shows a bar chart presenting L-allo-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-18 shows a bar chart presenting L-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-19 shows a bar chart presenting L-leucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid);

FIG. 10-20 shows a bar chart presenting L-phenylalanine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). In regard to the significant difference, p is less than 0.01;

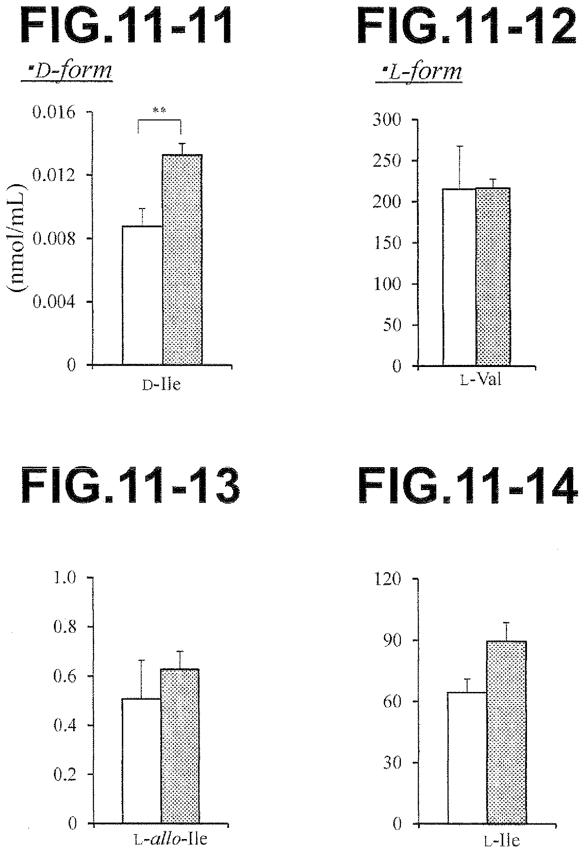

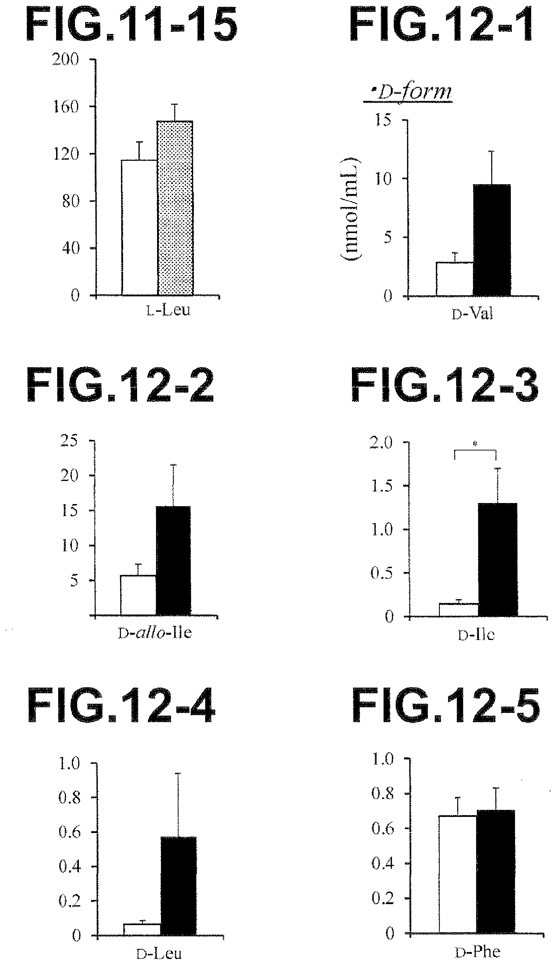

FIG. 11-1 shows a bar chart presenting D-valine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 11-2 shows a bar chart presenting D-allo-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 11-3 shows a bar chart presenting D-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.05;

FIG. 11-4 shows a bar chart presenting D-leucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

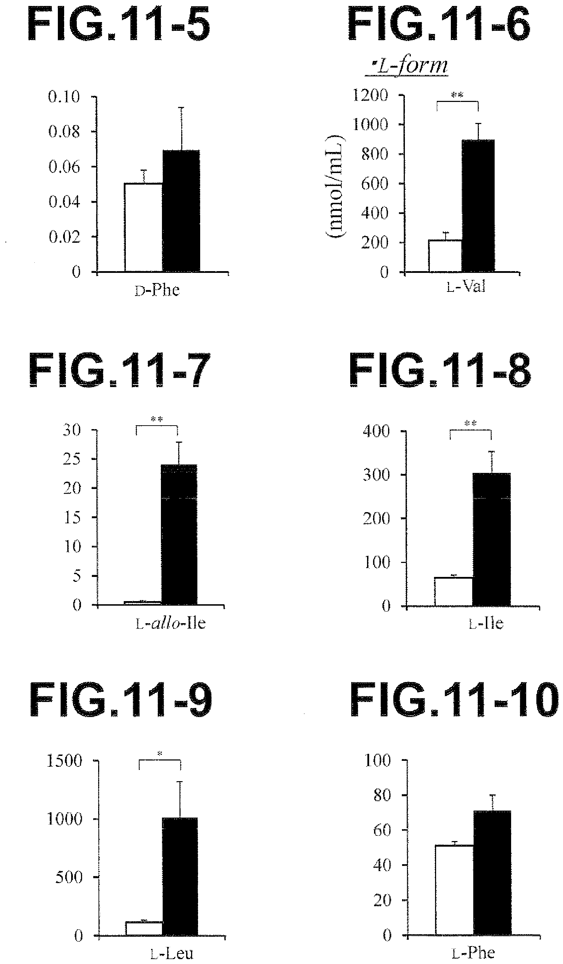

FIG. 11-5 shows a bar chart presenting D-phenylalanine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 11-6 shows a bar chart presenting L-valine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 11-7 shows a bar chart presenting L-allo-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 11-8 shows a bar chart presenting L-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 11-9 shows a bar chart presenting L-leucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.05;

FIG. 11-10 shows a bar chart presenting L-phenylalanine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 11-11 shows a bar chart presenting D-isoleucine concentrations (nanomol/mL) and standard errors thereof in the plasma from five maple syrup urine disease mice (branched-chain alpha-keto acid dehydrogenase (BCKDH) mutant mice, +/Dbt$^{tmlGeh}$; grey solid) and five control mice (white solid). In regard to the significant difference, p is less than 0.01;

FIG. 11-12 shows a bar chart presenting L-valine concentrations (nanomol/mL) and standard errors thereof in the plasma from five maple syrup urine disease mice (branched-chain alpha-keto acid dehydrogenase (BCKDH) mutant mice, +/Dbt$^{tmlGeh}$; grey solid) and five control mice (white solid);

FIG. 11-13 shows a bar chart presenting L-allo-isoleucine concentrations (nanomol/mL) and standard errors thereof in the plasma from five maple syrup urine disease mice (branched-chain alpha-keto acid dehydrogenase (BCKDH) mutant mice, +/Dbt$^{tmlGeh}$; grey solid) and five control mice (white solid);

FIG. 11-14 shows a bar chart presenting L-isoleucine concentrations (nanomol/mL) and standard errors thereof in the plasma from five maple syrup urine disease mice (branched-chain alpha-keto acid dehydrogenase (BCKDH) mutant mice, +/Dbt$^{tmlGeh}$; grey solid) and five control mice (white solid);

FIG. 11-15 shows a bar chart presenting L-leucine concentrations (nanomol/mL) and standard errors thereof in the plasma from five maple syrup urine disease mice (branched-chain alpha-keto acid dehydrogenase (BCKDH) mutant mice, +/Dbt$^{tmlGeh}$; grey solid) and five control mice (white solid);

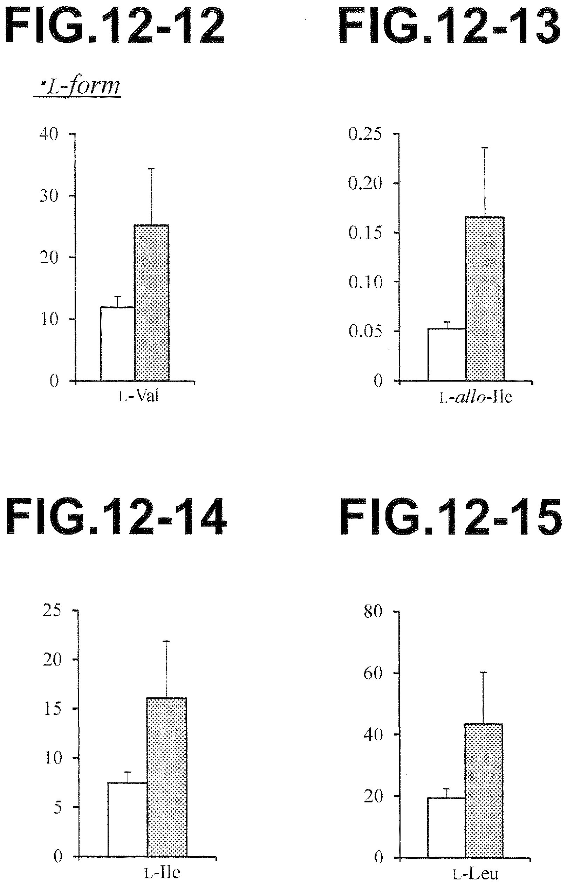

FIG. 12-1 shows a bar chart presenting D-valine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 12-2 shows a bar chart presenting D-allo-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 12-3 shows a bar chart presenting D-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.05;

FIG. 12-4 shows a bar chart presenting D-leucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 12-5 shows a bar chart presenting D-phenylalanine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$;

black solid) and five control mice (white solid);

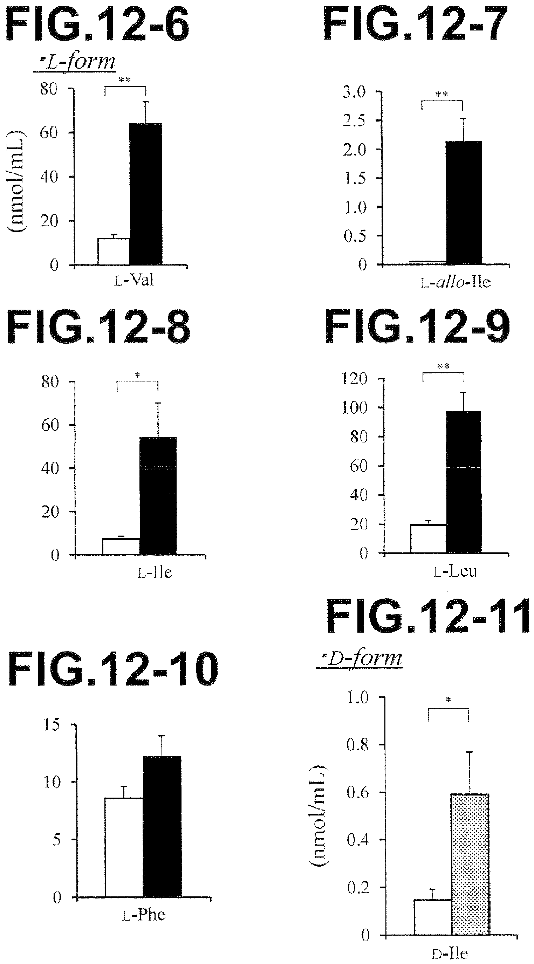

FIG. 12-6 shows a bar chart presenting L-valine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 12-7 shows a bar chart presenting L-allo-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 12-8 shows a bar chart presenting L-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.05;

FIG. 12-9 shows a bar chart presenting L-leucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). In regard to the significant difference, p is less than 0.01;

FIG. 12-10 shows a bar chart presenting L-phenylalanine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid);

FIG. 12-11 shows a bar chart presenting D-isoleucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid). In regard to the significant difference, p is less than 0.05;

FIG. 12-12 shows a bar chart presenting L-valine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid);

FIG. 12-13 shows a bar chart presenting L-allo-isoleucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid);

FIG. 12-14 shows a bar chart presenting L-isoleucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid);

FIG. 12-15 shows a bar chart presenting L-leucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid);

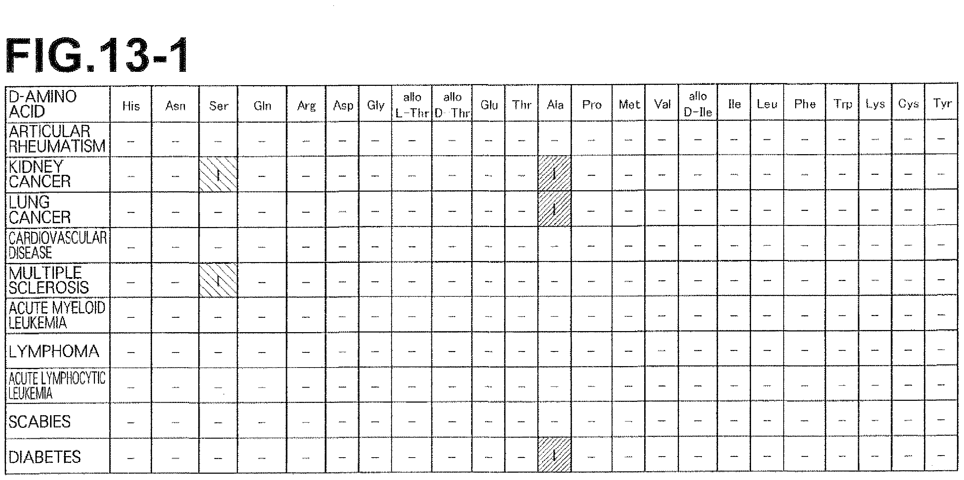

FIG. 13-1 shows an additional Table summarizing analysis results of D-amino acids in each initial sample from various types of disease patients; and

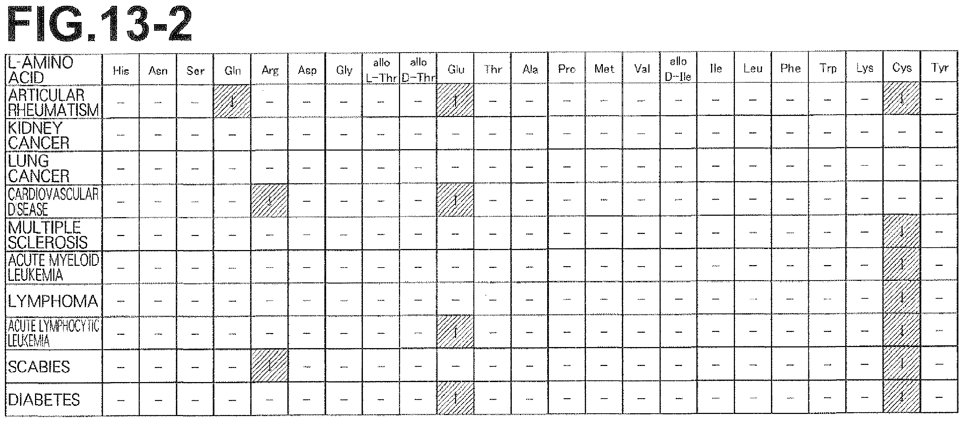

FIG. 13-2 shows an additional Table summarizing analysis results of L-amino acids in each initial sample from various types of disease patients.

DESCRIPTION OF EMBODIMENTS

Examples of the invention described below have been presented for the purpose of illustration only, and are not to be construed as limiting the technical scope of the invention. The technical scope of the present invention is limited solely by the language of the appended claims. Modifications of the present invention such as, for example, additions, deletions and substitutions of features of the present invention may be made without departing from the spirit of the present invention.

The following Examples were conducted in accordance with guidelines from National Institutes of Health (NIH) after an approval by an ethics committee of Shiseido Research Center.

Example 1

Total Analysis of Amino Acid Stereoisomers in Samples Derived From Various Types of Disease Patients (1)

1. Materials and Methods

(1) Sample

Serum samples derived from healthy humans and various types of disease patients were obtained from BioServe Biotechnologies, Ltd., USA, Maryland, Beltsville). The samples were collected by Genomic Collaborative Inc., which was purchased by BioServe Biotechnologies, Ltd. Blood collection was carried out under a control by an attending physician of the patient, and a consent by the blood donor on a document in accordance with United States Health Insurance Portability and Accountability Act (HIPAA) was confirmed. Among the donors of the samples employed in the analyses, healthy individuals selected were four males and four females, all 50 years old Caucasian, not having the past history of the subject disease to be studied. Renal disease patients selected were not suffering from diabetes in combination. Cognitive impairment patients selected were four males who were diagnosed to fall under the severity of stage 5 (stage when necessity for assistance from someone for daily life has started) among 7 stages defined by Alzheimer's disease association. Large intestine cancer patients selected were four male who were diagnosed to fall under stage II. Breast cancer patients selected were four females who were diagnosed to fall under stage II, with the cancer developed on only one side. A Prostate gland cancer patient selected was a male who was diagnosed to fall under stage II, and not treated by any of hormone therapy and chemotherapy. A hepatopathy patient selected was a female who was diagnosed to fall under stage II, with hepatopathy developed on only one side. An osteoporosis patient selected was a female. An ovary cancer patient selected was a female falling under stage II. The donors of the samples used in the present analyses excluded smokers.

(2) Total Analysis of Amino Acid Stereoisomers

The samples were subjected to a total analysis of amino acid stereoisomers by a D, L-amino acid simultaneous and highly sensitive analysis system developed by Zaitsu et. al., (Japanese Patent No. 4291628). Details of analytical conditions of each amino acid are described in Hamase K. et el., J. Chromatogr. A, 1143: 105 (2007); Hamase K., et el., J. Chromatogr. A, 1217: 1056 (2010); and Miyoshi Y, et el., J. Chromatogr. B, 879: 3184 (2011). In brief, the sample was homogenized in 20 times volume of methanol at 4°C and 3,500 rpm, for 2 min using a microhomogenizing system (Micro Smash MS-100R, Tomy Seiko Co., Ltd.), followed by centrifugation at 20,400 x g for 10 min. The centrifugation supernatant in a volume of 10 $\mu$L was dried under reduced pressure at 40°C. To the resulting residue were added 20 $\mu$L of 200 mM sodium borate buffer (pH 8.0), and 5 $\mu$L of a 40 mM NBD-F (4-fluoro-7-nitro-2,1,3-benzooxadiazole, Tokyo Chemical Industry Co., Ltd.) solution in anhydrous methyl cyanide, and the mixture was heated at 60°C, for 2 min. After completion of the reaction, 75 $\mu$L of a 2% (v/v) aqueous trifluoroacetic acid solution was added thereto. This mixture in a volume of 2 $\mu$L was subjected to an HPLC system (NANOSPACE SI-2; Shiseido Co., Ltd., see supplementary information of Sasabe, J. et el., Proc. Natl. Acad. Sci., 109: 627 (2012)). In brief, an analytical column for reverse phase separation employed was a monolithic ODS column (in-house product; internal diameter: 759 mm x 0.53 mm, attached to a quartz glass capillary) which had been incubated at 40°C. As a mobile phase, a mixture containing methyl cyanide, trifluoroacetic acid and water (volume ratio of methyl cyanide: trifluoroacetic acid: water = 5: 0.05: 95) was used. The flow rate was 35 $\mu$L per minute. After the reverse phase separation, a NBD-modified amino acid fraction intended was automatically transferred to an enantio-selective column via a column switching valve to which a 150-$\mu$L loop was attached. For enantiomer separation, a SUMICHIRAL OA-2500S column in which (S)-naphthylglycine is used as a chiral selector (internal diameter: 250 mm x 1.5 mm I.D., packed in-house, material: manufactured by Sumika Chemical Analysis Service, Ltd) was employed. Fluorescent detection was executed with an excitation wavelength of 470 nm and a detection wavelength of 530 nm.

2. Results

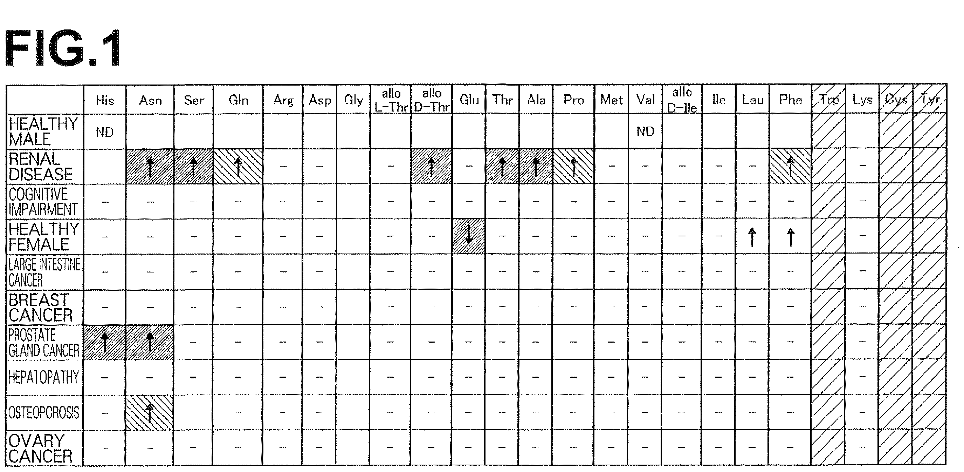

FIG. 1 shows a Table summarizing analysis results of D-amino acids on each initial samples from healthy individuals and various types of disease patients. Each line in the Table shows a disease name of the sample donor, and each row shows the type of D-amino acid analyzed. In Table, ND denotes "being the detection limit or below". Coarse backward diagonal hatching patterns in rows of tryptophan, cysteine and tyrosine indicate that quantitative determination of these amino acids failed on the sample at issue. Upward arrowheads indicate that the sample contained the amino acid in an amount more than that in the samples from healthy males shown in the first line, whereas downward arrowheads indicate that the sample contained the amino acid in an amount less than that in the samples from the healthy males shown in the first line. Fine backward diagonal hatching patterns (for example, asparagine in renal disease samples, etc.) indicate that a ratio of the amount of the amino acid in these samples to the amount of the amino acid in the samples from the healthy males is two times or greater (upward arrowhead), or 1/2 or less (downward arrowhead). Coarse downward diagonal hatching patterns (for example, glutamine in renal disease samples, etc.) indicate that a ratio of the amount of the amino acid in these samples to the amount of the amino acid in the samples from the healthy males is less than two times (upward arrowhead), or less than 1/2 (downward arrowhead).

Among the D-amino acids the serum concentration of which was higher in the samples from renal disease patients than in the samples from healthy males shown in FIG. 1, serine, allo-D-threonine, threonine, alanine, proline and phenylalanine are substrates for D-amino acid oxidase; however, aspartic acid and glutamine are substrates for D-aspartate oxidase. In addition, serum concentrations of histidine, arginine, methyl, valine, allo-D-isoleucine and isoleucine were decided not to vary between the renal disease patients and the healthy males, even though these amino acids are substrates for the same D-amino acid oxidase. Furthermore, although aspartic acid and glutamic acid are substrates for D-aspartate oxidase, it was determined that there was no difference in the serum concentrations of these between the renal disease patients and the healthy males. In these regards, it is impossible to explain the higher serum concentrations of the aforementioned types of D-amino acids in the samples from renal disease patients as compared with the samples from healthy males, on the basis of only the specificity of metabolic enzyme of D-amino acids conventionally known. The D-amino acids the serum concentration of which was higher in the sample from the prostate gland cancer patient than in the samples from healthy males were D-histidine and D-asparagine, and the D-amino acid the serum concentration of which was higher in the sample from the osteoporosis patient than in the samples from healthy males was D-asparagine. Also in these regards, it is impossible to explain the higher serum concentrations of these D-amino acids, on the basis of only the specificity of metabolic enzyme of D-amino acids conventionally known. Both D-amino acid oxidase and D-aspartate oxidase have been known to be localized in proximal tubule of kidney. Thus, these enzyme activities in renal disease patients can be expected to decrease. In this instance, it would be considered that degradation of all D-amino acids that may be substrates of these enzymes is suppressed, whereby the amount thereof in the body increases. However, the amount of only part of the D-amino acids in the body, but not all of the D-amino acids, increases in fact, although the mechanism of this event is not known. It is suggested that a change of the serum concentration of the D-amino acid specific to the diseases can be utilized in the diagnosis for the disease.

FIG. 1 suggests that there is no difference in amount regarding the greater part of types of the D-amino acids between healthy males and various types of disease patients; however, the amount of some types of amino acids was two times or more or 1/2 or less as compared with that in healthy males. Accordingly, focus was first made to a renal disease in which a significant difference was found in comparatively many types of D-amino acids as compared with the samples from healthy males, and thus samples from four donors suffering from the same disease were studied in detail.

FIG. 2-1, FIG. 2-2, FIG. 2-3, FIG. 2-4, FIG. 2-5 and FIG. 2-6 show a distribution chart presenting serum concentrations of D-serine, L-serine, D-threonine, L-threonine, D-alanine and L-alanine, respectively, in each disease sample. The sample 1 was from healthy males; the sample 2 was from renal disease patients; the sample 3 was from cognitive impairment patients; the sample 4 was from healthy females; the sample 5 was from large intestine cancer patients; and the sample 6 was from breast cancer patients. FIG. 2-1 indicates that the serum concentrations of D-serine in healthy male and female individuals, and those in the cognitive impairment, large intestine cancer and breast cancer patients were almost constant and low, whereas the serum concentrations of D-serine in renal disease patients were higher than those in other samples by at least two times or more. FIG. 2-3 indicates that the serum concentrations of D-threonine in the healthy male and female individuals, and those in the cognitive impairment, large intestine cancer and breast cancer patients were almost constant and low, whereas the serum concentrations of D-threonine in renal disease patients were higher than those in other samples by at least two times or more. Similarly, FIG. 2-5 indicates that the serum concentrations of D-alanine in the healthy male and female individuals, and those in the cognitive impairment, large intestine cancer and breast cancer patients were almost constant and low, whereas the serum concentrations of D-alanine in three among four renal disease patients were higher than those in other samples by at least two times or more. To the contrary, FIG. 2-2, FIG. 2-4 and FIG. 2-6 indicate that with respect to any one of L-serine, L-threonine and L-alanine, expanded ranges of distribution of the amounts of the amino acids in healthy individuals, and four types of the disease patients studied in this Example were found due to the individual differences. Moreover, in the samples from the renal disease patients, such remarkable differences as was the case with D-serine, D-threonine and D-alanine were not found in total for L-serine, L-threonine and L-alanine, although the amounts thereof tend to be somewhat smaller than those in the samples from the healthy male and female individuals, and other disease patients.

A characteristic feature of the serum concentrations of D-amino acids clarified from FIG. 2-1 to FIG. 2-6 is that any serum concentration of the D-amino acids in the samples from the healthy male and female individuals was very low, with a small deviation. The same applies to other D-amino acids (data not shown). Furthermore, in the case in which a change of the serum concentrations of D-amino acids in the samples from the patients that correlates with the disease is not found, the serum concentration of any of the D-amino acids was very low, with a small deviation, similarly to the cases of healthy individuals. This feature leads to a lower frequency of false positive results in diagnoses, which presents an important basis for usability in diagnoses according to the amount of the D-amino acid. In addition, when the amount of a D-amino acid that varies specifically to a disease is represented by a parameter combined with other factor, as long as a correlation of the other factor per se with the disease is inferior to that of the amount of the D-amino acid, only a diagnostic characteristic equivalent to that of a diagnosis conducted on the basis only of the amount of the D-amino acid is substantially attained.

FIG. 3-1, FIG. 3-2 and FIG. 3-3 show a distribution chart presenting percentages of the amount of D-form (%D) with respect to the sum of the amounts of the D-form and L-form of serine, threonine and alanine in samples, respectively. FIG. 3-1 indicates that the percentages of the serum concentrations of D-serine with respect to the total serum concentration of serine in the healthy male and female individuals, and those in the cognitive impairment, large intestine cancer and breast cancer patients were almost constant and low, whereas the percentages of the serum concentrations of D-serine with respect to the total serum concentration of serine in renal disease patients were higher than those in other samples by 4 times or more. FIG. 3-2 indicates that the percentages of the serum concentrations of D-threonine with respect to the total serum concentration of threonine in the healthy male and female individuals, and those in the cognitive impairment, large intestine cancer and breast cancer patients were almost constant and low, whereas the percentages of the serum concentrations of D-threonine with respect to the total serum concentration of threonine in renal disease patients were higher than those in other samples by 4 times or more. Similarly, FIG. 3-3 indicates that the percentages of the serum concentrations of D-alanine with respect to the total serum concentration of alanine in the healthy male and female individuals, and those in the cognitive impairment, large intestine cancer and breast cancer patients were almost constant and low, whereas the percentages of the serum concentrations of D-alanine with respect to the total serum concentration of alanine in three among four renal disease patients were higher than those in other samples by 4 times or more.

Example 2

Total Analysis of Amino Acid Stereoisomers in Samples Derived from Mouse Disease Model

1. Materials and Methods

1.1 Total Analysis of Amino Acid Stereoisomers

For the Total analysis of amino acid stereoisomers, a system similar to the D, L-amino acid simultaneous and highly sensitive analysis system explained in Example 1 was used, except that MS not having a damper was used as a liquid feeding pump, and that selection of a secondary mobile phase from a wide range of options was enabled through adopting MPS and a low-dose degasser.

1.2 Mouse Disease Model

The mouse disease models used in this Example were a dilated cardiomyopathy model mouse, a climacterium model mouse resulting from extirpation of the ovary, a sarcoma-transplanted mouse, an Alzheimer's disease model mouse, a DAO deficient mouse and a DDO deficient mouse. Each mouse will be explained in detail below. The experiments in which the mice were used were carried out in Graduate School of Pharmaceutical Sciences, Kyushu University.

1.2.1 Dilated Cardiomyopathy Model Mouse

As the cardiovascular disorder model mouse, MLP-KO mice (Arber, S. et el., Cell, 88: 393 (1997)) that are deficient in MLP (muscle LIM protein) which is one of cardiac muscle constituting proteins were employed. Urine samples from three 8 weeks old male dilated cardiomyopathy model mice (MLP-KO mouse, hereinafter, may be referred to as "diseased"), and four 8 weeks old male control normal mice (hereinafter, may be referred to as "normal") were obtained, and the total analysis of amino acid optical isomer contents was carried out.

1.2.2 Climacterium Model Mouse

As the climacterium model mouse, the ovary was extirpated from 9 weeks old female HR-1 mice under anesthesia, and the skin was sutured. Also, mice for a control experiment were prepared from the female mice of the same weeks old, through subjecting only to skin incision and suture without subjecting to the extirpation of the ovary. The body weight was measured before the operation, and also one to four weeks after the operation. The individuals whose body weight increase was found to be greater than that of the control mice were subjected to the total analysis of amino acid optical isomer contents in urine as climacterium model mice.

1.2.3 Sarcoma-Transplanted Mouse

As the sarcoma-transplanted mouse, 6 weeks old male ICR mice transplanted with $2 \times 10^7$ sarcoma cells were prepared. Three weeks after the transplantation, the individuals in which growth of the explanted tumor was verified were subjected to the total analysis of amino acid optical isomer contents in urine. As a control experiment of the sarcoma-transplanted mouse, 9 weeks old male ICR mice fed in the same environment were subjected to the total analysis of amino acid optical isomer contents in urine.

1.2.4 Alzheimer's Disease Model Mouse

As the Alzheimer's disease model mouse, 8 weeks old male hemizygote mice of an amyloid β precursor protein-highly expressing transgenic mouse Tg2576 (Hsiao, K. et el., Science, 274: 99 (1996)) were subjected to the total analysis of amino acid optical isomer contents in urine. 8 weeks old male C57BL mice were subjected to the total analysis of amino acid optical isomer contents in urine as control normal mice.

1.2.5 D-Amino Acid Oxidase (DAO) Deficient Mouse

As one of D-amino acid metabolism-related enzyme activity-altered models, 8 weeks old male D-amino acid oxidase (DAO) deficient mice (Konno, R. et el., Genetics 103: 277 (1983), ddY/DAO-, hereinafter, may be referred to as "DAO-/-") were subjected to the total analysis of amino acid optical isomer contents in urine. As control mice, 8 weeks old male D-amino acid oxidase (DAO) wild type mice were subjected to the total analysis of amino acid optical isomer contents in urine.

1.2.6 D-Aspartate Oxidase (DDO) Deficient Mouse

As one of D-amino acid metabolism-related enzyme activity-altered models, 8 weeks old male D-aspartate oxidase (DDO) deficient mice (Huang, A.S. et el., J. Neurosci., 26: 2814 (2006), hereinafter, may be referred to as "DDO-") were subjected to the total analysis of amino acid optical isomer contents in urine. As control mice, 8 weeks old male D-aspartate oxidase (DDO) wild type mice (hereinafter, may be referred to as "DDO+") were subjected to the total analysis of amino acid optical isomer contents in urine.

1.2.7 Phenylketonuria Disease Model Mouse

As one of amino acid metabolic disorder model mice, five 25-35 weeks old male phenylketonuria disease model mice (phenylalanine hydroxylase (PAH) mutant mouse, $Pah^{enu2}/Pah^{enu2}$, Shedlovsky, A. et el., Genetics 134: 1205 (1993)) fed under an SPF condition were subjected to the total analysis of amino acid optical isomer contents in urine. As control mice, five 25-35 weeks old male wild type allele homozygote in an identical genetic background, fed under the SPF condition were subjected to the total analysis of amino acid optical isomer contents in urine.

1.2.8 Maple Syrup Urine Disease Model Mouse

As one of amino acid metabolic disorder model mice, five 8-10 weeks old male branched-chain alpha-keto acid dehydrogenase (BCKDH) mutant mouse, $Dbt^{tmlGeh}/Dbt^{tmlGeh}$, Homanics GE. et el., BMC Med Genet, 7: 33 (2006) fed under an SPF condition were subjected to the total analysis of amino acid optical isomer contents in urine. As control mice, five 8-10 weeks old male wild type allele homozygote in an identical genetic background, fed under the SPF condition were subjected to the total analysis of amino acid optical isomer contents in urine. In a part of the experiments, five 8-10 weeks old male $+/Dbt^{tmlGeh}$ heterozygotes fed under the SPF condition as intermediate type mice were subjected to the total analysis of amino acid optical isomer contents in urine.

2. Results

2.1 dilated cardiomyopathy model mouse

FIG. 4-1 shows a bar chart presenting averages and standard errors of D-serine concentrations in the urine from three dilated cardiomyopathy model mice (MLP-KO mouse, hereinafter, may be referred to as "diseased") and four control normal mice (hereinafter, may be referred to as "normal"). The ordinate indicates the D-serine concentration (nanomol/mL). FIG. 4-2 shows a bar chart presenting averages and standard errors of L-serine concentrations in the urine from three dilated cardiomyopathy model mice and four control normal mice. The ordinate indicates the L-serine concentration (nanomol/mL). FIG. 4-3 shows a bar chart presenting averages and standard errors of total serine concentrations (the sums of D-serine concentration and L-serine concentration) in the urine from three dilated cardiomyopathy model mice and four control normal mice. The ordinate indicates the total serine concentration (nanomol/mL). FIG. 4-4 shows a bar chart presenting averages and standard errors of the percentages of D-serine concentration (%D) with respect to the total serine concentration in the urine from three dilated cardiomyopathy model mice and four control normal mice. The ordinate indicates the %D. In regard to the significant difference between the normal and the diseased, P was less than 0.02 according to a Student's t-test. FIG. 4-5 shows a bar chart presenting averages and standard errors of D-arginine concentrations in the urine from three dilated cardiomyopathy model mice and four control normal mice. The ordinate indicates the D-arginine concentration (nanomol/mL). FIG. 4-6 shows a bar chart presenting averages and standard errors of L-arginine concentrations in the urine from three dilated cardiomyopathy model mice and four control normal mice. The ordinate indicates the L-arginine concentration (nanomol/mL). In regard to the significant difference between the normal and the diseased, P was less than 0.01 according to a Student's t-test. FIG. 4-7 shows a bar chart presenting averages and standard errors of total arginine concentrations (the sums of D-arginine concentration and L-arginine concentration) in the urine from three dilated cardiomyopathy model mice and four control normal mice. The ordinate indicates the total arginine concentration (nanomol/mL). FIG. 4-8 shows a bar chart presenting averages and standard errors of D-glutamic acid concentrations in the urine from three dilated cardiomyopathy model mice and four control normal mice. The ordinate indicates the D-glutamic acid concentration (nanomol/mL). In regard to the significant difference between the normal and the diseased, P was less than 0.02 according to a Student's t-test. FIG. 4-9 shows a bar chart presenting averages and standard errors of the L-glutamic acid concentrations in the urine from three dilated cardiomyopathy model mice and four control normal mice. The ordinate indicates the L-glutamic acid concentration (nanomol/mL). FIG. 4-10 shows a bar chart presenting averages and standard errors of total glutamic acid concentrations (the sums of D-glutamic acid concentration and L-glutamic acid concentration) in the urine from three dilated cardiomyopathy model mice and four control normal mice. The ordinate indicates the total glutamic acid concentration (nanomol/mL). FIG. 4-11 shows a bar chart presenting averages and standard errors of D-proline concentrations in the urine from three dilated cardiomyopathy model mice and four control normal mice. The ordinate indicates the D-proline concentration (nanomol/mL). In regard to the significant difference between the normal and the diseased, P was less than 0.01 according to a Student's t-test. FIG. 4-12 shows a bar chart presenting averages and standard errors of L-proline concentrations in the urine from three dilated cardiomyopathy model mice and four control normal mice. The ordinate indicates the L-proline concentration (nanomol/mL). FIG. 4-13 shows a bar chart presenting averages and standard errors of total proline concentrations (the sums of D-proline concentration and L-proline concentration) in the urine from three dilated cardiomyopathy model mice and four control normal mice. The ordinate indicates the total proline concentration (nanomol/mL). FIG. 4-14 shows a bar chart presenting averages and standard errors of D-lysine concentrations in the urine from three dilated cardiomyopathy model mice and four control normal mice. The ordinate indicates the D-lysine concentration (nanomol/mL). In regard to the significant difference between the normal and the diseased, P was less than 0.01 according to a Student's t-test. FIG. 4-15 shows a bar chart presenting averages and standard errors of L-lysine concentrations in the urine from three dilated cardiomyopathy model mice and four control normal mice. The ordinate indicates the L-lysine concentration (nanomol/mL). FIG. 4-16 shows a bar chart presenting averages and standard errors of total lysine concentrations (the sums of D-lysine concentration and L-lysine concentration) in the urine from three dilated cardiomyopathy model mice and four control normal mice. From these results, the D-serine concentration tended to be lower in the diseased group than in the normal group, but a significant difference was not found. However, according to evaluations on the basis of "%D", %D of D-serine was significantly smaller in the diseased group than in the normal group. The L-arginine concentration was significantly lower in the diseased group than in the normal group. The D-glutamic acid concentration was significantly higher in the diseased group than in the normal group. The D-proline concentration was significantly lower in the diseased group than in the normal group. The D-lysine concentration was significantly lower in the diseased group than in the normal group.

2.2 Climacterium Model Mouse

FIG. 5-1 shows a graph presenting the changes in averages and standard deviations of the body weights of 12 climacterium model mice (hereinafter, may be referred to as "OVX") obtained by extirpation of the ovary from 9 weeks old HR-1 mice, and six control mice (hereinafter, may be referred to as "sham") subjected to only skin incision and suture without carrying out the extirpation of the ovary from female mice of the same weeks old. The ordinate indicates the body weight (gram) of the mice. The black solid bars and the diagonally hatched bars show the average and standard deviation of the body weights of: before the operation (9 weeks old) and one week (10 weeks old), two weeks (11 weeks old), three weeks (12 weeks old) and four weeks (13 weeks old) after the operation of both climacterium model mice (OVX) and control mice (sham), respectively. In regard to the significant difference of the body weights between the climacterium model mice (OVX) and the control mice (sham), P was less than 0.05 (*) or less than 0.01 (**) according to a Student's t-test. In climacterium model mice obtained by extirpation of the ovary, the body weight increase was significantly greater than that of the control mice from two weeks after the operation, suggesting that the climacterium model mice were successfully produced.

FIGs. 5-2, 5-3 and 5-4 show Tables presenting results of the total analysis of amino acid optical isomer contents in the urine from the first mouse (OVX-3), the second mouse (OVX-4) and the third mouse (OVX-5), respectively, among three climacterium model mice (OVX). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis. FIGs. 5-5, 5-6, 5-7 and 5-8 show Tables presenting results of the total analysis of amino acid optical isomer contents in the urine from the first mouse (Sham-16), the second mouse (Sham-17), the third mouse (Sham-19) and the fourth mouse (Sham-20), respectively, among four control mice (sham). FIG. 5-9 shows a Table comparing D-amino acid contents in the urine from three climacterium model mice (OVX) and four control mice (sham). The D-aspartic acid content was lower in the control mice (sham) than in the climacterium model mice (OVX), and in regard to the significant difference, P was 0.015 according to a Student's t-test. FIG. 5-10 shows a Table comparing L-amino acid contents in the urine from three climacterium model mice (OVX) and four control mice (sham). The contents of L-histidine and L-phenylalanine were lower in the climacterium model mice (OVX) than in the control mice (sham), and in regard to the significant difference, P was 0.017 and 0.037, respectively, according to a Student's t-test. FIG. 5-11 shows a Table comparing percentages of the D-form concentration (%D) with respect to the sum of the D-form concentration and the L-form concentration of each amino acid in the urine from three climacterium model mice (OVX) and four control mice (sham). Since D-forms of threonine and isoleucine turn to be allo-forms in a living body, the %D was evaluated in terms of the percentage of the D-allo-form concentration with respect to the sum of the D-allo-form concentration and the L-form concentration. The percentage of D-aspartic acid was lower in the climacterium model mice (OVX) than in the control mice (sham), and in regard to the significant difference, P was 0.002 according to a Student's t-test. FIG. 5-12 shows a Table comparing percentages of each D-amino acid concentration (%D/total L) with respect to the sum of the concentrations of all the L-amino acids in the urine from three climacterium model mice (OVX) and four control mice (sham). The percentage of D-aspartic acid was lower in the climacterium model mice (OVX) than in the control mice (sham), and in regard to the significant difference, P was 0.005 according to a Student's t-test. FIG. 5-13 shows a Table comparing percentages of each D-amino acid concentration (%D/total D) with respect to the sum of the concentrations of all the D-amino acids in the urine from three climacterium model mice (OVX) and four control mice (sham). The percentage of D-aspartic acid was lower in the climacterium model mice (OVX) than in the control mice (sham), and in regard to the significant difference, P was 0.017 according to a Student's t-test. FIG. 5-14 shows a Table comparing percentages of each D-amino acid concentration with respect to the D-glutamic acid concentration (%D/D-Glu) in the urine from three climacterium model mice (OVX) and four control mice (sham). Since D-aspartic acid is an acidic D-amino acid, an evaluation was made after a correction with the concentration of D-glutamic acid which is considered to be similarly metabolized. Also in the case of %D/D-Glu, The percentage of D-aspartic acid was lower in the climacterium model mice (OVX) than in the control mice (sham), and in regard to the significant difference, P was 0.006 according to a Student's t-test.

2.3 Sarcoma-Transplanted Mouse

FIGs. 6-1, 6-2 and 6-3 show Tables presenting results of the total analysis of amino acid optical isomer contents in the urine from the first mouse (S3), the second mouse (S4) and the third mouse (S5), respectively, among three 6 weeks old male ICR mice transplanted with $2 \times 10^7$ sarcoma cells in which growth of the explanted tumor was verified three weeks after the transplantation. Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis. FIGs. 6-4, 6-5 and 6-6 show Tables presenting results of the total analysis of amino acid optical isomer contents in the urine from the first mouse (C3), the second mouse (C4) and the third mouse (C5), respectively, among three 9 weeks old male ICR mice fed for a control experiment in the same environment as that of the sarcoma-transplanted mice. FIG. 6-7 shows a Table comparing D-amino acid contents in the urine from three sarcoma-transplanted mice (S3, S4 and S5) and three control mice (C3, C4 and C5). FIG. 6-8 shows a Table comparing L-amino acid contents in the urine from three sarcoma-transplanted mice (S3, S4 and S5) and three control mice (C3, C4 and C5). FIG. 6-9 shows a Table comparing percentages of the D-form concentration (%D) with respect to the sum of the D-form concentration and the L-form concentration of each amino acid in the urine from three sarcoma-transplanted mice (S3, S4 and S5) and three control mice (C3, C4 and C5). The %D of serine was lower in the sarcoma-transplanted mice than in the control mice, and in regard to the significant difference, P was 0.016 according to a Student's t-test. FIG. 6-10 shows a Table comparing percentages of each D-amino acid concentration (%D/total L) with respect to the sum of the concentrations of all the L-amino acids in the urine from three sarcoma-transplanted mice (S3, S4 and S5) and three control mice (C3, C4 and C5). FIG. 6-11 shows a Table comparing percentages of each D-amino acid concentration (%D/total D) with respect to the sum of the concentrations of all the D-amino acids in the urine from three sarcoma-transplanted mice (S3, S4 and S5) and three control mice (C3, C4 and C5). The percentages of D-asparagine and D-arginine tended to be greater in the sarcoma-transplanted mice than in the control mice, and in regard to the significant difference of D-arginine, P was 0.035 according to a Student's t-test. FIG. 6-12 shows a Table comparing percentages of each D-amino acid concentration with respect to the D-asparagine concentration (%D/D-Asn) in the urine from three sarcoma-transplanted mice (S3, S4 and S5) and three control mice (C3, C4 and C5). D-asparagine was used as a basis for a correction of the concentration in the urine since D-asparagine is present in mammalian urine at a comparatively high concentration, and the proportion with respect to the total D-amino acid concentrations was most stable. The percentages of D-arginine tended to be greater in the sarcoma-transplanted mice than in the control mice, and in regard to the significant difference, P was 0.016 according to a Student's t-test.

2.4 Alzheimer's disease model mouse

FIG. 7-1 shows a bar chart presenting averages and standard errors of D-serine concentrations (D-Ser), L-serine concentrations (L-Ser) and the sum of both concentrations (Ser) in the urine from three Alzheimer's disease model mice (8 weeks old male hemizygote mice of an amyloid β precursor protein-highly expressing transgenic mouse Tg2576, hereinafter, may be referred to as "hemi") and three control normal mice (C57BL, hereinafter, may be referred to as "Wild"). The ordinate indicates the concentration (nanomol/mL). FIG. 7-2 shows a bar chart presenting averages and standard errors of the percentages of D-serine concentration (%D) with respect to the total serine concentration in the urine from three Alzheimer's disease model mice (hemi) and three control normal mice (Wild). The ordinate indicates the %D. FIG. 7-3 shows a graph presenting a relative ratio of the D-serine concentration to the D-allo-threonine concentration (D-Ser/D-allo-Thr) or a relative ratio of the D-serine concentration to the D-allo-isoleucine concentration (D-Ser/D-allo-Ile) in the urine from three Alzheimer's disease model mice (hemi) and three control normal mice (Wild). Both the relative ratio of the D-serine concentration to the D-allo-threonine concentration in the urine, and the relative ratio of the D-serine concentration to the D-allo-isoleucine concentration in the urine were higher in the Alzheimer's disease model mice than in the control mice, and in regard to the significant difference, P was less than 0.01 according to a Student's t-test in both cases. FIG. 7-4 shows a bar chart presenting averages and standard errors of D-alanine concentrations (D-Ala), L-alanine concentrations (L-Ala) and the sum of both concentrations (Ala) in the urine from three Alzheimer's disease model mice (hemi) and three control normal mice (Wild). The ordinate indicates the concentration (nanomol/mL). The D-alanine concentration in the urine was higher in the Alzheimer's disease model mice than in the control normal mice, and in regard to the significant difference, P was less than 0.01 according to a Student's t-test. FIG. 7-5 shows a bar chart presenting averages and standard errors of the percentages of D-alanine concentration (%D) with respect to the total alanine concentration in the urine from three Alzheimer's disease model mice (hemi) and three control normal mice (Wild). The ordinate indicates the %D. The percentage of the D-alanine concentration (%D) with respect to the total alanine concentration in the urine was higher in the Alzheimer's disease model mice than in the control normal mice , and in regard to the significant difference, P was less than 0.01 according to a Student's t-test. FIG. 7-6 shows a bar chart presenting averages and standard errors of D-methionine concentrations (D-Met), L-methionine concentrations (L-Met) and the sum of both concentrations (Met) in the urine from three Alzheimer's disease model mice (hemi) and three control normal mice (Wild). The ordinate indicates the concentration (nanomol/mL). The D-Met concentration in the urine tended to be higher in the Alzheimer's disease model mice than in the control normal mice. FIG. 7-7 shows a bar chart presenting averages and standard errors of the percentages of D-methionine concentration (%D) with respect to the total serine concentration in the urine from three Alzheimer's disease model mice (hemi) and three control normal mice (Wild). The ordinate indicates the %D. FIG. 7-8 shows a graph presenting a relative ratio of the D-methionine concentration to the D-allo-threonine concentration (D-Met/D-allo-Thr) or a relative ratio of the D-methionine concentration to the D-allo-isoleucine concentration (D-Met/D-allo-Ile) in the urine from three Alzheimer's disease model mice (hemi) and three control normal mice (Wild). Both the relative ratio of the D-methionine concentration to the D-allo-threonine concentration in the urine, and the relative ratio of the D-methionine concentration to the D-allo-isoleucine concentration in the urine were higher in the Alzheimer's disease model mice than in the control mice, and in regard to the significant difference, P was less than 0.05 according to a Student's t-test in both cases. FIG. 7-9 shows a bar chart presenting averages and standard errors of D-leucine concentrations (D-Leu), L-leucine concentrations (L-Leu) and the sum of both concentrations (Leu) in the urine from three Alzheimer's disease model mice (hemi) and three control normal mice (Wild). The ordinate indicates the concentration (nanomol/mL). FIG. 7-10 shows a bar chart presenting averages and standard errors of the percentages of D-leucine concentration (%D) with respect to the total serine concentration in the urine from three Alzheimer's disease model mice (hemi) and three control normal mice (Wild). FIG. 7-11 shows a graph presenting a relative ratio of the D-leucine concentration to the D-allo-threonine concentration (D-Leu/D-allo-Thr) or a relative ratio of the D-leucine concentration to the D-allo-isoleucine concentration (D-Leu/D-allo-Ile) in the urine from three Alzheimer's disease model mice (hemi) and three control normal mice (Wild). Both the relative ratio of the D-leucine concentration to the D-allo-threonine concentration in the urine, and the relative ratio of the D-leucine concentration to the D-allo-isoleucine concentration in the urine were higher in the Alzheimer's disease model mice than in the control mice, and in regard to the significant difference, P was less than 0.05 and less than 0.01, respectively, according to a Student's t-test. FIG. 7-12 shows a bar chart presenting averages and standard errors of D-aspartic acid concentrations (D-Asp), L-aspartic acid concentrations (L-Asp) and the sum of both concentrations (Asp) in the urine from three Alzheimer's disease model mice (hemi) and three control normal mice (Wild). The ordinate indicates the concentration (nanomol/mL). The D-aspartic acid concentration in the urine was lower in the Alzheimer's disease model mice than in the control normal mice, and in regard to the significant difference, P was less than 0.05 according to a Student's t-test. FIG. 7-13 shows a bar chart presenting averages and standard errors of the percentages of the D-aspartic acid concentration (%D) with respect to the total aspartic acid concentration in the urine from three Alzheimer's disease model mice (hemi) and three control normal mice (Wild). The ordinate indicates the %D. FIG. 7-14 shows a bar chart presenting averages and standard errors of D-phenylalanine concentrations (D-Phe), L-phenylalanine concentrations (L-Phe) and the sum of both concentrations (Phe) in the urine from three Alzheimer's disease model mice (hemi) and three control normal mice (Wild). The ordinate indicates the concentration (nanomol/mL). FIG. 7-15 shows a bar chart presenting averages and standard errors of the percentages of D-phenylalanine concentration (%D) with respect to the total phenylalanine concentration in the urine from three Alzheimer's disease model mice (hemi) and three control normal mice (Wild). FIG. 7-16 shows a graph presenting a relative ratio of the D-phenylalanine concentration to the D-allo-threonine concentration (D-Phe/D-allo-Thr) or a relative ratio of the D-phenylalanine concentration to the D-allo-isoleucine concentration (D-Phe/D-allo-Ile) in the urine from three Alzheimer's disease model mice (hemi) and three control normal mice (Wild). The relative ratio of the D-phenylalanine concentration to the D-allo-isoleucine concentration in the urine was higher in the Alzheimer's disease model mice than in the control mice, and in regard to the significant difference, P was less than 0.05 according to a Student's t-test.

2.5 D-amino acid oxidase (DAO) deficient mouse

FIGs. 8-1, 8-2 and 8-3 show Tables presenting results of the total analysis of amino acid optical isomer contents in the urine from the first mouse (DAO+/+ 1), the second mouse (DAO+/+ 2) and the third mouse (DAO+/+ 3), respectively, among three D-amino acid oxidase (DAO) wild type mice (ddY/DAO+, which may be referred to as "DAO+/+"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis. FIGs. 8-4, 8-5 and 8-6 show Tables presenting results of the total analysis of amino acid optical isomer contents in the urine from the first mouse (DAO-/- 1), the second mouse (DAO-/- 2) and the third mouse (DAO-/- 3), respectively, among three D-amino acid oxidase (DAO) deficient mice (ddY/DAO-, hereinafter, may be referred to as "DAO-/-"). Since no optical isomer of glycine is present, the results on glycine are described in a column of "L-form". All amino acids were fluorescent-derivatized with NBD-F, and analyzed using the apparatus for the total analysis of amino acid optical isomer contents of the embodiment of the present invention. Since the NBD derivative of tryptophan is poorly sensitive, "nd" was designated in this analysis. Since cysteine produces cystine by air oxidization, the content of cysteine is extremely low, and thus "nd" was designated in this analysis. FIG. 8-7 shows a Table comparing D-amino acid contents in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The contents of D-serine, D-allo-threonine, D-alanine, D-proline, D-leucine and D-phenylalanine were significantly higher in the D-amino acid oxidase (DAO) enzyme deficient mice than in the DAO wild type mice. FIG. 8-8 shows a Table comparing L-amino acid contents in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The contents of D-serine, D-allo-threonine, D-alanine, D-proline, D-leucine and D-phenylalanine were significantly higher in the D-amino acid oxidase (DAO) enzyme deficient mice than in the DAO wild type mice. FIG. 8-9 shows a Table comparing percentages of the D-form concentration (%D) with respect to the sum of the D-form concentration and the L-form concentration of each amino acid in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-proline, D-leucine and D-phenylalanine were significantly higher in the D-amino acid oxidase (DAO) enzyme deficient mice than in the DAO wild type mice. FIG. 8-10 shows a Table comparing percentages of each D-amino acid concentration (%D/total L) with respect to the sum of the concentrations of all the L-amino acids in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-leucine and D-phenylalanine were significantly higher in the D-amino acid oxidase (DAO) enzyme deficient mice than in the DAO wild type mice. FIG. 8-11 shows a Table comparing percentages of each D-amino acid concentration (%D/total D) with respect to the sum of the concentrations of all the D-amino acids in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). FIG. 8-12 shows a Table comparing percentages of each D-amino acid concentration (%D/D-Asn) with respect to the D-asparagine concentration in the urine from three D-amino acid oxidase (DAO) wild type mice (DAO+/+ 1, DAO+/+ 2 and DAO+/+ 3) and three D-amino acid oxidase (DAO) deficient mice (DAO-/- 1, DAO-/- 2 and DAO-/- 3). The percentages of D-serine, D-allo-threonine, D-alanine, D-proline, D-leucine and D-phenylalanine were significantly higher in the D-amino acid oxidase (DAO) enzyme deficient mice than in the DAO wild type mice.

2.6 D-Aspartate Oxidase (DDO) Deficient Mouse

FIGs. 9-1, 9-2, 9-3 and 9-4 show wave form charts presenting results of the total analysis of amino acid optical isomer contents in the urine from the first mouse, the second mouse, the third mouse and the fourth mouse, respectively, among four D-aspartate oxidase (DDO) wild type mice (DDO+). FIGs. 9-5, 9-6, 9-7 and 9-8 show wave form charts presenting results of the total analysis of amino acid optical isomer contents in the urine from the first mouse, the second mouse, the third mouse and the fourth mouse, respectively, among four D-aspartate oxidase (DDO) deficient mice (DDO-). The D-asparagine concentration was higher in the urine from the DDO deficient mice than in the urine from the wild type mice, and the concentrations of D-aspartic acid and D-arginine were also higher in the urine from the DDO deficient mice. D-glutamic acid which is a good substrate for the DDO enzyme was scarcely found in the urine from the DDO deficient mice.

From the results discussed in 2.5 and 2.6 above, deficiency of DAO and DDO that are D-amino acid metabolic enzymes resulted in change in the concentrations of the D-amino acids specific to these enzymes, respectively. In other words, it is believed that the specific D-amino acid concentrations can be controlled by controlling the activities of these enzymes.

2.7 Phenylketonuria Disease Model Mouse

FIG. 10-1 shows a bar chart presenting D-valine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-2 shows a bar chart presenting D-allo-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-3 shows a bar chart presenting D-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-4 shows a bar chart presenting D-leucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-5 shows a bar chart presenting D-phenylalanine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). The D-phenylalanine concentration was higher in the phenylketonuria disease model mice than in the control mice, and in regard to the significant difference, p was less than 0.01. FIG. 10-6 shows a bar chart presenting L-valine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-7 shows a bar chart presenting L-allo-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-8 shows a bar chart presenting L-isoleucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-9 shows a bar chart presenting L-leucine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). FIG. 10-10 shows a bar chart presenting L-phenylalanine concentrations (nanomol/mL) and standard errors thereof in the plasma from five phenylketonuria disease model mice (phenylalanine hydroxylase (PAH) mutant mice, Pah$^{enu2}$/Pah$^{enu2}$; black solid) and five control mice (white solid). The concentration of L-phenylalanine was higher in the phenylketonuria disease model mice than in the control mice, and in regard to the significant difference, p was less than 0.01.

FIG. 10-11 shows a bar chart presenting D-valine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-12 shows a bar chart presenting D-allo-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-13 shows a bar chart presenting D-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-14 shows a bar chart presenting D-leucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-15 shows a bar chart presenting D-phenylalanine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-16 shows a bar chart presenting L-valine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-17 shows a bar chart presenting L-allo-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-18 shows a bar chart presenting L-isoleucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-19 shows a bar chart presenting L-leucine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). FIG. 10-20 shows a bar chart presenting L-phenylalanine concentrations (nanomol/mL) and standard errors thereof in the urine from five phenylketonuria disease model mice (phenylalanine hydroxylase (PAH) mutant mice, Pah$^{enu2}$/Pah$^{enu2}$; black solid) and five control mice (white solid). The L-phenylalanine concentration was higher in the phenylketonuria disease model mice than in the control mice, and in regard to the significant difference, p was less than 0.01.

2.8 Maple Syrup Urine Disease Model Mouse

FIG. 11-1 shows a bar chart presenting D-valine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 11-2 shows a bar chart presenting D-allo-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 11-3 shows a bar chart presenting D-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The D-isoleucine concentration in the plasma was higher in the maple syrup urine disease mice than in the control mice, and in regard to the significant difference, p was less than 0.05. FIG. 11-4 shows a bar chart presenting D-leucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 11-5 shows a bar chart presenting D-phenylalanine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 11-6 shows a bar chart presenting L-valine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-valine concentration in the plasma was higher in the maple syrup urine disease mice than in the control mice, and in regard to the significant difference, p is less than 0.01. FIG. 11-7 shows a bar chart presenting L-allo-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-allo-isoleucine concentration in the plasma was higher in the maple syrup urine disease mice than in the control mice, and in regard to the significant difference, p was less than 0.01. FIG. 11-8 shows a bar chart presenting L-isoleucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-isoleucine concentration in the plasma was higher in the maple syrup urine disease mice than in the control mice, and in regard to the significant difference, p was less than 0.01. FIG. 11-9 shows a bar chart presenting L-leucine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-leucine concentration in the plasma was higher in the maple syrup urine disease mice than in the control mice, and in regard to the significant difference, p was less than 0.05. FIG. 11-10 shows a bar chart presenting L-phenylalanine concentrations (nanomol/mL) and standard errors thereof in the plasma from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 11-11 shows a bar chart presenting D-isoleucine concentrations (nanomol/mL) and standard errors thereof in the plasma from five maple syrup urine disease intermediate type mice (branched-chain alpha-keto acid dehydrogenase (BCKDH) mutant mice, +/Dbt$^{tmlGeh}$; grey solid) and five control mice (white solid). The D-isoleucine concentration in the plasma was higher in the maple syrup urine disease mice than in the control mice, and in regard to the significant difference, p was less than 0.01. FIG. 11-12 shows a bar chart presenting L-valine concentrations (nanomol/mL) and standard errors thereof in the plasma from five maple syrup urine disease intermediate type mice (branched-chain alpha-keto acid dehydrogenase (BCKDH) mutant mice, +/Dbt$^{tmlGeh}$; grey solid) and five control mice (white solid). FIG. 11-13 shows a bar chart presenting L-allo-isoleucine concentrations (nanomol/mL) and standard errors thereof in the plasma from five maple syrup urine disease intermediate type mice (branched-chain alpha-keto acid dehydrogenase (BCKDH) mutant mice, +/Dbt$^{tmlGeh}$; grey solid) and five control mice (white solid). 11-14 shows a bar chart presenting L-isoleucine concentrations (nanomol/mL) and standard errors thereof in the plasma from five maple syrup urine disease intermediate type mice(branched-chain alpha-keto acid dehydrogenase (BCKDH) mutant mice, +/Dbt$^{tmlGeh}$; grey solid) and five control mice (white solid). 11-15 shows a bar chart presenting L-leucine concentrations (nanomol/mL) and standard errors thereof in the plasma from five maple syrup urine disease intermediate type mice (branched-chain alpha-keto acid dehydrogenase (BCKDH) mutant mice, +/Dbt$^{tmlGeh}$; grey solid) and five control mice (white solid).

12-1 shows a bar chart presenting D-valine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). 12-2 shows a bar chart presenting D-allo-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). FIG. 12-3 shows a bar chart presenting D-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The D-isoleucine concentration in the urine was higher in the maple syrup urine disease mice than in the control mice, and in regard to the significant difference, p was less than 0.05. 12-4 shows a bar chart presenting D-leucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). 12-5 shows a bar chart presenting D-phenylalanine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). 12-6 shows a bar chart presenting L-valine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-valine concentration in the urine was higher in the maple syrup urine disease mouse than in the control mouse, and in regard to the significant difference, p was less than 0.01. 12-7 shows a bar chart presenting L-allo-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-allo-isoleucine concentration in the urine was higher in the maple syrup urine disease mouse than in the control mouse, and in regard to the significant difference, p was less than 0.01. 12-8 shows a bar chart presenting L-isoleucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-isoleucine concentration in the urine was higher in the maple syrup urine disease mouse than in the control mouse, and in regard to the significant difference, p was less than 0.05. FIG. 12-9 shows a bar chart presenting L-leucine concentrations (nanomol/mL) and standard errors thereof in the urine from five maple syrup urine disease mice (branched-chain alpha-keto acid dehydrogenase (BCKDH) mutant mice, Dbt$^{tmlGeh}$/Dbt$^{tmlGeh}$; black solid) and five control mice (white solid). The L-leucine concentration in the urine was higher in the maple syrup urine disease mouse than in the control mouse, and in regard to the significant difference, p was less than 0.01. FIG. 12-11 shows a bar chart presenting D-isoleucine concentrations (nanomol/mL) and standard errors thereof in the urine from five maple syrup urine disease intermediate type mice (branched-chain alpha-keto acid dehydrogenase (BCKDH) mutant mice, +/Dbt$^{tmlGeh}$; grey solid) and five control mice (white solid). The D-isoleucine concentration in the urine was higher in the maple syrup urine disease intermediate type mice than in the control mice, and in regard to the significant difference, p was less than 0.05. FIG. 12-12 shows a bar chart presenting L-valine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid). FIG. 12-13 shows a bar chart presenting L-allo-isoleucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid). FIG. 12-14 shows a bar chart presenting L-isoleucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid). FIG. 12-15 shows a bar chart presenting L-leucine concentrations (nanomol/mL) and standard errors thereof in the urine from five maple syrup urine disease mice (branched-chain alpha-keto acid dehydrogenase (BCKDH) mutant mice, +/Dbt$^{tmlGeh}$; grey solid) and five control mice (white solid).

Example 3

Total Analysis of Amino Acid Stereoisomers in Samples Derived from Various Types of Disease Patients (2)

1. Materials and Methods

(1) Samples

Similarly to Example 1, serum samples derived from various types of disease patients were obtained from BioServe Biotechnologies, Ltd., USA, Maryland, Beltsville). The samples were collected by Genomic Collaborative Inc., which was purchased by BioServe Biotechnologies, Ltd. Blood collection was carried out under a control by an attending physician of the patient, and a consent by the blood donor on a document in accordance with United States Health Insurance Portability and Accountability Act (HIPAA) was confirmed. An articular rheumatism sample derived from a 49 years old Caucasian male in a some movable disease state. A kidney cancer sample derived from a 47 years old Vietnamese male falling under stage I. A lung cancer sample derived from a 65 years old Vietnamese male falling under stage stage I. A cardiovascular disease sample derived from a 43 years old Caucasian male. A multiple sclerosis sample derived from a 33 years old Caucasian male of relapsing-remitting type. An acute myeloid leukemia sample derived from a 16 years old male. A lymphoma sample derived from a 49 years old Vietnamese female. A psoriasis sample derived from a 40 years old Caucasian male. A diabetes sample derived from a 50 years old Caucasian male. A systemic lupus erythematosus sample derived from a 38 years old Caucasian female.

(2) Total Analysis of Amino Acid Stereoisomers

The total analysis of amino acid stereoisomers of the samples was carried out in a similar manner to Example 1.

2. Results

Among the analysis results on disease samples in Example 3, the analysis results of L-amino acids are summarized in FIG. 13-1, and the analysis results of D-amino acids are summarized in FIG. 13-2. Similarly to Example 1, each line in the Table shows a disease name of the sample donor, and each row shows the type of D-amino acid analyzed. In Table, ND denotes "being the detection limit or below". Upward arrowheads indicate that the sample contained the amino acid in an amount more than that in the samples from healthy males shown in the first line, whereas downward arrowheads indicate that the sample contained the amino acid in an amount less than that in the samples from the healthy males shown in the first line. Fine backward diagonal hatching patterns indicate that a ratio of the amount of the amino acid in these samples to the amount of the amino acid in the samples from the healthy males is two times or greater (upward arrowhead), or 1/2 or less (downward arrowhead). Coarse downward diagonal hatching patterns indicate that a ratio of the amount of the amino acid in these samples to the amount of the amino acid in the samples from the healthy males is less than two times (upward arrowhead), or less than 1/2 (downward arrowhead). In articular rheumatism, the serum concentration of L-glutamic acid was higher than those in control samples from healthy males, whereas the serum concentrations of L-glutamine and L-cysteine were lower. In kidney cancer, the serum concentration of D-serine was higher than those in the samples from healthy males, whereas the serum concentration of D-alanine was lower. In lung cancer, the serum concentration of D-alanine was lower than those in the samples from healthy males. In cardiovascular disease, the serum concentration of L-arginine was lower than those in the samples from healthy males, whereas the serum concentration of L-glutamic acid was higher. In multiple sclerosis, the serum concentration of D-serine was higher than those in the samples from healthy males, whereas the serum concentration of L-cysteine was lower. In acute myeloid leukemia, the serum concentration of L-cysteine was lower than those in the samples from healthy males. In lymphoma, the serum concentration of L-cysteine was lower than those in the samples from healthy males. In acute lymphocytic leukemia, the serum concentration of L-glutamic acid was higher than those in the samples from healthy males, whereas the L-cysteine concentration was lower. In psoriasis, the serum concentrations of L-arginine and of L-cysteine were lower than those in the samples from healthy males. In diabetes, the serum concentrations of D-alanine and L-cysteine were lower than those in the samples from healthy males, whereas the serum concentration of L-glutamic acid was higher. Note that in systemic lupus erythematosus, no change was found.

Quantitative determination of each amino acid has heretofore been made in terms of the sum of all stereoisomers thereof because of the impossibility of discriminating stereoisomers in accordance with conventional quantitative determination analyses of amino acids. However, according to the diagnostic method of the embodiment of the the present invention, stereoisomers can be discriminated as each distinct substance, and a quantitative determination thereof is enabled. As shown in FIGs. 3-1 to FIG. 3-3, when the amount of the D-form is expressed in terms of the percentage (%D) with respect to the sum of the amounts of the D-form and L-form, less variance was found for the %D of serine in the large intestine cancer patients (FIG 3-1) as compared with the D-serine amounts in the large intestine cancer patients (FIG 2-1). Similarly, less variance was found for the %D of alanine in the renal disease patients (FIG 3-3) as compared with the D-alanine amounts in renal disease patients (FIG 2-5). The reason for this phenomenon is assumed that since the amount of the D-amino acid correlates with the disease, and may be able to also correlate with the L-form amount through racemization, it is possible to exclude influences from a change of the L-form amounts, by normalization with the sum of the D-form and L-form. As suggested above, there is a case in which the amount of a D-amino acid correlating with a disease also correlates with the amount of other substance. In this respect, when the amount of the other substance per se does not correlate with the aforementioned disease, a diagnostic characteristics can be further improved by producing a parameter (for example, %D) that normalizes the amount of the D-amino acid with the amount of the other substance.

In near future, investigations of correlations of a large number of diseases with the amount of D-amino acids would be extensively evolved. Accordingly, there exists an expectation for developments of diagnostic techniques for a still larger number of diseases on the basis of the amount of the D-amino acid.

The invention is now further described by reference to the following embodiments of the invention.

1. An apparatus for analyzing a disease sample comprising:

a member for separating and quantitatively determining an amino acid stereoisomer in a biological material from a subject;
a member for obtaining an disease state index value through a calculation by substituting an amount of the amino acid stereoisomer into a discriminant equation; and
a member for outputting disease state information on the subject on a basis of the disease state index value.

2. The apparatus for analyzing a disease sample according to embodiment 1, wherein the discriminant equation is either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

3. The apparatus for analyzing a disease sample according to embodiment 2, wherein the member for outputting disease state information on the subject on a basis of the disease state index value is a member for outputting disease state information on the subject that the subject is suffering from the disease when the disease state index value is 2.0 or greater.

4. The apparatus for analyzing a disease sample according to embodiment 2 or 3, wherein the amino acid stereoisomer that correlates with the disease is: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine when the disease is DAO deficiency; D-asparagine, D-aspartic acid and D-arginine when the disease is DDO deficiency; L-phenylalanine when the disease is phenylketonuria; L-valine, L-allo-isoleucine, D-isoleucine, L-isoleucine and L-leucine when the disease is a maple syrup urine disease; L-glutamic acid, L-glutamine and L-cysteine when the disease is articular rheumatism; D-serine and D-alanine when the disease is kidney cancer; D-alanine when the disease is lung cancer; L-arginine and L-glutamic acid when the disease is a cardiovascular disease; D-serine and L-cysteine when the disease is multiple sclerosis; L-cysteine when the disease is acute myeloid leukemia; L-cysteine when the disease is lymphoma; L-glutamic acid and L-cysteine when the disease is acute lymphocytic leukemia; L-arginine and L-cysteine when the disease is psoriasis; or D-alanine, L-cysteine and L-glutamic acid when the disease is diabetes.

5. A system for analyzing a disease sample comprising:

a quantitative determination analysis unit for separating and quantitatively determining an amino acid stereoisomer in a biological material from a subject;
a disease state index value computation unit for obtaining a disease state index value through a calculation by substituting the amount of the amino acid stereoisomer into a discriminant equation; and
a disease state information output unit for outputting disease state information on the subject on a basis of the disease state index value.

6. The system for analyzing a disease sample according to embodiment 5, wherein the discriminant equation is either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

7. The system for analyzing a disease sample according to embodiment 6, wherein the disease state information output unit is for outputting disease state information on the subject that the subject is suffering from the disease when the disease state index value is 2.0 or greater.

8. The system for analyzing a disease sample according to embodiment 6 or 7, wherein the amino acid stereoisomer that correlates with the disease is: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine when the disease is DAO deficiency; D-asparagine, D-aspartic acid and D-arginine when the disease is DDO deficiency; L-phenylalanine when the disease is phenylketonuria; L-valine, L-allo-isoleucine, D-isoleucine, L-isoleucine and L-leucine when the disease is a maple syrup urine disease; L-glutamic acid, L-glutamine and L-cysteine when the disease is articular rheumatism; D-serine and D-alanine when the disease is kidney cancer; D-alanine when the disease is lung cancer; L-arginine and L-glutamic acid when the disease is a cardiovascular disease; D-serine and L-cysteine when the disease is multiple sclerosis; L-cysteine when the disease is acute myeloid leukemia; L-cysteine when the disease is lymphoma; L-glutamic acid and L-cysteine when the disease is acute lymphocytic leukemia; L-arginine and L-cysteine when the disease is psoriasis; or D-alanine, L-cysteine and L-glutamic acid when the disease is diabetes.

9. A method for analyzing a disease sample comprising:

a step of measuring the amount of an amino acid stereoisomer in a biological material from a subject;
a step of obtaining a disease state index value through a calculation by substituting the amount of the amino acid stereoisomer into a discriminant equation; and
a step of outputting disease state information on the subject on a basis of the disease state index value.

10. The method for analyzing a disease sample according to embodiment 9, wherein the discriminant equation is either:

disease state index value = (a measurement value for an amino acid stereoisomer that correlates with the disease from among the measurement values in a biological material from the subject)/ (a reference value for the amino acid stereoisomer that correlates with the disease in a biological material from a healthy individual);

or

disease state index value = [(a measurement value for an amino acid stereoisomer that correlates with the disease from among the measurement values in a biological material from the subject)/ {(a measurement value for an amino acid stereoisomer that correlates with the disease from among the measurement values in a biological material from the subject) + (a measurement value for an enantiomer of the amino acid stereoisomer that correlates with the disease from among the measurement values in a biological material from the subject)}] ÷ [(a reference value for the amino acid stereoisomer that correlates with the disease from among reference values from a healthy individual)/ {(a reference value for the amino acid stereoisomer that correlates with the disease from among reference values from the healthy individual) + (a reference value for the enantiomer of the amino acid stereoisomer that correlates with the disease from among reference values from the healthy individual)}].

11. The method for analyzing a disease sample according to embodiment 10, wherein the step of outputting disease state information on the subject on a basis of the disease state index value is a step of outputting disease state information on the subject that the subject is suffering from the disease when the disease state index value is 2.0 or greater.

12. The method for analyzing a disease sample according to embodiment 10 or 11, wherein the amino acid stereoisomer that correlates with the disease is: one, or two or more types of amino acids selected from the group consisting of D-serine, D-threonine, D-alanine, D-asparagine, allo-D-threonine, D-glutamine, D-proline and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is a climacteric disorder; D-arginine when the disease is sarcoma; D-allo-isoleucine, D-serine, D-alanine, D-methionine, D-leucine, D-aspartic acid, D-phenylalanine and L-phenylalanine when the disease is Alzheimer's disease; D-serine, D-allo-threonine, D-alanine, D-proline, D-leucine and D-phenylalanine when the disease is DAO deficiency; D-asparagine, D-aspartic acid and D-arginine when the disease is DDO deficiency; L-phenylalanine when the disease is phenylketonuria; L-valine, L-allo-isoleucine, D-isoleucine, L-isoleucine and L-leucine when the disease is a maple syrup urine disease; L-glutamic acid, L-glutamine and L-cysteine when the disease is articular rheumatism; D-serine and D-alanine when the disease is kidney cancer; D-alanine when the disease is lung cancer; L-arginine and L-glutamic acid when the disease is a cardiovascular disease; D-serine and L-cysteine when the disease is multiple sclerosis; L-cysteine when the disease is acute myeloid leukemia; L-cysteine when the disease is lymphoma; L-glutamic acid and L-cysteine when the disease is acute lymphocytic leukemia; L-arginine and L-cysteine when the disease is psoriasis; or D-alanine, L-cysteine and L-glutamic acid when the disease is diabetes.

**Claims**

1. A method for diagnosing a disease, said method including:

   - a step of measuring the amount of an amino acid stereoisomer in a biological material from a subject; and
   - a step of diagnosing the disease on the basis of a measurement value of the amount of the amino acid stereoisomer and a reference value from a healthy individual.

2. The method according to claim 1, wherein the amino acid stereoisomer is one, or two or more types of amino acids selected from the group consisting of D-threonine, D-asparagine, allo-D-threonine, D-glutamine, and D-phenylalanine when the disease is a renal disease; D-histidine and/or D-asparagine when the disease is prostate gland cancer; D-asparagine when the disease is osteoporosis; D-serine, L-arginine, D-glutamic acid and D-proline when the disease is dilated cardiomyopathy; L-histidine, L-phenylalanine and D-aspartic acid when the disease is climacteric disorder; D-arginine when the disease is sarcoma; D-allo-isoleucine, D-methionine, D-leucine, D-phenylalanine and L-phenylalanine when the disease is Alzheimer's disease; D-serine, D-allo-threonine, D-alanine, D-proline, D-leucine and D-phenylalanine when the disease is DAO deficiency; D-asparagine, D-aspartic acid, D-arginine when the disease is DDO deficiency; L-phenylalanine when the disease is phenylketonuria; L-valine, L-allo-isoleucine, D-isoleucine, L-isoleucine and L-leucine when the disease is a maple syrup urine disease; L-glutamic acid, L-glutamine and L-cysteine when the disease is articular rheumatism; D-serine and D-alanine when the disease is kidney cancer; D-alanine when the disease is lung cancer; L-arginine and L-glutamic acid when the disease is cardiovascular disease; D-serine and L-cysteine when the disease is multiple sclerosis; L-cysteine when the disease is acute myeloid leukemia; L-cysteine when the disease is lymphoma; L-glutamic acid and L-cysteine when the disease is acute lymphocytic leukemia; L-arginine and L-cysteine when the disease is psoriasis; or D-alanine, L-cysteine and L-glutamic acid when the disease is diabetes.

3. The method according to any of claims 1-2, wherein the amino acid stereoisomer is one, or two or more types of amino acids selected from the group consisting of D-threonine, D-asparagine, allo-D-threonine, D-glutamine, and D-phenylalanine when the disease is a renal disease.

4. The method according to any of claims 1-2 wherein the amino acid stereoisomer is one, or two or more types of amino acids selected from D-alanine, L-cysteine and L-glutamic acid when the disease is diabetes.

5. The method according to any of claims 1-2, wherein the amino acid stereoisomer is one, or two or more types of amino acids selected from the group consisting of D-serine and D-alanine when the disease is renal cancer.

6. The method according to any of claims 1-2, wherein the amino acid stereoisomer is one, or two or more types of amino acids selected from the group consisting of D-serine and L-cysteine when the disease is multiple sclerosis.

7. The method according to any of claims 1-2, wherein the amino acid stereoisomer is D-alanine when the disease is lung cancer.

8. The method according to any of the forgoing claims, wherein the biological material is selected from body fluids such as blood, plasma, serum, ascites, amniotic fluid, lymph fluid, saliva, seminal fluid and urine; excrement such as faeces, sweat and nasal discharge; and body tissues such as body hair, nail, skin tissues and visceral organ tissues.

9. The method according to any of the forgoing claims, wherein the method is used for one or several of the following: an indicator for diagnoses and prophylaxes of diseases, an indicator for progression of the medical condition of a disease, an indicator for deciding the effectiveness of a medial drug for a treatment and/or prophylaxis of a disease, an indicator for deciding an influence of medical drugs, quasi-drugs, cosmetics, food and other chemical substances on living bodies and an indicator of the influence of other physical and/or biological environmental factors on living bodies.

# FIG.1

| | His | Asn | Ser | Gln | Arg | Asp | Gly | allo L-Thr | allo D-Thr | Glu | Thr | Ala | Pro | Met | Val | allo D-Ile | Ile | Leu | Phe | Trp | Lys | Cys | Tyr |
|---|---|---|---|---|---|---|---|---|---|---|---|---|---|---|---|---|---|---|---|---|---|---|---|
| HEALTHY MALE | ND | | | | | | | | | | | | | | ND | | | | | | | | |
| RENAL DISEASE | | ↑ | ↑ | ↑ | — | — | | — | ↑ | — | ↑ | ↑ | ↑ | — | — | — | — | — | ↑ | | — | | |
| COGNITIVE IMPAIRMENT | — | — | — | — | — | — | | — | — | — | — | — | — | — | — | — | — | — | — | | — | | |
| HEALTHY FEMALE | — | — | — | — | — | — | | — | — | ↓ | — | — | — | — | — | — | — | ↑ | ↑ | | — | | |
| LARGE INTESTINE CANCER | — | — | — | — | — | — | | — | — | — | — | — | — | — | — | — | — | — | — | | — | | |
| BREAST CANCER | — | — | — | — | — | — | | — | — | — | — | — | — | — | — | — | — | — | — | | — | | |
| PROSTATE GLAND CANCER | ↑ | ↑ | — | — | — | — | | — | — | — | — | — | — | — | — | — | — | — | — | | — | | |
| HEPATOPATHY | — | — | — | — | — | — | | — | — | — | — | — | — | — | — | — | — | — | — | | — | | |
| OSTEOPOROSIS | — | ↑ | — | — | — | — | | — | — | — | — | — | — | — | — | — | — | — | — | | — | | |
| OVARY CANCER | — | — | — | — | — | — | | — | — | — | — | — | — | — | — | — | — | — | — | | — | | |

EP 3 663 758 A1

EP 3 663 758 A1

FIG.2-1

FIG.2-2

FIG.2-3

FIG.2-4

# FIG.2-5

# FIG.2-6

# FIG.3-1

# FIG.3-2

# FIG.3-3

# FIG.4-1

D-Ser

# FIG.4-2

L-Ser

# FIG.4-3

Ser

# FIG.4-4

Ser(%D)

# FIG.4-5

D-Arg

# FIG.4-6

L-Arg

# FIG.4-7

Arg

# FIG.4-8

D-Glu

# FIG.4-9

L-Glu

# FIG.4-10

Glu

# FIG.4-11

D-Pro

# FIG.4-12

L-Pro

# FIG.4-13

Pro

# FIG.4-14

D-Lys

# FIG.4-15

L-Lys

# FIG.4-16

Lys

# FIG.5-1

# FIG.5-2

sample: OVX DA urine-3   acquired: 100214/0007        ref 091221/2327

| | D (height) | L (height) | D (fmol/inj) | L (fmol / inj) | D (nmol/mL) | L (nmol/mL) |
|---|---|---|---|---|---|---|
| His | 642 | 3627 | 116.9 | 903.8 | 11.69 | 90.38 |
| Asn | 41367 | 49033 | 689.4 | 954.1 | 68.94 | 95.41 |
| Ser | 5833 | 69326 | 97.8 | 1349.5 | 9.78 | 134.95 |
| Gln | 12123 | 361194 | 196.5 | 6536.9 | 19.65 | 653.69 |
| Arg | 1255 | 3243 | 688.0 | 1854.2 | 68.80 | 185.42 |
| Asp | 224 | 12646 | 16.2 | 1078.6 | 1.62 | 107.86 |
| Gly | – | 449598 | – | 6690.0 | – | 570.6 |
| allo-Thr | 21328 | nd | 260.8 | nd | 26.08 | nd |
| Glu | 3266 | 54771 | 155.5 | 2960.8 | 15.55 | 296.08 |
| Thr | nd | 214958 | nd | 3210.0 | nd | 321.00 |
| Ala | nd | 257576 | nd | 3499.7 | nd | 349.97 |
| Pro | 2484 | 24968 | 82.9 | 972.2 | 8.29 | 97.22 |
| Met | 903 | 14306 | 54.4 | 1075.5 | 5.44 | 107.55 |
| Val | 6248 | 53948 | 115.6 | 1102.2 | 11.56 | 110.22 |
| allo-Ile | 12445 | nd | 240.3 | nd | 24.03 | nd |
| Ile | nd | 23461 | nd | 385.6 | nd | 38.56 |
| Leu | nd | 75395 | nd | 1230.0 | nd | 123.00 |
| Phe | 1805 | 53538 | 19.2 | 651.9 | 1.92 | 65.19 |
| Trp | nd | nd | nd | nd | nd | nd |
| Lys | 2485 | 17699 | 139.5 | 1065.6 | 13.95 | 106.56 |
| Cys | nd | nd | nd | nd | nd | nd |
| Tyr | nd | 329 | nd | 835.4 | nd | 83.54 |

# FIG.5-3

sample: OVX DA urine-4  acquired: 100214/1440      ref 091221/2327

|  | D (height) | L (height) | D (fmol/inj) | L (fmol / inj) | D (nmol/mL) | L (nmol/mL) |
|---|---|---|---|---|---|---|
| His | 607 | 4522 | 110.5 | 1126.8 | 11.05 | 112.68 |
| Asn | 31938 | 44479 | 532.3 | 865.5 | 53.23 | 86.55 |
| Ser | 4999 | 69793 | 83.8 | 1358.6 | 8.38 | 135.86 |
| Gln | 12135 | 338819 | 196.7 | 6131.9 | 19.67 | 613.19 |
| Arg | 3042 | 3436 | 1667.8 | 1964.6 | 166.78 | 196.46 |
| Asp | 206 | 7023 | 14.9 | 599.0 | 1.49 | 59.90 |
| Gly | − | 497762 | − | 7406.7 | − | 631.8 |
| allo-Thr | 27463 | nd | 335.8 | nd | 33.58 | nd |
| Glu | 3723 | 35770 | 177.3 | 1933.6 | 17.73 | 193.36 |
| Thr | nd | 198667 | nd | 2966.7 | nd | 296.67 |
| Ala | nd | 212235 | nd | 2883.6 | nd | 288.36 |
| Pro | 2294 | 22463 | 76.6 | 874.6 | 7.66 | 87.46 |
| Met | 1124 | 12451 | 67.8 | 936.0 | 6.78 | 93.60 |
| Val | 4738 | 57858 | 87.7 | 1182.1 | 8.77 | 118.21 |
| allo-Ile | 11862 | nd | 229.0 | nd | 22.90 | nd |
| Ile | nd | 31811 | nd | 522.9 | nd | 52.29 |
| Leu | nd | 77101 | nd | 1257.8 | nd | 125.78 |
| Phe | 1644 | 49309 | 17.5 | 600.4 | 1.75 | 60.04 |
| Trp | nd | nd | nd | nd | nd | nd |
| Lys | 4645 | 23701 | 260.7 | 1426.9 | 26.07 | 142.69 |
| Cys | nd | nd | nd | nd | nd | nd |
| Tyr | nd | 439 | nd | 1114.8 | nd | 111.48 |

# FIG.5-4

sample: OVX DA urine-5   acquired: 100215/0512         ref 091221/2327

| | D (height) | L (height) | D (fmol/inj) | L (fmol / inj) | D (nmol/mL) | L (nmol/mL) |
|---|---|---|---|---|---|---|
| His | 425 | 3352 | 77.4 | 835.3 | 7.74 | 83.53 |
| Asn | 26978 | 42116 | 449.6 | 819.5 | 44.96 | 81.95 |
| Ser | 3334 | 117852 | 55.9 | 2294.2 | 5.59 | 229.42 |
| Gln | 13791 | 288001 | 223.6 | 5212.2 | 22.36 | 521.22 |
| Arg | 2570 | 4662 | 1409.0 | 2665.5 | 140.90 | 266.55 |
| Asp | 247 | 9665 | 17.8 | 824.4 | 1.78 | 82.44 |
| Gly | – | 417767 | – | 6216.4 | – | 530.2 |
| allo-Thr | 20602 | nd | 251.9 | nd | 25.19 | nd |
| Glu | 3971 | 39070 | 189.1 | 2112.0 | 18.91 | 211.20 |
| Thr | nd | 157276 | nd | 2348.6 | nd | 234.86 |
| Ala | nd | 273891 | nd | 3721.3 | nd | 372.13 |
| Pro | 1725 | 19876 | 57.6 | 773.9 | 5.76 | 77.39 |
| Met | nd | 6345 | nd | 477.0 | nd | 47.70 |
| Val | 6211 | 47623 | 114.9 | 973.0 | 11.49 | 97.30 |
| allo-Ile | 18167 | nd | 350.7 | nd | 35.07 | nd |
| Ile | nd | 21178 | nd | 348.1 | nd | 34.81 |
| Leu | nd | 58884 | nd | 960.6 | nd | 96.06 |
| Phe | 1151 | 43131 | 12.2 | 525.2 | 1.22 | 52.52 |
| Trp | nd | nd | nd | nd | nd | nd |
| Lys | 5259 | 30312 | 295.2 | 1824.9 | 29.52 | 182.49 |
| Cys | nd | nd | nd | nd | nd | nd |
| Tyr | nd | 401 | nd | 1018.3 | nd | 101.83 |

# FIG.5-5

sample: OVX Sham urine-acquired: 100215/1945          ref 091221/2327

| | D (height) | L (height) | D (fmol/inj) | L (fmol / inj) | D (nmol/mL) | L (nmol/mL) |
|---|---|---|---|---|---|---|
| His | 1644 | 8171 | 299.3 | 2036.1 | 29.93 | 203.61 |
| Asn | 120117 | 63970 | 2001.8 | 1244.8 | 200.18 | 124.48 |
| Ser | 28339 | 138715 | 474.9 | 2700.3 | 47.49 | 270.03 |
| Gln | 30577 | 600794 | 495.7 | 10873.1 | 49.57 | 1087.31 |
| Arg | 3351 | 6175 | 1837.2 | 3530.6 | 183.72 | 353.06 |
| Asp | 712 | 16470 | 51.4 | 1404.8 | 5.14 | 140.48 |
| Gly | – | 695444 | – | 10348.2 | – | 882.7 |
| allo-Thr | 46934 | nd | 573.9 | nd | 57.39 | nd |
| Glu | 5522 | 78380 | 263.0 | 4237.0 | 26.30 | 423.70 |
| Thr | nd | 358162 | nd | 5348.4 | nd | 534.84 |
| Ala | nd | 378653 | nd | 5144.7 | nd | 514.47 |
| Pro | 10009 | 35246 | 334.2 | 1372.3 | 33.42 | 137.23 |
| Met | 2518 | 25212 | 151.8 | 1895.4 | 15.18 | 189.54 |
| Val | 7059 | 105548 | 130.6 | 2156.4 | 13.06 | 215.64 |
| allo-Ile | 14622 | nd | 282.3 | nd | 28.23 | nd |
| Ile | nd | 48355 | nd | 794.8 | nd | 79.48 |
| Leu | nd | 138040 | nd | 2252.0 | nd | 225.20 |
| Phe | 5103 | 106689 | 54.3 | 1299.1 | 5.43 | 129.91 |
| Trp | nd | nd | nd | nd | nd | nd |
| Lys | 10007 | 60451 | 561.7 | 3639.4 | 56.17 | 363.94 |
| Cys | nd | nd | nd | nd | nd | nd |
| Tyr | nd | 647 | nd | 1643.0 | nd | 164.30 |

# FIG.5-6

sample: ICR control urine—acquired: 100208/1312                    ref 091221/2327

| | D (height) | L (height) | D (fmol/inj) | L (fmol / inj) | D (nmol/mL) | L (nmol/mL) |
|---|---|---|---|---|---|---|
| His | 371 | 4938 | 67.6 | 1230.5 | 6.76 | 123.05 |
| Asn | 65646 | 38318 | 1094.0 | 745.6 | 109.40 | 74.56 |
| Ser | 134817 | 65133 | 2259.5 | 1267.9 | 225.95 | 126.79 |
| Gln | nd | 852808 | nd | 15434.0 | nd | 1543.40 |
| Arg | 2929 | 1333 | 1605.8 | 762.1 | 160.58 | 76.21 |
| Asp | 362 | 8283 | 26.1 | 706.5 | 2.61 | 70.65 |
| Gly | — | 513840 | — | 7646.0 | — | 652.2 |
| allo-Thr | 14910 | nd | 182.3 | nd | 18.23 | nd |
| Glu | 5398 | 30681 | 257.0 | 1658.5 | 25.70 | 165.85 |
| Thr | nd | 402455 | nd | 6009.8 | nd | 600.98 |
| Ala | 200864 | 158704 | 2176.4 | 2156.3 | 217.64 | 215.63 |
| Pro | 13382 | 39627 | 446.8 | 1542.9 | 44.68 | 154.29 |
| Met | 10475 | 72122 | 631.6 | 5422.0 | 63.16 | 542.20 |
| Val | 16934 | 72487 | 313.3 | 1481.0 | 31.33 | 148.10 |
| allo-Ile | 18913 | nd | 365.1 | nd | 36.51 | nd |
| Ile | nd | 37476 | nd | 616.0 | nd | 61.60 |
| Leu | 2506 | 268876 | 37.9 | 4223.3 | 3.79 | 422.33 |
| Phe | 5209 | 47264 | 55.4 | 575.5 | 5.54 | 57.55 |
| Trp | nd | nd | nd | nd | nd | nd |
| Lys | 9384 | 21696 | 526.7 | 1306.2 | 52.67 | 130.62 |
| Cys | nd | nd | nd | nd | nd | nd |
| Tyr | nd | 400 | nd | 1015.7 | nd | 101.57 |

# FIG.5-7

sample: ICR control urine-acquired: 100209/0345          ref 091221/2327

| | D (height) | L (height) | D (fmol/inj) | L (fmol / inj) | D (nmol/mL) | L (nmol/mL) |
|---|---|---|---|---|---|---|
| His | 734 | 11249 | 133.6 | 2803.1 | 13.36 | 280.31 |
| Asn | 148594 | 67191 | 2476.4 | 1307.4 | 247.64 | 130.74 |
| Ser | 309193 | 161397 | 5181.9 | 3141.9 | 518.19 | 314.19 |
| Gln | nd | 2269771 | nd | 41078.1 | nd | 4107.81 |
| Arg | 5299 | 8667 | 2905.2 | 4955.4 | 290.52 | 495.54 |
| Asp | 845 | 16978 | 61.0 | 1448.1 | 6.10 | 144.81 |
| Gly | – | 1853067 | – | 27573.8 | – | 2351.9 |
| allo-Thr | 29962 | nd | 366.4 | nd | 36.64 | nd |
| Glu | 3063 | 86865 | 145.9 | 4695.7 | 14.59 | 469.57 |
| Thr | nd | 1109266 | nd | 16564.6 | nd | 1656.46 |
| Ala | 336859 | 386239 | 3649.9 | 5247.8 | 364.99 | 524.78 |
| Pro | 38545 | 138299 | 1287.1 | 5384.8 | 128.71 | 538.48 |
| Met | 45626 | 313853 | 2750.9 | 23594.8 | 275.09 | 2359.48 |
| Val | 28951 | 160406 | 535.7 | 3277.2 | 53.57 | 327.72 |
| allo-Ile | 27798 | nd | 536.7 | nd | 53.67 | nd |
| Ile | nd | 77443 | nd | 1272.9 | nd | 127.29 |
| Leu | 4835 | 498596 | 73.2 | 8134.1 | 7.32 | 813.41 |
| Phe | 10244 | 82289 | 109.0 | 1002.0 | 10.90 | 100.20 |
| Trp | nd | nd | nd | nd | nd | nd |
| Lys | 5760 | 25769 | 323.3 | 1551.4 | 32.33 | 155.14 |
| Cys | nd | nd | nd | nd | nd | nd |
| Tyr | nd | 399 | nd | 1013.2 | nd | 101.32 |

# FIG.5-8

sample: OVX Sham urine-1acquired: 100217/1523                    ref 091221/2327

|       | D (height) | L (height) | D (fmol/inj) | L (fmol / inj) | D (nmol/mL) | L (nmol/mL) |
|-------|-----------|-----------|-------------|---------------|------------|------------|
| His   | 511       | 5489      | 93.0        | 1367.8        | 9.30       | 136.78     |
| Asn   | 28556     | 42554     | 475.9       | 828.0         | 47.59      | 82.80      |
| Ser   | 3334      | 72215     | 55.9        | 1405.8        | 5.59       | 140.58     |
| Gin   | 12384     | 344082    | 200.8       | 6227.2        | 20.08      | 622.72     |
| Arg   | 2045      | 3255      | 1121.2      | 1861.1        | 112.12     | 186.11     |
| Asp   | 351       | 8498      | 25.3        | 724.8         | 2.53       | 72.48      |
| Gly   | –         | 440039    | –           | 6547.8        | –          | 558.5      |
| allo-Thr | 24870  | nd        | 304.1       | nd            | 30.41      | nd         |
| Glu   | 3542      | 33450     | 168.7       | 1808.2        | 16.87      | 180.82     |
| Thr   | nd        | 155210    | nd          | 2317.7        | nd         | 231.77     |
| Ala   | nd        | 230408    | nd          | 3130.5        | nd         | 313.05     |
| Pro   | 1812      | 18229     | 60.5        | 709.8         | 6.05       | 70.98      |
| Met   | 861       | 9669      | 51.9        | 726.9         | 5.19       | 72.69      |
| Val   | 7136      | 47100     | 132.0       | 962.3         | 13.20      | 96.23      |
| allo-Ile | 17821  | nd        | 344.1       | nd            | 34.41      | nd         |
| Ile   | nd        | 23257     | nd          | 382.3         | nd         | 38.23      |
| Leu   | nd        | 63249     | nd          | 1031.8        | nd         | 103.18     |
| Phe   | 1306      | 56786     | 13.9        | 691.4         | 1.39       | 69.14      |
| Trp   | nd        | nd        | nd          | nd            | nd         | nd         |
| Lys   | 4959      | 20478     | 278.4       | 1232.9        | 27.84      | 123.29     |
| Cys   | nd        | nd        | nd          | nd            | nd         | nd         |
| Tyr   | nd        | 307       | nd          | 779.6         | nd         | 77.96      |

# FIG.5-9

sample: D-AMINO ACID

| | OVX-3 | OVX-4 | OVX-5 | Sham-16 | Sham-17 | Sham-19 | Sham-20 | t test |
|---|---|---|---|---|---|---|---|---|
| His | 11.69 | 11.05 | 7.74 | 29.93 | 27.68 | 16.51 | 9.30 | 0.125 |
| Asn | 68.94 | 53.23 | 44.96 | 200.18 | 159.61 | 119.92 | 47.59 | 0.108 |
| Ser | 9.78 | 8.38 | 5.59 | 47.49 | 34.92 | 12.57 | 5.59 | 0.196 |
| Gln | 19.65 | 19.67 | 22.36 | 49.57 | 45.26 | 33.15 | 20.08 | 0.091 |
| Arg | 68.80 | 166.78 | 140.90 | 183.72 | 115.63 | 121.00 | 112.12 | 0.820 |
| Asp | 1.62 | 1.49 | 1.78 | 5.14 | 5.27 | 4.46 | 2.53 | 0.015 |
| Gly | -- | - | - | - | - | - | - | - |
| allo-Thr | 26.08 | 33.58 | 25.19 | 57.39 | 50.67 | 39.47 | 30.41 | 0.080 |
| Glu | 15.55 | 17.73 | 18.91 | 26.30 | 34.40 | 21.40 | 16.87 | 0.165 |
| Thr | nd | nd | nd | nd | nd | nd | nd | - |
| Ala | nd | nd | nd | nd | nd | nd | nd | - |
| Pro | 8.29 | 7.66 | 5.76 | 33.42 | 31.36 | 9.30 | 6.05 | 0.193 |
| Met | 5.44 | 6.78 | nd | 15.18 | 17.20 | 8.28 | 5.19 | - |
| Val | 11.56 | 8.77 | 11.49 | 13.06 | 17.78 | 6.56 | 13.20 | 0.503 |
| allo-Ile | 24.03 | 22.90 | 35.07 | 28.23 | 42.32 | 10.82 | 34.41 | 0.858 |
| Ile | nd | nd | nd | nd | nd | nd | nd | - |
| Leu | nd | nd | nd | nd | nd | nd | nd | - |
| Phe | 1.92 | 1.75 | 1.22 | 5.43 | 6.67 | 1.81 | 1.39 | 0.219 |
| Trp | nd | nd | nd | nd | nd | nd | nd | - |
| Lys | 13.95 | 26.07 | 29.52 | 56.17 | 78.01 | 38.62 | 27.84 | 0.103 |
| Cys | nd | nd | nd | nd | nd | nd | nd | - |
| Tyr | nd | nd | nd | nd | nd | nd | nd | - |

# FIG.5-10

sample: L-AMINO ACID

| | OVX-3 | OVX-4 | OVX-5 | Sham-16 | Sham-17 | Sham-19 | Sham-20 | t test |
|---|---|---|---|---|---|---|---|---|
| His | 90.38 | 112.68 | 83.53 | 203.61 | 142.89 | 172.14 | 136.78 | 0.017 |
| Asn | 95.41 | 86.55 | 81.95 | 124.48 | 102.42 | 138.76 | 82.80 | 0.167 |
| Ser | 134.95 | 135.86 | 229.42 | 270.03 | 169.12 | 202.64 | 140.58 | 0.524 |
| Gln | 653.69 | 613.19 | 521.22 | 1087.31 | 821.36 | 820.39 | 622.72 | 0.095 |
| Arg | 185.42 | 196.46 | 266.55 | 353.06 | 248.54 | 245.91 | 186.11 | 0.402 |
| Asp | 107.86 | 59.90 | 82.44 | 140.48 | 143.86 | 120.03 | 72.48 | 0.175 |
| Gly | 570.63 | 631.76 | 530.23 | 882.66 | 602.49 | 813.91 | 558.50 | 0.216 |
| allo-Thr | nd | nd | nd | nd | nd | nd | nd | – |
| Glu | 296.08 | 193.36 | 211.20 | 423.70 | 373.06 | 281.00 | 180.82 | 0.290 |
| Thr | 321.00 | 296.67 | 234.86 | 534.84 | 337.86 | 352.37 | 231.77 | 0.349 |
| Ala | 349.97 | 288.36 | 372.13 | 514.47 | 384.06 | 472.21 | 313.05 | 0.202 |
| Pro | 97.22 | 87.46 | 77.39 | 137.23 | 114.84 | 77.07 | 70.98 | 0.539 |
| Met | 107.55 | 93.60 | 47.70 | 189.54 | 132.36 | 106.96 | 72.69 | 0.253 |
| Val | 110.22 | 118.21 | 97.30 | 215.64 | 179.09 | 156.60 | 96.23 | 0.136 |
| allo-Ile | nd | nd | nd | nd | nd | nd | nd | – |
| Ile | 38.56 | 52.29 | 34.81 | 79.48 | 69.14 | 61.72 | 38.23 | 0.133 |
| Leu | 123.00 | 125.78 | 96.06 | 225.20 | 171.40 | 165.51 | 103.18 | 0.154 |
| Phe | 65.19 | 60.04 | 52.52 | 129.91 | 111.52 | 100.67 | 69.14 | 0.037 |
| Trp | nd | nd | nd | nd | nd | nd | nd | – |
| Lys | 106.56 | 142.69 | 182.49 | 363.94 | 378.31 | 201.61 | 123.29 | 0.167 |
| Cys | nd | nd | nd | nd | nd | nd | nd | – |
| Tyr | 83.54 | 111.46 | 101.83 | 164.30 | 57.39 | 94.72 | 77.96 | 0.990 |

# FIG.5-11

sample: D/(D+L)

| | OVX-3 | OVX-4 | OVX-5 | Sham-16 | Sham-17 | Sham-19 | Sham-20 | t test |
|---|---|---|---|---|---|---|---|---|
| His | 11.45 | 8.93 | 8.48 | 12.82 | 16.23 | 8.75 | 6.37 | 0.621 |
| Asn | 41.95 | 38.08 | 35.43 | 61.66 | 60.91 | 46.36 | 36.50 | 0.141 |
| Ser | 6.75 | 5.81 | 2.38 | 14.96 | 17.12 | 5.84 | 3.82 | 0.236 |
| Gln | 2.92 | 3.11 | 4.11 | 4.36 | 5.22 | 3.88 | 3.12 | 0.261 |
| Arg | 27.06 | 45.91 | 34.58 | 34.23 | 31.75 | 32.98 | 37.59 | 0.736 |
| Asp | 1.48 | 2.42 | 2.12 | 3.53 | 3.53 | 3.58 | 3.38 | 0.002 |
| Gly | — | — | — | — | — | — | — | — |
| allo-Thr | 7.51 | 10.17 | 9.69 | 9.69 | 13.04 | 10.07 | 11.60 | 0.142 |
| Glu | 4.99 | 8.40 | 8.22 | 5.84 | 8.44 | 7.08 | 8.53 | 0.829 |
| Thr | to allo-Thr | to allo-Thr | to allo-Thr | to allo-Thr | to allo-Thr | to allo-Thr | to allo-Thr | to allo-Thr |
| Ala | — | — | — | — | — | — | — | — |
| Pro | 7.86 | 8.05 | 6.93 | 19.58 | 21.45 | 10.77 | 7.85 | 0.122 |
| Met | 4.82 | 6.75 | — | 7.42 | 11.50 | 7.18 | 6.67 | 0.246 |
| Val | 9.49 | 6.90 | 10.56 | 5.71 | 9.03 | 4.02 | 12.07 | 0.602 |
| allo-Ile | 38.39 | 30.46 | 50.19 | 26.21 | 37.97 | 14.92 | 47.37 | 0.440 |
| Ile | to allo-Ile | to allo-Ile | to allo-Ile | to allo-Ile | to allo-Ile | to allo-Ile | to allo-Ile | to allo-Ile |
| Leu | — | — | — | — | — | — | — | — |
| Phe | 2.86 | 2.83 | 2.28 | 4.01 | 5.64 | 1.77 | 1.97 | 0.556 |
| Trp | — | — | — | — | — | — | — | — |
| Lys | 11.58 | 15.45 | 13.92 | 13.37 | 17.10 | 16.08 | 18.42 | 0.162 |
| Cys | — | — | — | — | — | — | — | — |
| Tyr | — | — | — | — | — | — | — | — |

# FIG.5-12

sample: D/total L

|  | OVX-3 | OVX-4 | OVX-5 | Sham-16 | Sham-17 | Sham-19 | Sham-20 | t test |
|---|---|---|---|---|---|---|---|---|
| His | 0.34 | 0.34 | 0.24 | 0.51 | 0.62 | 0.37 | 0.30 | 0.165 |
| Asn | 2.00 | 1.62 | 1.40 | 3.41 | 3.56 | 2.67 | 1.54 | 0.104 |
| Ser | 0.28 | 0.25 | 0.17 | 0.81 | 0.78 | 0.28 | 0.18 | 0.220 |
| Gln | 0.57 | 0.60 | 0.70 | 0.84 | 1.01 | 0.74 | 0.65 | 0.112 |
| Arg | 1.99 | 5.06 | 4.40 | 3.13 | 2.58 | 2.69 | 3.62 | 0.372 |
| Asp | 0.05 | 0.05 | 0.06 | 0.09 | 0.12 | 0.10 | 0.08 | 0.005 |
| Gly | – | – | – | – | – | – | – | – |
| allo-Thr | 0.76 | 1.02 | 0.79 | 0.98 | 1.13 | 0.88 | 0.98 | 0.197 |
| Glu | 0.45 | 0.54 | 0.59 | 0.45 | 0.77 | 0.48 | 0.54 | 0.738 |
| Thr | – | – | – | – | – | – | – | – |
| Ala | – | – | – | – | – | – | – | – |
| Pro | 0.24 | 0.23 | 0.18 | 0.57 | 0.70 | 0.21 | 0.20 | 0.245 |
| Met | 0.16 | 0.21 | – | 0.26 | 0.38 | 0.18 | 0.17 | 0.425 |
| Val | 0.33 | 0.27 | 0.36 | 0.22 | 0.40 | 0.15 | 0.43 | 0.802 |
| allo-Ile | 0.70 | 0.70 | 1.10 | 0.48 | 0.94 | 0.24 | 1.11 | 0.630 |
| Ile | – | – | – | – | – | – | – | – |
| Leu | – | – | – | – | – | – | – | – |
| Phe | 0.06 | 0.05 | 0.04 | 0.09 | 0.15 | 0.04 | 0.04 | 0.330 |
| Trp | – | – | – | – | – | – | – | – |
| Lys | 0.40 | 0.79 | 0.92 | 0.96 | 1.74 | 0.86 | 0.90 | 0.205 |
| Cys | – | – | – | – | – | – | – | – |
| Tyr | – | – | – | – | – | – | – | – |

# FIG.5-13

sample: D/total D

| | OVX-3 | OVX-4 | OVX-5 | Sham-16 | Sham-17 | Sham-19 | Sham-20 | t test |
|---|---|---|---|---|---|---|---|---|
| His | 4.07 | 2.86 | 2.20 | 3.99 | 4.15 | 3.72 | 2.79 | 0.338 |
| Asn | 24.02 | 13.79 | 12.81 | 26.66 | 23.93 | 27.01 | 14.29 | 0.244 |
| Ser | 3.41 | 2.17 | 1.59 | 6.32 | 5.24 | 2.83 | 1.68 | 0.279 |
| Gln | 6.85 | 5.10 | 6.37 | 6.60 | 6.79 | 7.47 | 6.03 | 0.322 |
| Arg | 23.97 | 43.21 | 40.14 | 24.46 | 17.34 | 27.25 | 33.67 | 0.176 |
| Asp | 0.56 | 0.39 | 0.51 | 0.68 | 0.79 | 1.00 | 0.76 | 0.017 |
| Gly | – | – | – | – | – | – | – | – |
| allo-Thr | 9.09 | 8.70 | 7.18 | 7.64 | 7.60 | 8.89 | 9.13 | 0.993 |
| Glu | 5.42 | 4.59 | 5.39 | 3.50 | 5.16 | 4.82 | 5.07 | 0.373 |
| Thr | – | – | – | – | – | – | – | – |
| Ala | – | – | – | – | – | – | – | – |
| Pro | 2.89 | 1.98 | 1.64 | 4.45 | 4.70 | 2.09 | 1.82 | 0.302 |
| Met | 1.90 | 1.76 | – | 2.02 | 2.58 | 1.86 | 1.56 | – |
| Val | 4.03 | 2.27 | 3.27 | 1.74 | 2.67 | 1.48 | 3.96 | 0.398 |
| allo-Ile | 8.37 | 5.93 | 9.99 | 3.76 | 6.34 | 2.44 | 10.33 | 0.345 |
| Ile | – | – | – | – | – | – | – | – |
| Leu | – | – | – | – | – | – | – | – |
| Phe | 0.67 | 0.45 | 0.35 | 0.72 | 1.00 | 0.41 | 0.42 | 0.463 |
| Trp | – | – | – | – | – | – | – | – |
| Lys | 4.86 | 6.75 | 8.41 | 7.48 | 11.70 | 8.70 | 8.36 | 0.145 |
| Cys | – | – | – | – | – | – | – | – |
| Tyr | – | – | – | – | – | – | – | – |

# FIG.5-14

sample: D/D-Glu

| | OVX-3 | OVX-4 | OVX-5 | Sham-16 | Sham-17 | Sham-19 | Sham-20 | t test |
|---|---|---|---|---|---|---|---|---|
| His | 75.18 | 62.34 | 40.92 | 113.82 | 80.46 | 77.17 | 55.15 | 0.239 |
| Asn | 443.35 | 300.21 | 237.76 | 761.15 | 464.00 | 560.38 | 282.10 | 0.200 |
| Ser | 62.87 | 47.25 | 29.55 | 180.59 | 101.52 | 58.74 | 33.12 | 0.283 |
| Gln | 126.38 | 110.95 | 118.22 | 188.47 | 131.56 | 154.89 | 119.00 | 0.165 |
| Arg | 442.47 | 940.64 | 745.10 | 698.54 | 336.12 | 585.41 | 664.59 | 0.398 |
| Asp | 10.40 | 8.39 | 9.43 | 19.54 | 15.32 | 20.85 | 15.02 | 0.006 |
| Gly | — | — | — | — | — | — | — | — |
| allo-Thr | 167.72 | 189.41 | 133.23 | 218.23 | 147.28 | 184.46 | 180.27 | 0.424 |
| Glu | 100.02 | 99.99 | 100.00 | 99.98 | 100.00 | 99.98 | 99.98 | 0.106 |
| Thr | — | — | — | — | — | — | — | — |
| Ala | — | — | — | — | — | — | — | — |
| Pro | 53.34 | 43.20 | 30.46 | 127.08 | 91.17 | 43.46 | 35.87 | 0.271 |
| Met | 35.01 | 36.22 | — | 57.73 | 50.00 | 38.68 | 30.77 | 0.442 |
| Val | 74.34 | 49.44 | 60.77 | 49.66 | 51.69 | 30.65 | 78.26 | 0.523 |
| allo-Ile | 154.51 | 129.17 | 185.48 | 107.34 | 123.02 | 50.58 | 203.95 | 0.418 |
| Ile | — | — | — | — | — | — | — | — |
| Leu | — | — | — | — | — | — | — | — |
| Phe | 12.35 | 9.87 | 6.48 | 20.65 | 19.38 | 8.48 | 8.24 | 0.326 |
| Trp | — | — | — | — | — | — | — | — |
| Lys | 89.70 | 147.06 | 156.11 | 213.58 | 226.78 | 180.46 | 165.00 | 0.043 |
| Cys | — | — | — | — | — | — | — | — |
| Tyr | — | — | — | — | — | — | — | — |

# FIG.6-1

sample: Cancer S urine-3  acquired: 100205/2307          ref 091221/2327

|         | D (height) | L (height) | D (fmol/inj) | L (fmol / inj) | D (nmol/mL) | L (nmol/mL) |
|---------|-----------|-----------|-------------|---------------|------------|------------|
| His     | 397       | 3306      | 72.3        | 823.8         | 7.23       | 82.38      |
| Asn     | 80655     | 40173     | 1344.2      | 781.7         | 134.42     | 78.17      |
| Ser     | 97224     | 71724     | 1629.4      | 1396.2        | 162.94     | 139.62     |
| Gln     | 15626     | 631146    | 253.3       | 11422.4       | 25.33      | 1142.24    |
| Arg     | 10223     | 3000      | 5604.7      | 1715.3        | 560.47     | 171.53     |
| Asp     | 429       | 8219      | 31.0        | 701.0         | 3.10       | 70.10      |
| Gly     | –         | 582765    | –           | 8671.6        | –          | 739.6      |
| allo-Thr| 22190     | nd        | 271.4       | nd            | 27.14      | nd         |
| Glu     | 4736      | 39588     | 225.5       | 2140.0        | 22.55      | 214.00     |
| Thr     | nd        | 257594    | nd          | 3846.6        | nd         | 384.66     |
| Ala     | 93929     | 216425    | 1017.7      | 2940.6        | 101.77     | 294.06     |
| Pro     | 18737     | 24361     | 625.7       | 948.5         | 62.57      | 94.85      |
| Met     | 2780      | 33204     | 167.6       | 2496.2        | 16.76      | 249.62     |
| Val     | 24862     | 50321     | 460.0       | 1028.1        | 46.00      | 102.81     |
| allo-Ile| 44308     | nd        | 855.4       | nd            | 85.54      | nd         |
| Ile     | nd        | 30821     | nd          | 506.6         | nd         | 50.66      |
| Leu     | 1279      | 170275    | 19.4        | 2777.9        | 1.94       | 277.79     |
| Phe     | 4231      | 40068     | 45.0        | 487.9         | 4.50       | 48.79      |
| Trp     | nd        | nd        | nd          | nd            | nd         | nd         |
| Lys     | 12411     | 44306     | 696.7       | 2667.4        | 69.67      | 266.74     |
| Cys     | nd        | nd        | nd          | nd            | nd         | nd         |
| Tyr     | nd        | 4086      | nd          | 10375.8       | nd         | 1037.58    |

# FIG.6-2

sample: Cancer S urine-4  acquired: 100206/1340          ref 091221/2327

| | D (height) | L (height) | D (fmol/inj) | L (fmol / inj) | D (nmol/mL) | L (nmol/mL) |
|---|---|---|---|---|---|---|
| His | 450 | 20855 | 81.9 | 5196.9 | 8.19 | 519.69 |
| Asn | 102440 | 83655 | 1707.2 | 1627.8 | 170.72 | 162.78 |
| Ser | 204795 | 200668 | 3432.2 | 3906.3 | 343.22 | 390.63 |
| Gln | nd | 1826174 | nd | 33049.9 | nd | 3304.99 |
| Arg | 7708 | 3333 | 4225.9 | 1905.7 | 422.59 | 190.57 |
| Asp | 418 | 13208 | 30.2 | 1126.6 | 3.02 | 112.66 |
| Gly | – | 3950073 | – | 58777.3 | – | 5013.4 |
| allo-Thr | 19106 | nd | 233.6 | nd | 23.36 | nd |
| Glu | 5854 | 71695 | 278.8 | 3875.6 | 27.88 | 387.56 |
| Thr | nd | 1422546 | nd | 21242.8 | nd | 2124.28 |
| Ala | 395161 | 446964 | 4281.6 | 6072.9 | 428.16 | 607.29 |
| Pro | 20416 | 55889 | 681.7 | 2176.1 | 68.17 | 217.61 |
| Met | 27713 | 240761 | 1670.9 | 18099.9 | 167.09 | 1809.99 |
| Val | 16246 | 225110 | 300.6 | 4599.2 | 30.06 | 459.92 |
| allo-Ile | 19179 | nd | 370.3 | nd | 37.03 | nd |
| Ile | nd | 128800 | nd | 2117.0 | nd | 211.70 |
| Leu | 5331 | 764460 | 80.7 | 12471.4 | 8.07 | 1247.14 |
| Phe | 7375 | 144957 | 78.5 | 1765.1 | 7.85 | 176.51 |
| Trp | nd | nd | nd | nd | nd | nd |
| Lys | 16628 | 39159 | 933.4 | 2357.5 | 93.34 | 235.75 |
| Cys | nd | nd | nd | nd | nd | nd |
| Tyr | nd | 5750 | nd | 14601.3 | nd | 1460.13 |

# FIG.6-3

sample: Cancer S urine-5  acquired: 100207/0412          ref 091221/2327

|          | D (height) | L (height) | D (fmol/inj) | L (fmol / inj) | D (nmol/mL) | L (nmol/mL) |
|----------|-----------|-----------|-------------|----------------|-------------|-------------|
| His      | 343       | 5515      | 62.5        | 1374.3         | 6.25        | 137.43      |
| Asn      | 70636     | 46633     | 1177.2      | 907.4          | 117.72      | 90.74       |
| Ser      | 101756    | 87036     | 1705.4      | 1694.3         | 170.54      | 169.43      |
| Gln      | nd        | 1090635   | nd          | 19738.2        | nd          | 1973.82     |
| Arg      | 8921      | 3667      | 4890.9      | 2096.6         | 489.09      | 209.66      |
| Asp      | 439       | 13607     | 31.7        | 1160.6         | 3.17        | 116.06      |
| Gly      | –         | 884559    | –           | 13162.3        | –           | 1122.7      |
| allo-Thr | 17163     | nd        | 209.9       | nd             | 20.99       | nd          |
| Glu      | 2632      | 66047     | 125.3       | 3570.3         | 12.53       | 357.03      |
| Thr      | nd        | 364161    | nd          | 5438.0         | nd          | 543.80      |
| Ala      | 126754    | 309079    | 1373.4      | 4199.4         | 137.34      | 419.94      |
| Pro      | 16714     | 45287     | 558.1       | 1763.3         | 55.81       | 176.33      |
| Met      | 1959      | 85098     | 118.1       | 6397.5         | 11.81       | 639.75      |
| Val      | 13506     | 77831     | 249.9       | 1590.1         | 24.99       | 159.01      |
| allo-Ile | 19610     | nd        | 378.6       | nd             | 37.86       | nd          |
| Ile      | nd        | 52448     | nd          | 862.1          | nd          | 86.21       |
| Leu      | 694       | 274621    | 10.5        | 4480.2         | 1.05        | 448.02      |
| Phe      | 2154      | 51844     | 22.9        | 631.3          | 2.29        | 63.13       |
| Trp      | nd        | nd        | nd          | nd             | nd          | nd          |
| Lys      | 5760      | 25769     | 323.3       | 1551.4         | 32.33       | 155.14      |
| Cys      | nd        | nd        | nd          | nd             | nd          | nd          |
| Tyr      | nd        | 399       | nd          | 1013.2         | nd          | 101.32      |

# FIG.6-4

sample: ICR control urine—acquired: 100207/1845          ref 091221/2327

| | D (height) | L (height) | D (fmol/inj) | L (fmol / inj) | D (nmol/mL) | L (nmol/mL) |
|---|---|---|---|---|---|---|
| His | 739 | 6607 | 134.6 | 1646.4 | 13.46 | 164.64 |
| Asn | 94824 | 44003 | 1580.3 | 856.2 | 158.03 | 85.62 |
| Ser | 154212 | 96721 | 2584.5 | 1882.8 | 258.45 | 188.28 |
| Gln | nd | 1253936 | nd | 22693.6 | nd | 2269.36 |
| Arg | 3964 | 4333 | 2173.2 | 2477.4 | 217.32 | 247.74 |
| Asp | 535 | 11667 | 38.6 | 995.1 | 3.86 | 99.51 |
| Gly | – | 1225889 | – | 18241.3 | – | 1555.9 |
| allo-Thr | 19914 | nd | 243.5 | nd | 24.35 | nd |
| Glu | 2380 | 44545 | 113.3 | 2408.0 | 11.33 | 240.80 |
| Thr | nd | 607246 | nd | 9068.0 | nd | 906.80 |
| Ala | 270729 | 205013 | 2933.4 | 2785.5 | 293.34 | 278.55 |
| Pro | 49530 | 63256 | 1653.9 | 2463.0 | 165.39 | 246.30 |
| Met | 9703 | 140664 | 585.0 | 10574.8 | 58.50 | 1057.48 |
| Val | 17180 | 98013 | 317.9 | 2002.5 | 31.79 | 200.25 |
| allo-Ile | 25570 | nd | 493.7 | nd | 49.37 | nd |
| Ile | nd | 51437 | nd | 845.4 | nd | 84.54 |
| Leu | 1912 | 338085 | 28.9 | 5515.5 | 2.89 | 551.55 |
| Phe | 5177 | 58320 | 55.1 | 710.1 | 5.51 | 71.01 |
| Trp | nd | nd | nd | nd | nd | nd |
| Lys | 8836 | 29055 | 496.0 | 1749.2 | 49.60 | 174.92 |
| Cys | nd | nd | nd | nd | nd | nd |
| Tyr | nd | 470 | nd | 1193.5 | nd | 119.35 |

# FIG.6-5

sample: ICR control urine—acquired: 100208/1312          ref 091221/2327

|  | D (height) | L (height) | D (fmol/inj) | L (fmol / inj) | D (nmol/mL) | L (nmol/mL) |
|---|---|---|---|---|---|---|
| His | 371 | 4938 | 67.6 | 1230.5 | 6.76 | 123.05 |
| Asn | 65646 | 38318 | 1094.0 | 745.6 | 109.40 | 74.56 |
| Ser | 134817 | 65133 | 2259.5 | 1267.9 | 225.95 | 126.79 |
| Gln | nd | 852808 | nd | 15434.0 | nd | 1543.40 |
| Arg | 2929 | 1333 | 1605.8 | 762.1 | 160.58 | 76.21 |
| Asp | 362 | 8283 | 26.1 | 706.5 | 2.61 | 70.65 |
| Gly | -- | 513840 | -- | 7646.0 | -- | 652.2 |
| allo-Thr | 14910 | nd | 182.3 | nd | 18.23 | nd |
| Glu | 5398 | 30681 | 257.0 | 1658.5 | 25.70 | 165.85 |
| Thr | nd | 402455 | nd | 6009.8 | nd | 600.98 |
| Ala | 200864 | 158704 | 2176.4 | 2156.3 | 217.64 | 215.63 |
| Pro | 13382 | 39627 | 446.8 | 1542.9 | 44.68 | 154.29 |
| Met | 10475 | 72122 | 631.6 | 5422.0 | 63.16 | 542.20 |
| Val | 16934 | 72487 | 313.3 | 1481.0 | 31.33 | 148.10 |
| allo-Ile | 18913 | nd | 365.1 | nd | 36.51 | nd |
| Ile | nd | 37476 | nd | 616.0 | nd | 61.60 |
| Leu | 2506 | 258876 | 37.9 | 4223.3 | 3.79 | 422.33 |
| Phe | 5209 | 47264 | 55.4 | 575.5 | 5.54 | 57.55 |
| Trp | nd | nd | nd | nd | nd | nd |
| Lys | 9384 | 21696 | 526.7 | 1306.2 | 52.67 | 130.62 |
| Cys | nd | nd | nd | nd | nd | nd |
| Tyr | nd | 400 | nd | 1015.7 | nd | 101.57 |

# FIG.6-6

sample: ICR control urine—acquired: 100209/0345          ref 091221/2327

| | D (height) | L (height) | D (fmol/inj) | L (fmol / inj) | D (nmol/mL) | L (nmol/mL) |
|---|---|---|---|---|---|---|
| His | 734 | 11249 | 133.6 | 2803.1 | 13.36 | 280.31 |
| Asn | 148594 | 67191 | 2476.4 | 1307.4 | 247.64 | 130.74 |
| Ser | 309193 | 161397 | 5181.9 | 3141.9 | 518.19 | 314.19 |
| Gln | nd | 2269771 | nd | 41078.1 | nd | 4107.81 |
| Arg | 5299 | 8667 | 2905.2 | 4955.4 | 290.52 | 495.54 |
| Asp | 845 | 16978 | 61.0 | 1448.1 | 6.10 | 144.81 |
| Gly | – | 1853067 | – | 27573.8 | – | 2351.9 |
| allo-Thr | 29962 | nd | 366.4 | nd | 36.64 | nd |
| Glu | 3063 | 86865 | 145.9 | 4695.7 | 14.59 | 469.57 |
| Thr | nd | 1109266 | nd | 16564.6 | nd | 1656.46 |
| Ala | 336859 | 386239 | 3649.9 | 5247.8 | 364.99 | 524.78 |
| Pro | 38545 | 138299 | 1287.1 | 5384.8 | 128.71 | 538.48 |
| Met | 45626 | 313853 | 2750.9 | 23594.8 | 275.09 | 2359.48 |
| Val | 28951 | 160406 | 535.7 | 3277.2 | 53.57 | 327.72 |
| allo-Ile | 27798 | nd | 536.7 | nd | 53.67 | nd |
| Ile | nd | 77443 | nd | 1272.9 | nd | 127.29 |
| Leu | 4835 | 498596 | 73.2 | 8134.1 | 7.32 | 813.41 |
| Phe | 10244 | 82289 | 109.0 | 1002.0 | 10.90 | 100.20 |
| Trp | nd | nd | nd | nd | nd | nd |
| Lys | 5760 | 25769 | 323.3 | 1551.4 | 32.33 | 155.14 |
| Cys | nd | nd | nd | nd | nd | nd |
| Tyr | nd | 399 | nd | 1013.2 | nd | 101.32 |

# FIG.6-7

sample: D-AMINO ACID

| | S3 | S4 | S5 | C3 | C4 | C5 | t test |
|---|---|---|---|---|---|---|---|
| His | 7.23 | 8.19 | 6.25 | 13.46 | 6.76 | 13.36 | 0.158 |
| Asn | 134.42 | 170.72 | 117.72 | 158.03 | 109.40 | 247.64 | 0.518 |
| Ser | 162.94 | 343.22 | 170.54 | 258.45 | 225.95 | 518.19 | 0.378 |
| Gln | 25.33 | nd | nd | nd | nd | nd | – |
| Arg | 560.47 | 422.59 | 489.09 | 217.32 | 160.58 | 290.52 | 0.008 |
| Asp | 3.10 | 3.02 | 3.17 | 3.86 | 2.61 | 6.10 | 0.343 |
| Gly | – | – | – | – | – | – | – |
| allo-Thr | 27.14 | 23.36 | 20.99 | 24.35 | 18.23 | 36.64 | 0.674 |
| Glu | 22.55 | 27.88 | 12.53 | 11.33 | 25.70 | 14.59 | 0.578 |
| Thr | nd | nd | nd | nd | nd | nd | – |
| Ala | 101.77 | 428.16 | 137.34 | 293.34 | 217.64 | 364.99 | 0.567 |
| Pro | 62.57 | 68.17 | 55.81 | 165.39 | 44.68 | 128.71 | 0.230 |
| Met | 16.76 | 167.09 | 11.81 | 58.50 | 63.16 | 275.09 | 0.487 |
| Val | 46.00 | 30.06 | 24.99 | 31.79 | 31.33 | 53.57 | 0.619 |
| allo-Ile | 85.54 | 37.03 | 37.86 | 49.37 | 36.51 | 53.67 | 0.701 |
| Ile | nd | nd | nd | nd | nd | nd | – |
| Leu | 1.94 | 8.07 | 1.05 | 2.89 | 3.79 | 7.32 | 0.723 |
| Phe | 4.50 | 7.85 | 2.29 | 5.51 | 5.54 | 10.90 | 0.370 |
| Trp | nd | nd | nd | nd | nd | nd | – |
| Lys | 69.67 | 93.34 | 32.33 | 49.60 | 52.67 | 32.33 | 0.343 |
| Cys | nd | nd | nd | nd | nd | nd | – |
| Tyr | nd | nd | nd | nd | nd | nd | – |

# FIG.6-8

sample: L-AMINO ACID

| | S3 | S4 | S5 | C3 | C4 | C5 | t test |
|---|---|---|---|---|---|---|---|
| His | 82.38 | 519.69 | 137.43 | 164.64 | 123.05 | 280.31 | 0.714 |
| Asn | 78.17 | 162.78 | 90.74 | 85.62 | 74.56 | 130.74 | 0.688 |
| Ser | 139.62 | 390.63 | 169.43 | 188.28 | 126.79 | 314.19 | 0.820 |
| Gln | 1142.24 | 3304.99 | 1973.82 | 2269.36 | 1543.40 | 4107.81 | 0.640 |
| Arg | 171.53 | 190.57 | 209.66 | 247.74 | 76.21 | 495.54 | 0.536 |
| Asp | 70.10 | 112.66 | 116.06 | 99.51 | 70.65 | 144.81 | 0.847 |
| Gly | 739.64 | 5013.42 | 1122.68 | 1555.90 | 652.16 | 2351.91 | 0.623 |
| allo-Thr | nd | nd | nd | nd | nd | nd | – |
| Glu | 214.00 | 387.56 | 357.03 | 240.80 | 165.85 | 469.57 | 0.808 |
| Thr | 384.66 | 2124.28 | 543.80 | 906.80 | 600.98 | 1656.46 | 0.956 |
| Ala | 294.06 | 607.29 | 419.94 | 278.55 | 215.63 | 524.78 | 0.485 |
| Pro | 94.85 | 217.61 | 176.33 | 246.30 | 154.29 | 538.48 | 0.284 |
| Met | 249.62 | 1809.99 | 639.75 | 1057.48 | 542.20 | 2359.48 | 0.589 |
| Val | 102.81 | 459.92 | 159.01 | 200.25 | 148.10 | 327.72 | 0.907 |
| allo-Ile | nd | nd | nd | nd | nd | nd | – |
| Ile | 50.66 | 211.70 | 86.21 | 84.54 | 61.60 | 127.29 | 0.658 |
| Leu | 277.79 | 1247.14 | 448.02 | 551.55 | 422.33 | 813.41 | 0.856 |
| Phe | 48.79 | 176.51 | 63.13 | 71.01 | 57.55 | 100.20 | 0.663 |
| Trp | nd | nd | nd | nd | nd | nd | – |
| Lys | 266.74 | 235.75 | 155.14 | 174.92 | 130.62 | 155.14 | 0.139 |
| Cys | nd | nd | nd | nd | nd | nd | – |
| Tyr | 1037.58 | 1460.13 | 101.32 | 119.35 | 101.57 | 101.32 | 0.132 |

# FIG.6-9

sample: D/(D+L)

| | S3 | S4 | S5 | C3 | C4 | C5 | t test |
|---|---|---|---|---|---|---|---|
| His | 8.07 | 1.55 | 4.35 | 7.56 | 5.20 | 4.55 | 0.623 |
| Asn | 63.23 | 51.19 | 56.47 | 64.86 | 59.47 | 65.45 | 0.188 |
| Ser | 53.85 | 46.77 | 50.16 | 57.85 | 64.05 | 62.25 | 0.016 |
| Gln | 2.17 | – | – | – | – | – | – |
| Arg | 76.57 | 68.92 | 69.99 | 46.73 | 57.81 | 36.96 | 0.086 |
| Asp | 4.23 | 2.61 | 2.66 | 3.74 | 3.57 | 4.04 | 0.326 |
| Gly | – | – | – | – | – | – | – |
| allo-Thr | 6.59 | 1.09 | 3.72 | 2.62 | 2.94 | 2.16 | 0.488 |
| Glu | 9.53 | 6.71 | 3.39 | 4.50 | 13.42 | 3.01 | 0.913 |
| Thr | to allo-Thr | to allo-Thr | to allo-Thr | to allo-Thr | to allo-Thr | to allo-Thr | to allo-Thr |
| Ala | 25.71 | 41.35 | 24.64 | 51.29 | 50.23 | 41.02 | 0.055 |
| Pro | 39.74 | 23.85 | 24.04 | 40.17 | 22.46 | 19.29 | 0.831 |
| Met | 6.29 | 8.45 | 1.81 | 5.24 | 10.43 | 10.44 | 0.289 |
| Val | 30.91 | 6.13 | 13.58 | 13.70 | 17.46 | 14.05 | 0.820 |
| allo-Ile | 62.81 | 14.89 | 30.52 | 36.87 | 37.22 | 29.66 | 0.922 |
| Ile | to allo-Ile | to allo-Ile | to allo-Ile | to allo-Ile | to allo-Ile | to allo-Ile | to allo-Ile |
| Leu | 0.69 | 0.64 | 0.23 | 0.52 | 0.89 | 0.89 | 0.267 |
| Phe | 8.45 | 4.26 | 3.50 | 7.20 | 8.79 | 9.81 | 0.136 |
| Trp | – | – | – | – | – | – | – |
| Lys | 20.71 | 28.36 | 17.25 | 22.09 | 28.74 | 17.25 | 0.906 |
| Cys | – | – | – | – | – | – | – |
| Tyr | – | – | – | – | – | – | – |

# FIG.6-10

sample: D/total L

|  | S3 | S4 | S5 | C3 | C4 | C5 | t test |
|---|---|---|---|---|---|---|---|
| His | 0.16 | 0.05 | 0.09 | 0.16 | 0.13 | 0.09 | 0.549 |
| Asn | 3.05 | 0.99 | 1.71 | 1.88 | 2.12 | 1.66 | 0.959 |
| Ser | 3.70 | 2.00 | 2.48 | 3.07 | 4.37 | 3.48 | 0.224 |
| Gln | 0.57 | – | – | – | – | – | – |
| Arg | 12.71 | 2.46 | 7.12 | 2.58 | 3.11 | 1.95 | 0.177 |
| Asp | 0.07 | 0.02 | 0.05 | 0.05 | 0.05 | 0.04 | 0.945 |
| Gly | – | – | – | – | – | – | – |
| allo–Thr | 0.62 | 0.14 | 0.31 | 0.29 | 0.35 | 0.25 | 0.715 |
| Glu | 0.51 | 0.16 | 0.18 | 0.13 | 0.50 | 0.10 | 0.817 |
| Thr | – | – | – | – | – | – | – |
| Ala | 2.31 | 2.49 | 2.00 | 3.48 | 4.21 | 2.45 | 0.104 |
| Pro | 1.42 | 0.40 | 0.81 | 1.96 | 0.86 | 0.86 | 0.494 |
| Met | 0.38 | 0.97 | 0.17 | 0.69 | 1.22 | 1.85 | 0.143 |
| Val | 1.04 | 0.18 | 0.36 | 0.38 | 0.61 | 0.36 | 0.787 |
| allo–Ile | 1.94 | 0.22 | 0.55 | 0.59 | 0.71 | 0.36 | 0.549 |
| Ile | – | – | – | – | – | – | – |
| Leu | 0.04 | 0.05 | 0.02 | 0.03 | 0.07 | 0.05 | 0.329 |
| Phe | 0.10 | 0.05 | 0.03 | 0.07 | 0.11 | 0.07 | 0.433 |
| Trp | – | – | – | – | – | – | – |
| Lys | 1.58 | 0.54 | 0.47 | 0.59 | 1.02 | 0.22 | 0.580 |
| Cys | – | – | – | – | – | – | – |
| Tyr | – | – | – | – | – | – | – |

# FIG.6-11

sample: D/total D

|          | S3    | S4    | S5    | C3    | C4    | C5    | t test |
|----------|-------|-------|-------|-------|-------|-------|--------|
| His      | 0.54  | 0.45  | 0.56  | 1.00  | 0.67  | 0.65  | 0.094  |
| Asn      | 10.09 | 9.28  | 10.47 | 11.77 | 10.89 | 12.06 | 0.031  |
| Ser      | 12.23 | 18.66 | 15.17 | 19.24 | 22.48 | 25.23 | 0.052  |
| Gln      | 1.90  | —     | —     | —     | —     | —     | —      |
| Arg      | 42.08 | 22.98 | 43.51 | 16.18 | 15.98 | 14.14 | 0.035  |
| Asp      | 0.23  | 0.16  | 0.28  | 0.29  | 0.26  | 0.30  | 0.198  |
| Gly      | —     | —     | —     | —     | —     | —     | —      |
| allo-Thr | 2.04  | 1.27  | 1.87  | 1.81  | 1.81  | 1.78  | 0.752  |
| Glu      | 1.69  | 1.52  | 1.12  | 0.84  | 2.56  | 0.71  | 0.914  |
| Thr      | —     | —     | —     | —     | —     | —     | —      |
| Ala      | 7.64  | 23.28 | 12.22 | 21.84 | 21.66 | 17.77 | 0.279  |
| Pro      | 4.70  | 3.71  | 4.97  | 12.31 | 4.45  | 6.27  | 0.252  |
| Met      | 1.26  | 9.09  | 1.05  | 4.36  | 6.28  | 13.39 | 0.331  |
| Val      | 3.45  | 1.63  | 2.22  | 2.37  | 3.12  | 2.61  | 0.677  |
| allo-Ile | 6.42  | 2.01  | 3.37  | 3.68  | 3.63  | 2.61  | 0.666  |
| Ile      | —     | —     | —     | —     | —     | —     | —      |
| Leu      | 0.15  | 0.44  | 0.09  | 0.22  | 0.38  | 0.36  | 0.489  |
| Phe      | 0.34  | 0.43  | 0.20  | 0.41  | 0.55  | 0.53  | 0.090  |
| Trp      | —     | —     | —     | —     | —     | —     | —      |
| Lys      | 5.23  | 5.08  | 2.88  | 3.69  | 5.24  | 1.57  | 0.533  |
| Cys      | —     | —     | —     | —     | —     | —     | —      |
| Tyr      | —     | —     | —     | —     | —     | —     | —      |

# FIG.6-12

sample: D/D-Asn

| | S3 | S4 | S5 | C3 | C4 | C5 | t test |
|---|---|---|---|---|---|---|---|
| His | 5.38 | 4.80 | 5.31 | 8.51 | 6.17 | 5.40 | 0.183 |
| Asn | 100.00 | 100.00 | 100.00 | 100.00 | 100.00 | 100.00 | 0.320 |
| Ser | 121.22 | 201.05 | 144.87 | 163.54 | 206.53 | 209.25 | 0.251 |
| Gln | 18.84 | – | – | – | – | – | – |
| Arg | 416.96 | 247.53 | 415.47 | 137.52 | 146.78 | 117.31 | 0.016 |
| Asp | 2.30 | 1.77 | 2.69 | 2.44 | 2.39 | 2.46 | 0.545 |
| Gly | – | – | – | – | – | – | – |
| allo-Thr | 20.19 | 13.69 | 17.83 | 15.41 | 16.67 | 14.80 | 0.461 |
| Glu | 16.78 | 16.33 | 10.65 | 7.17 | 23.50 | 5.89 | 0.710 |
| Thr | – | – | – | – | – | – | – |
| °Ala | 75.71 | 250.80 | 116.67 | 185.62 | 198.94 | 147.39 | 0.620 |
| Pro | 46.54 | 39.93 | 47.41 | 104.66 | 40.84 | 51.97 | 0.345 |
| Met | 12.47 | 97.87 | 10.03 | 37.02 | 57.73 | 111.09 | 0.477 |
| Val | 34.22 | 17.61 | 21.23 | 20.11 | 28.64 | 21.63 | 0.883 |
| allo-Ile | 63.64 | 21.69 | 32.16 | 31.24 | 33.39 | 21.67 | 0.472 |
| Ile | – | – | – | – | – | – | – |
| Leu | 1.44 | 4.73 | 0.89 | 1.83 | 3.47 | 2.96 | 0.773 |
| Phe | 3.35 | 4.60 | 1.95 | 3.49 | 5.07 | 4.40 | 0.316 |
| Trp | – | – | – | – | – | – | – |
| Lys | 51.83 | 54.67 | 27.47 | 31.39 | 48.15 | 13.06 | 0.359 |
| Cys | – | – | – | – | – | – | – |
| Tyr | – | – | – | – | – | – | – |

# FIG.7-1

Ser (nmol/mL)

# FIG.7-2

%D

# FIG.7-3

# FIG.7-4

Ala (nmol/mL)

# FIG.7-5

%D

# FIG.7-6

Met (nmol/mL)

# FIG.7-7

%D

# FIG.7-8

D-Met /    D-Met /
D-allo-Thr    D-allo-Ile

# FIG.7-9

Leu (nmol/mL)

# FIG.7-10

%D

# FIG.7-11

D-Leu /    D-Leu /
D-allo-Thr    D-allo-Ile

# FIG.7-12

Asp (nmol/mL)

# FIG.7-13

%D

# FIG.7-14

Phe (nmol/mL)

D-Phe  L-Phe  Phe
n = 3
Average ± SE

Wild Hemi  Wild Hemi  Wild Hemi

# FIG.7-15

%D

Wild Hemi

# FIG.7-16

D-Phe /   D-Phe /
D-allo-Thr   D-allo-Ile

P<0.05
*

Wild Hemi  Wild Hemi

# FIG.8-1

sample: ddY/DAO+ urine-1acquired: 100107/1150    standard: 091221/2327

|  | D (height) | L (height) | D (fmol/inj) | L (fmol / inj) | D (nmol/mL) | L (nmol/mL) |
|---|---|---|---|---|---|---|
| His | 205 | 3229 | 37.3 | 804.6 | 3.73 | 80.46 |
| Asn | 36981 | 41076 | 616.3 | 799.3 | 61.63 | 79.93 |
| Ser | 46430 | 41717 | 778.1 | 812.1 | 77.81 | 81.21 |
| Gln | 5204 | 254689 | 84.4 | 4609.3 | 8.44 | 460.93 |
| Arg | 312 | 1544 | 171.1 | 882.8 | 17.11 | 88.28 |
| Asp | 108 | 4180 | 7.8 | 356.5 | 0.78 | 35.65 |
| Gly | – | 203612 | – | 3029.8 | – | 258.4 |
| allo-Thr | 6473 | nd | 79.2 | nd | 7.92 | nd |
| Glu | 653 | 35932 | 31.1 | 1942.4 | 3.11 | 194.24 |
| Thr | nd | 91402 | nd | 1364.9 | nd | 136.49 |
| Ala | 39695 | 182443 | 430.1 | 2478.8 | 43.01 | 247.88 |
| Pro | 7389 | 23098 | 246.7 | 899.3 | 24.67 | 89.93 |
| Met | 6962 | 12400 | 419.8 | 932.2 | 41.98 | 93.22 |
| Val | 2253 | 27246 | 41.7 | 556.7 | 4.17 | 55.67 |
| allo-Ile | 4241 | nd | 81.9 | nd | 8.19 | nd |
| Ile | nd | 15009 | nd | 246.7 | nd | 24.67 |
| Leu | 1125 | 76766 | 17.0 | 1252.4 | 1.70 | 125.24 |
| Phe | 1889 | 42878 | 20.1 | 522.1 | 2.01 | 52.21 |
| Trp | nd | nd | nd | nd | nd | nd |
| Lys | 2003 | 20140 | 112.4 | 1212.5 | 11.24 | 121.25 |
| Cys | nd | nd | nd | nd | nd | nd |
| Tyr | nd | 566 | nd | 1437.3 | nd | 143.73 |

# FIG.8-2

sample: ddY/DAO+ urine-2acquired: 100108/0152    standard: 091221/2327

|  | D (height) | L (height) | D (fmol/inj. | (fmol / in | (nmol/ml | (nmol/mL) |
|---|---|---|---|---|---|---|
| His | 526 | 7957 | 95.8 | 1982.8 | 9.58 | 198.28 |
| Asn | 103998 | 74346 | 1733.2 | 1446.7 | 173.32 | 144.67 |
| Ser | 124441 | 116029 | 2085.6 | 2258.7 | 208.56 | 225.87 |
| Gln | 13924 | 472122 | 225.7 | 8544.4 | 22.57 | 854.44 |
| Arg | 590 | 3610 | 323.5 | 2064.0 | 32.35 | 206.40 |
| Asp | 271 | 7742 | 19.6 | 660.4 | 1.96 | 66.04 |
| Gly | – | 850162 | – | 12650.5 | – | 1079.0 |
| allo–Thr | 21703 | nd | 265.4 | nd | 26.54 | nd |
| Glu | 1144 | 65140 | 54.5 | 3521.3 | 5.45 | 352.13 |
| Thr | nd | 424007 | nd | 6331.7 | nd | 633.17 |
| Ala | 83342 | 291070 | 903.0 | 3954.8 | 90.30 | 395.48 |
| Pro | 13549 | 37378 | 452.4 | 1455.4 | 45.24 | 145.54 |
| Met | 16112 | 42435 | 971.4 | 3190.2 | 97.14 | 319.02 |
| Val | 6519 | 87425 | 120.6 | 1786.2 | 12.06 | 178.62 |
| allo–Ile | 12804 | nd | 247.2 | nd | 24.72 | nd |
| Ile | nd | 47554 | nd | 781.6 | nd | 78.16 |
| Leu | 3247 | 273577 | 49.1 | 4463.1 | 4.91 | 446.31 |
| Phe | 4649 | 66725 | 49.5 | 812.5 | 4.95 | 81.25 |
| Trp | nd | nd | nd | nd | nd | nd |
| Lys | 2722 | 26832 | 152.8 | 1615.4 | 15.28 | 161.54 |
| Cys | nd | nd | nd | nd | nd | nd |
| Tyr | nd | 263 | nd | 667.9 | nd | 66.79 |

# FIG.8-3

sample: ddY/DAO+ urine–(acquired: 100121/0137    standard: 091221/2327

|  | D (height) | L (height) | D (fmol/inj) | L (fmol / inj) | D (nmol/mL) | L (nmol/mL) |
|---|---|---|---|---|---|---|
| His | 896 | 5024 | 163.1 | 1251.9 | 16.31 | 125.19 |
| Asn | 137509 | 31377 | 2291.7 | 610.6 | 229.17 | 61.06 |
| Ser | 83876 | 56621 | 1405.7 | 1102.2 | 140.57 | 110.22 |
| Gln | 15810 | 347321 | 256.3 | 6285.8 | 25.63 | 628.58 |
| Arg | 7233 | 5363 | 3965.5 | 3066.3 | 396.55 | 306.63 |
| Asp | 190 | 4095 | 13.7 | 349.3 | 1.37 | 34.93 |
| Gly | – | 632713 | – | 9414.8 | – | 803.0 |
| allo-Thr | 22827 | nd | 279.1 | nd | 27.91 | nd |
| Glu | 1799 | 26903 | 85.7 | 1454.3 | 8.57 | 145.43 |
| Thr | nd | 224739 | nd | 3356.0 | nd | 335.60 |
| Ala | 167673 | 139831 | 1816.8 | 1899.9 | 181.68 | 189.99 |
| Pro | 8143 | 10900 | 271.9 | 424.4 | 27.19 | 42.44 |
| Met | nd | 20964 | nd | 1576.0 | nd | 157.60 |
| Val | 23113 | 49573 | 427.6 | 1012.8 | 42.76 | 101.28 |
| allo-Ile | 46155 | nd | 891.1 | nd | 89.11 | nd |
| Ile | nd | 30207 | nd | 496.5 | nd | 49.65 |
| Leu | 2485 | 182629 | 37.6 | 2979.4 | 3.76 | 297.94 |
| Phe | 14621 | 37137 | 155.6 | 452.2 | 15.56 | 45.22 |
| Trp | nd | nd | nd | nd | nd | nd |
| Lys | 22592 | 21397 | 1268.1 | 1288.2 | 126.81 | 128.82 |
| Cys | nd | nd | nd | nd | nd | nd |
| Tyr | nd | 1849 | nd | 4695.3 | nd | 469.53 |

# FIG.8-4

sample: ddY/DAO- urine-1acquired: 100108/1553    standard: 091221/2327

| | D (height) | L (height) | D (fmol/inj) | L (fmol / inj) | D (nmol/mL) | L (nmol/mL) |
|---|---|---|---|---|---|---|
| His | 1572 | 7144 | 286.2 | 1780.2 | 28.62 | 178.02 |
| Asn | 147059 | 72585 | 2450.8 | 1412.4 | 245.08 | 141.24 |
| Ser | 810429 | 79698 | 13582.3 | 1551.5 | 1358.23 | 155.15 |
| Gln | 19762 | 570459 | 320.4 | 10324.1 | 32.04 | 1032.41 |
| Arg | 6072 | 4464 | 3328.9 | 2552.3 | 332.89 | 255.23 |
| Asp | 755 | 8502 | 54.5 | 725.2 | 5.45 | 72.52 |
| Gly | – | 420666 | – | 6259.5 | – | 533.9 |
| allo-Thr | 37582 | nd | 459.6 | nd | 45.96 | nd |
| Glu | 2171 | 55318 | 103.4 | 2990.3 | 10.34 | 299.03 |
| Thr | nd | 228597 | nd | 3413.6 | nd | 341.36 |
| Ala | 4098335 | 236056 | 44406.2 | 3207.3 | 4440.62 | 320.73 |
| Pro | 150921 | 25428 | 5039.4 | 990.1 | 503.94 | 99.01 |
| Met | 828235 | 27891 | 49937.0 | 2096.8 | 4993.70 | 209.68 |
| Val | 24422 | 75055 | 451.9 | 1533.4 | 45.19 | 153.34 |
| allo-Ile | 34027 | nd | 656.9 | nd | 65.69 | nd |
| Ile | nd | 41573 | nd | 683.3 | nd | 68.33 |
| Leu | 169421 | 184833 | 2564.5 | 3015.4 | 256.45 | 301.54 |
| Phe | 37312 | 69310 | 397.1 | 843.9 | 39.71 | 84.39 |
| Trp | nd | nd | nd | nd | nd | nd |
| Lys | 6243 | 46412 | 350.4 | 2794.2 | 35.04 | 279.42 |
| Cys | nd | nd | nd | nd | nd | nd |
| Tyr | nd | 495 | nd | 1257.0 | nd | 125.70 |

# FIG.8-5

sample: ddY/DAO- urine-2acquired: 100109/0555    standard: 091221/2327

| | D (height) | L (height) | D (fmol/inj) | L (fmol / inj) | D (nmol/mL) | L (nmol/mL) |
|---|---|---|---|---|---|---|
| His | 1390 | 5964 | 253.1 | 1486.2 | 25.31 | 148.62 |
| Asn | 135552 | 71605 | 2259.0 | 1393.3 | 225.90 | 139.33 |
| Ser | 787541 | 76908 | 13198.7 | 1497.1 | 1319.87 | 149.71 |
| Gln | 19040 | 511922 | 308.6 | 9264.7 | 30.86 | 926.47 |
| Arg | 5542 | 3731 | 3038.4 | 2133.2 | 303.84 | 213.32 |
| Asp | 408 | 8158 | 29.5 | 695.8 | 2.95 | 69.58 |
| Gly | – | 422775 | – | 6290.9 | – | 536.6 |
| allo-Thr | 36705 | nd | 448.8 | nd | 44.88 | nd |
| Glu | 1890 | 55214 | 90.0 | 2984.7 | 9.00 | 298.47 |
| Thr | nd | 225423 | nd | 3366.2 | nd | 336.62 |
| Ala | 4524703 | 227707 | 49026.0 | 3093.8 | 4902.60 | 309.38 |
| Pro | 125826 | 24174 | 4201.5 | 941.2 | 420.15 | 94.12 |
| Met | 727290 | 26930 | 43850.7 | 2024.5 | 4385.07 | 202.45 |
| Val | 27073 | 70291 | 500.9 | 1436.1 | 50.09 | 143.61 |
| allo-Ile | 40478 | nd | 781.5 | nd | 78.15 | nd |
| Ile | nd | 40793 | nd | 670.5 | nd | 67.05 |
| Leu | 168716 | 179624 | 2553.8 | 2930.4 | 255.38 | 293.04 |
| Phe | 39803 | 63484 | 423.6 | 773.0 | 42.36 | 77.30 |
| Trp | nd | nd | nd | nd | nd | nd |
| Lys | 5083 | 39403 | 285.3 | 2372.2 | 28.53 | 237.22 |
| Cys | nd | nd | nd | nd | nd | nd |
| Tyr | nd | 498 | nd | 1264.6 | nd | 126.46 |

# FIG.8-6

sample: ddY/DAO- urine (acquired: 100121/1539     standard: 091221/2327

|  | D (height) | L (height) | D (fmol/inj) | L (fmol / inj) | D (nmol/mL) | L (nmol/mL) |
|---|---|---|---|---|---|---|
| His | 3215 | 3628 | 585.4 | 904.1 | 58.54 | 90.41 |
| Asn | 133803 | 32277 | 2229.9 | 628.1 | 222.99 | 62.81 |
| Ser | 672264 | 47192 | 11266.7 | 918.7 | 1126.67 | 91.87 |
| Gln | 29985 | 288576 | 486.1 | 5222.6 | 48.61 | 522.26 |
| Arg | 12430 | 5485 | 6814.7 | 3136.1 | 681.47 | 313.61 |
| Asp | 350 | 6090 | 25.3 | 519.4 | 2.53 | 51.94 |
| Gly | – | 414565 | – | 6168.8 | – | 526.2 |
| allo-Thr | 38905 | nd | 475.8 | nd | 47.58 | nd |
| Glu | 1792 | 28297 | 85.3 | 1529.7 | 8.53 | 152.97 |
| Thr | nd | 154345 | nd | 2304.8 | nd | 230.48 |
| Ala | 5207589 | 154249 | 56425.1 | 2095.8 | 5642.51 | 209.58 |
| Pro | 253001 | 8203 | 8448.0 | 319.4 | 844.80 | 31.94 |
| Met | 14075 | 4103 | 848.6 | 308.5 | 84.86 | 30.85 |
| Val | 55980 | 83980 | 1035.7 | 1715.8 | 103.57 | 171.58 |
| allo-Ile | 80327 | nd | 1550.8 | nd | 155.08 | nd |
| Ile | nd | 46110 | nd | 757.9 | nd | 75.79 |
| Leu | 219867 | 162732 | 3328.1 | 2654.8 | 332.81 | 265.48 |
| Phe | 52095 | 37548 | 554.4 | 457.2 | 55.44 | 45.72 |
| Trp | nd | nd | nd | nd | nd | nd |
| Lys | 15146 | 14045 | 850.2 | 845.6 | 85.02 | 84.56 |
| Cys | nd | nd | nd | nd | nd | nd |
| Tyr | nd | 83 | nd | 210.8 | nd | 21.08 |

# FIG.8-7

sample: D-AMINO ACID

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 3.73 | 9.58 | 16.31 | 28.62 | 25.31 | 58.54 | 0.069 |
| Asn | 61.63 | 173.32 | 229.17 | 245.08 | 225.90 | 222.99 | 0.198 |
| Ser | 77.81 | 208.56 | 140.57 | 1358.23 | 1319.87 | 1126.67 | 0.000 |
| Gln | 8.44 | 22.57 | 25.63 | 32.04 | 30.86 | 48.61 | – |
| Arg | 17.11 | 32.35 | 396.55 | 332.89 | 303.84 | 681.47 | 0.169 |
| Asp | 0.78 | 1.96 | 1.37 | 5.45 | 2.95 | 2.53 | 0.080 |
| Gly | – | – | – | – | – | – | – |
| allo-Thr | 7.92 | 26.54 | 27.91 | 45.96 | 44.88 | 47.58 | 0.018 |
| Glu | 3.11 | 5.45 | 8.57 | 10.34 | 9.00 | 8.53 | 0.099 |
| Thr | nd | nd | nd | nd | nd | nd | – |
| Ala | 43.01 | 90.30 | 181.68 | 4440.62 | 4902.60 | 5642.51 | 0.000 |
| Pro | 24.67 | 45.24 | 27.19 | 503.94 | 420.15 | 844.80 | 0.013 |
| Met | 41.98 | 97.14 | nd | 4993.70 | 4385.07 | 84.86 | 0.220 |
| Val | 4.17 | 12.06 | 42.76 | 45.19 | 50.09 | 103.57 | 0.103 |
| allo-Ile | 8.19 | 24.72 | 89.11 | 65.69 | 78.15 | 155.08 | 0.189 |
| Ile | nd | nd | nd | nd | nd | nd | – |
| Leu | 1.70 | 4.91 | 3.76 | 256.45 | 255.38 | 332.81 | 0.000 |
| Phe | 2.01 | 4.95 | 15.56 | 39.71 | 42.36 | 55.44 | 0.004 |
| Trp | nd | nd | nd | nd | nd | nd | – |
| Lys | 11.24 | 15.28 | 126.81 | 35.04 | 28.53 | 85.02 | 0.972 |
| Cys | nd | nd | nd | nd | nd | nd | – |
| Tyr | nd | nd | nd | nd | nd | nd | – |

# FIG.8-8

sample: L-AMINO ACID

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 80.46 | 198.28 | 125.19 | 178.02 | 148.62 | 90.41 | 0.924 |
| Asn | 79.93 | 144.67 | 61.06 | 141.24 | 139.33 | 62.81 | 0.623 |
| Ser | 81.21 | 225.87 | 110.22 | 155.15 | 149.71 | 91.87 | 0.895 |
| Gln | 460.93 | 854.44 | 628.58 | 1032.41 | 926.47 | 522.26 | 0.406 |
| Arg | 88.28 | 206.40 | 306.63 | 255.23 | 213.32 | 313.61 | 0.435 |
| Asp | 35.65 | 66.04 | 34.93 | 72.52 | 69.58 | 51.94 | 0.189 |
| Gly | 258.42 | 1079.02 | 803.04 | 533.91 | 536.58 | 526.16 | 0.494 |
| allo-Thr | nd | nd | nd | nd | nd | nd | – |
| Glu | 194.24 | 352.13 | 145.43 | 299.03 | 298.47 | 152.97 | 0.817 |
| Thr | 136.49 | 633.17 | 335.60 | 341.36 | 336.62 | 230.48 | 0.682 |
| Ala | 247.88 | 395.48 | 189.99 | 320.73 | 309.38 | 209.58 | 0.978 |
| Pro | 89.93 | 145.54 | 42.44 | 99.01 | 94.12 | 31.94 | 0.657 |
| Met | 93.22 | 319.02 | 157.60 | 209.68 | 202.45 | 30.85 | 0.660 |
| Val | 55.67 | 178.62 | 101.28 | 153.34 | 143.61 | 171.58 | 0.295 |
| allo-Ile | nd | nd | nd | nd | nd | nd | – |
| Ile | 24.67 | 78.16 | 49.65 | 68.33 | 67.05 | 75.79 | 0.281 |
| Leu | 125.24 | 446.31 | 297.94 | 301.54 | 293.04 | 265.48 | 0.975 |
| Phe | 52.21 | 81.25 | 45.22 | 84.39 | 77.30 | 45.72 | 0.586 |
| Trp | nd | nd | nd | nd | nd | nd | – |
| Lys | 121.25 | 161.54 | 128.82 | 279.42 | 237.22 | 84.56 | 0.355 |
| Cys | nd | nd | nd | nd | nd | nd | – |
| Tyr | 143.73 | 66.79 | 469.53 | 125.70 | 126.46 | 21.08 | 0.350 |

# FIG.8-9

sample: D/(D+L)

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 4.43 | 4.61 | 11.53 | 13.85 | 14.55 | 39.30 | 0.145 |
| Asn | 43.54 | 54.51 | 78.96 | 63.44 | 61.85 | 78.02 | 0.494 |
| Ser | 48.93 | 48.01 | 56.05 | 89.75 | 89.81 | 92.46 | 0.000 |
| Gln | 1.80 | – | – | – | – | – | – |
| Arg | 16.23 | 13.55 | 56.39 | 56.60 | 58.75 | 68.48 | 0.086 |
| Asp | 2.14 | 2.88 | 3.78 | 6.99 | 4.06 | 4.64 | 0.086 |
| Gly | – | – | – | – | – | – | – |
| allo-Thr | 5.48 | 4.02 | 7.68 | 11.87 | 11.77 | 17.11 | 0.019 |
| Glu | 1.58 | 1.52 | 5.56 | 3.34 | 2.93 | 5.28 | 0.561 |
| Thr | to allo-Thr | to allo-Thr | to allo-Thr | to allo-Thr | to allo-Thr | to allo-Thr | to allo-Thr |
| Ala | 14.79 | 18.59 | 48.88 | 93.26 | 94.06 | 96.42 | 0.003 |
| Pro | 21.53 | 23.71 | 39.05 | 83.58 | 81.70 | 96.36 | 0.001 |
| Met | 31.05 | 23.34 | – | 95.97 | 95.59 | 73.34 | – |
| Val | 6.97 | 6.33 | 29.69 | 22.76 | 25.86 | 37.64 | 0.181 |
| allo-Ile | 24.92 | 24.03 | 64.22 | 49.02 | 53.82 | 67.17 | 0.256 |
| Ile | to allo-Ile | to allo-Ile | to allo-Ile | to allo-Ile | to allo-Ile | to allo-Ile | to allo-Ile |
| Leu | 1.34 | 1.09 | 1.25 | 45.96 | 46.57 | 55.63 | 0.000 |
| Phe | 3.71 | 5.74 | 25.60 | 32.00 | 35.40 | 54.80 | 0.043 |
| Trp | – | – | – | – | – | – | – |
| Lys | 8.49 | 8.64 | 49.61 | 11.14 | 10.74 | 50.14 | 0.930 |
| Cys | – | – | – | – | – | – | – |
| Tyr | – | – | – | – | – | – | – |

# FIG.8-10

sample: D/total L

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 0.17 | 0.17 | 0.46 | 0.63 | 0.60 | 1.98 | 0.159 |
| Asn | 2.77 | 3.11 | 6.43 | 5.42 | 5.32 | 7.54 | 0.221 |
| Ser | 3.50 | 3.75 | 3.94 | 30.02 | 31.11 | 38.09 | 0.000 |
| Gln | 0.38 | 0.41 | 0.72 | 0.71 | 0.73 | 1.64 | – |
| Arg | 0.77 | 0.58 | 11.13 | 7.36 | 7.16 | 23.04 | 0.256 |
| Asp | 0.04 | 0.04 | 0.04 | 0.12 | 0.07 | 0.09 | 0.021 |
| Gly | – | – | – | – | – | – | – |
| allo-Thr | 0.36 | 0.48 | 0.78 | 1.02 | 1.06 | 1.61 | 0.040 |
| Glu | 0.14 | 0.10 | 0.24 | 0.23 | 0.21 | 0.29 | 0.158 |
| Thr | – | – | – | – | – | – | – |
| Ala | 1.93 | 1.62 | 5.10 | 98.14 | 115.55 | 190.75 | 0.010 |
| Pro | 1.11 | 0.81 | 0.76 | 11.14 | 9.90 | 28.56 | 0.060 |
| Met | 1.89 | 1.75 | – | 110.36 | 103.35 | 2.87 | – |
| Val | 0.19 | 0.22 | 1.20 | 1.00 | 1.18 | 3.50 | 0.194 |
| allo-Ile | 0.37 | 0.44 | 2.50 | 1.45 | 1.84 | 5.24 | 0.279 |
| Ile | – | – | – | – | – | – | – |
| Leu | 0.08 | 0.09 | 0.11 | 5.67 | 6.02 | 11.25 | 0.014 |
| Phe | 0.09 | 0.09 | 0.44 | 0.88 | 1.00 | 1.87 | 0.035 |
| Trp | – | – | – | – | – | – | – |
| Lys | 0.51 | 0.27 | 3.56 | 0.77 | 0.67 | 2.87 | 0.997 |
| Cys | – | – | – | – | – | – | – |
| Tyr | – | – | – | – | – | – | – |

# FIG.8-11

sample: D/total D

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 1.17 | 1.24 | 1.22 | 0.23 | 0.21 | 0.62 | 0.003 |
| Asn | 19.38 | 22.36 | 17.19 | 1.97 | 1.86 | 2.35 | 0.000 |
| Ser | 24.47 | 26.91 | 10.55 | 10.92 | 10.89 | 11.86 | 0.139 |
| Gln | 2.65 | 2.91 | 1.92 | 0.26 | 0.25 | 0.51 | 0.002 |
| Arg | 5.38 | 4.17 | 29.75 | 2.68 | 2.51 | 7.17 | 0.349 |
| Asp | 0.25 | 0.25 | 0.10 | 0.04 | 0.02 | 0.03 | 0.026 |
| Gly | – | – | – | – | – | – | – |
| allo-Thr | 2.49 | 3.42 | 2.09 | 0.37 | 0.37 | 0.50 | 0.005 |
| Glu | 0.98 | 0.70 | 0.64 | 0.08 | 0.07 | 0.09 | 0.003 |
| Thr | – | – | – | – | – | – | – |
| Ala | 13.53 | 11.65 | 13.63 | 35.70 | 40.43 | 59.39 | 0.011 |
| Pro | 7.76 | 5.84 | 2.04 | 4.05 | 3.47 | 8.89 | 0.920 |
| Met | 13.20 | 12.53 | – | 40.15 | 36.17 | 0.89 | 0.483 |
| Val | 1.31 | 1.56 | 3.21 | 0.36 | 0.41 | 1.09 | 0.094 |
| allo-Ile | 2.57 | 3.19 | 6.68 | 0.53 | 0.64 | 1.63 | 0.073 |
| Ile | – | – | – | – | – | – | – |
| Leu | 0.54 | 0.63 | 0.28 | 2.06 | 2.11 | 3.50 | 0.013 |
| Phe | 0.63 | 0.64 | 1.17 | 0.32 | 0.35 | 0.58 | 0.114 |
| Trp | – | – | – | – | – | – | – |
| Lys | 3.54 | 1.97 | 9.51 | 0.28 | 0.24 | 0.89 | 0.121 |
| Cys | – | – | – | – | – | – | – |
| Tyr | – | – | – | – | – | – | – |

# FIG.8-12

sample: D/D-Asn

| | DAO+/+ 1 | DAO+/+ 2 | DAO+/+ 3 | DAO-/- 1 | DAO-/- 2 | DAO-/- 3 | t test |
|---|---|---|---|---|---|---|---|
| His | 6.06 | 5.53 | 7.12 | 11.68 | 11.20 | 26.25 | 0.110 |
| Asn | 100.00 | 100.00 | 100.00 | 100.00 | 100.00 | 100.00 | 0.286 |
| Ser | 126.26 | 120.33 | 61.34 | 554.20 | 584.27 | 505.26 | 0.000 |
| Gln | 13.69 | 13.02 | 11.18 | 13.07 | 13.66 | 21.80 | – |
| Arg | 27.75 | 18.66 | 173.04 | 135.83 | 134.50 | 305.61 | 0.192 |
| Asp | 1.27 | 1.13 | 0.60 | 2.22 | 1.30 | 1.13 | 0.232 |
| Gly | – | – | – | – | – | – | – |
| allo-Thr | 12.84 | 15.31 | 12.18 | 18.75 | 19.87 | 21.34 | 0.006 |
| Glu | 5.05 | 3.14 | 3.74 | 4.22 | 3.98 | 3.83 | 0.955 |
| Thr | – | – | – | – | – | – | – |
| Ala | 69.79 | 52.10 | 79.28 | 1811.91 | 2170.25 | 2530.39 | 0.001 |
| Pro | 40.03 | 26.10 | 11.86 | 205.62 | 185.99 | 378.85 | 0.020 |
| Met | 68.11 | 56.05 | – | 2037.58 | 1941.15 | 38.06 | 0.226 |
| Val | 6.76 | 6.96 | 18.66 | 18.44 | 22.17 | 46.45 | 0.131 |
| allo-Ile | 13.29 | 14.26 | 38.88 | 26.81 | 34.59 | 69.55 | 0.240 |
| Ile | – | – | – | – | – | – | – |
| Leu | 2.76 | 2.84 | 1.64 | 104.64 | 113.05 | 149.25 | 0.001 |
| Phe | 3.26 | 2.85 | 6.79 | 16.20 | 18.75 | 24.86 | 0.005 |
| Trp | – | – | – | – | – | – | – |
| Lys | 18.24 | 8.82 | 55.34 | 14.30 | 12.63 | 38.13 | 0.743 |
| Cys | – | – | – | – | – | – | – |
| Tyr | – | – | – | – | – | – | – |

# FIG.9-1

**Area % Report**
Data File:        C:\EZChrom Elite\Enterprise\Projects\AAA\data\100302\2010-03-09 21-44-47 AAA DDO+ urine 2uL 1D ML-1000 3G-K001 45C  PA5u PC20u PD75%MeCN 2D OA250015250-S042 R028 25C 2D 012.dat
Acquired:        2010/03/09 21:46:01

1st Dimension, Reversed-phase separation

2 nd Dimension, Enantiomer separation

His

Asn

Ser

Gln

Arg

Asp

# FIG.9-2

**Area % Report**
Data File:     C:\EZChrom Elite\Enterprise\Projects\AAA\data\100302\2010-03-09 21-44-47 AAA DDO
urine 2uL 1D ML-1000 3G-K001 45C  PA5u PC20u PD75%MeCN 2D OA250015250-S042 R028 25C 2D
012.dat
Acquired:     2010/03/09 21:46:01

1st Dimension, Reversed-phase separation

2 nd Dimension, Enantiomer separation

Gly

a-Thr

Glu

Thr

Ala

Pro

# FIG.9-3

**Area % Report**

Data File:  C:\EZChrom Elite\Enterprise\Projects\AAA\data\100302\2010-03-09 21-44-47 AAA DDO+ urine 2uL 1D ML-1000 3G-K001 45C PA5u PC20u PD75%MeCN 2D OA250015250-S042 R028 25C 2D 012.dat

Acquired:  2010/03/09 21:46:01

1st Dimension, Reversed-phase separation

2 nd Dimension, Enantiomer separation

Met

Val

aIle

Ile

Leu

Phe

85

# FIG.9-4

Area % Report
Data File: C:\EZChrom Elite\Enterprise\Projects\AAA\data\100302\2010-03-09 21-44-47 AAA DDO+
urine 2uL 1D ML-1000 3G-K001 45C PA5u PC20u PD75%MeCN 2D OA250015250-S042 R028 25C 2D
012.dat
Acquired: 2010/03/09 21:46:01

1st Dimension, Reversed-phase separation

2 nd Dimension, Enantiomer separation

Trp

Lys

Cys

Tyr

# FIG.9-5

Area % Report
Data File:      C:\EZChrom Elite\Enterprise\Projects\AAA\data\100302\2010-03-10 11-46-28 AAA DDO-
urine 2uL 1D ML-1000 3G-K001 45C  PA5u PC20u PD75%MeCN 2D OA250015250-S042 R028 25C 2D
013.dat
Acquired:      2010/03/10 11:47:41

1st Dimension, Reversed-phase separation

2 nd Dimension, Enantiomer separation

His

Asn

Ser

Gln

Arg

Asp

EP 3 663 758 A1

# FIG.9-6

**Area % Report**

Data File:        C:\EZChrom Elite\Enterprise\Projects\AAA\data\100302\2010-03-10 11-46-28 AAA_DDO-urine 2uL 1D ML-1000 3G-K001 45C  PA5u PC20u PD75%MeCN 2D OA250015250-S042 R028 25C 2D 013.dat

Acquired:        2010/03/10 11:47:41

1st Dimension,  Reversed-phase separation

2 nd Dimension, Enantiomer separation

Gly

a-Thr

Glu

Thr

Ala

Pro

# FIG.9-7

**Area % Report**

Data File:         C:\EZChrom Elite\Enterprise\Projects\AAA\data\100302\2010-03-10 11-46-28 AAA DDO-urine 2uL 1D ML-1000 3G-K001 45C PA5u PC20u PD75%MeCN 2D OA250015250-S042 R028 25C 2D 013.dat

Acquired:          2010/03/10 11:47:41

1st Dimension, Reversed-phase separation

2 nd Dimension, Enantiomer separation

Met

Val

aIle

Ile

Leu

Phe

# FIG.9-8

**Area % Report**

Data File:　　C:\EZChrom Elite\Enterprise\Projects\AAA\data\100302\2010-03-10 11-46-28 AAA DDO-urine 2uL 1D ML-1000 3G-K001 45C PA5u PC20u PD75%MeCN 2D OA250015250-S042 R028 25C 2D 013.dat

Acquired:　　2010/03/10 11:47:41

1st Dimension, Reversed-phase separation

2nd Dimension, Enantiomer separation

Trp

Lys

Cys

Tyr

# FIG.10-1

*•D-form*

D-Val

# FIG.10-2

D-*allo*-Ile

# FIG.10-3

D-Ile

# FIG.10-4

D-Leu

# FIG.10-5

D-Phe

# FIG.10-6

*•L-form*

L-Val

# FIG.10-7

L-*allo*-Ile

# FIG.10-8

L-Ile

# FIG.10-9

L-Leu

# FIG.10-10

L-Phe

# FIG.10-11

*D-form*

(nmol/mL)

D-Val

# FIG.10-12

D-*allo*-Ile

# FIG.10-13

D-Ile

# FIG.10-14

D-Leu

# FIG.10-15

D-Phe

# FIG.10-16

### ▪L-form

(nmol/mL)

L-Val

# FIG.10-17

L-allo-Ile

# FIG.10-18

L-Ile

# FIG.10-19

# FIG.10-20

# FIG.11-1

# FIG.11-2

# FIG.11-3

# FIG.11-4

# FIG.11-5

# FIG.11-6

### *L-form*

# FIG.11-7

# FIG.11-8

# FIG.11-9

# FIG.11-10

# FIG.11-11

*D-form*

# FIG.11-12

*L-form*

# FIG.11-13

# FIG.11-14

# FIG.11-15

# FIG.12-1

# FIG.12-2

# FIG.12-3

# FIG.12-4

# FIG.12-5

# FIG.12-6

## *L-form*

# FIG.12-7

# FIG.12-8

# FIG.12-9

# FIG.12-11

## *D-form*

# FIG.12-10

# FIG.12-12

*L-form*

# FIG.12-13

# FIG.12-14

# FIG.12-15

# FIG.13-1

| D-AMINO ACID | His | Asn | Ser | Gln | Arg | Asp | Gly | allo L-Thr | allo D-Thr | Glu | Thr | Ala | Pro | Met | Val | allo D-Ile | Ile | Leu | Phe | Trp | Lys | Cys | Tyr |
|---|---|---|---|---|---|---|---|---|---|---|---|---|---|---|---|---|---|---|---|---|---|---|---|
| ARTICULAR RHEUMATISM | — | — | — | — | — | — | — | — | — | — | — | — | — | — | — | — | — | — | — | — | — | — | — |
| KIDNEY CANCER | — | — | ▨ | — | — | — | — | — | — | — | — | ▨ | — | — | — | — | — | — | — | — | — | — | — |
| LUNG CANCER | — | — | — | — | — | — | — | — | — | — | — | ▨ | — | — | — | — | — | — | — | — | — | — | — |
| CARDIOVASCULAR DISEASE | — | — | — | — | — | — | — | — | — | — | — | — | — | — | — | — | — | — | — | — | — | — | — |
| MULTIPLE SCLEROSIS | — | — | ▨ | — | — | — | — | — | — | — | — | — | — | — | — | — | — | — | — | — | — | — | — |
| ACUTE MYELOID LEUKEMIA | — | — | — | — | — | — | — | — | — | — | — | — | — | — | — | — | — | — | — | — | — | — | — |
| LYMPHOMA | — | — | — | — | — | — | — | — | — | — | — | — | — | — | — | — | — | — | — | — | — | — | — |
| ACUTE LYMPHOCYTIC LEUKEMIA | — | — | — | — | — | — | — | — | — | — | — | — | — | — | — | — | — | — | — | — | — | — | — |
| SCABIES | — | — | — | — | — | — | — | — | — | — | — | — | — | — | — | — | — | — | — | — | — | — | — |
| DIABETES | — | — | — | — | — | — | — | — | — | — | — | ▨ | — | — | — | — | — | — | — | — | — | — | — |

# FIG.13-2

| L-AMINO ACID | His | Asn | Ser | Gln | Arg | Asp | Gly | allo L–Thr | allo D–Thr | Glu | Thr | Ala | Pro | Met | Val | allo D–Ile | Ile | Leu | Phe | Trp | Lys | Cys | Tyr |
|---|---|---|---|---|---|---|---|---|---|---|---|---|---|---|---|---|---|---|---|---|---|---|---|
| ARTICULAR RHEUMATISM | — | — | — | 1 | — | — | — | — | — | 1 | — | — | — | — | — | — | — | — | — | — | — | 1 | — |
| KIDNEY CANCER | — | — | — | — | — | — | — | — | — | — | — | — | — | — | — | — | — | — | — | — | — | — | — |
| LUNG CANCER | — | — | — | — | — | — | — | — | — | — | — | — | — | — | — | — | — | — | — | — | — | — | — |
| CARDIOVASCULAR DISEASE | — | — | — | — | 1 | — | — | — | — | 1 | — | — | — | — | — | — | — | — | — | — | — | — | — |
| MULTIPLE SCLEROSIS | — | — | — | — | — | — | — | — | — | — | — | — | — | — | — | — | — | — | — | — | — | 1 | — |
| ACUTE MYELOID LEUKEMIA | — | — | — | — | — | — | — | — | — | — | — | — | — | — | — | — | — | — | — | — | — | 1 | — |
| LYMPHOMA | — | — | — | — | — | — | — | — | — | — | — | — | — | — | — | — | — | — | — | — | — | 1 | — |
| ACUTE LYMPHOCYTIC LEUKEMIA | — | — | — | — | — | — | — | — | — | 1 | — | — | — | — | — | — | — | — | — | — | — | 1 | — |
| SCABIES | — | — | — | — | 1 | — | — | — | — | — | — | — | — | — | — | — | — | — | — | — | — | 1 | — |
| DIABETES | — | — | — | — | — | — | — | — | — | 1 | — | — | — | — | — | — | — | — | — | — | — | 1 | — |

Europäisches
Patentamt

European
Patent Office

Office européen
des brevets

# EUROPEAN SEARCH REPORT

Application Number

EP 20 15 2640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAGDALENA C. WALDHIER ET AL: "Improved enantiomer resolution and quantification of free d-amino acids in serum and urine by comprehensive two-dimensional gas chromatography-time-of-flight mass spectrometry", JOURNAL OF CHROMATOGRAPHY A, vol. 1218, no. 28, 1 July 2011 (2011-07-01), pages 4537-4544, XP055166406, ISSN: 0021-9673, DOI: 10.1016/j.chroma.2011.05.039 * the whole document * * abstract * * especially chaper 2, 2.1-2.7, 3.3, 3.4, figure 5; figures 3-6; tables 1-4 * ----- | 1-9 | INV. G01N33/48 G01N33/68 G01N30/88 G16B20/00 G16H50/30 |
| X | HAMASE K ET AL: "d-Amino acids in mammals and their diagnostic value", JOURNAL OF CHROMATOGRAPHY B: BIOMEDICAL SCIENCES & APPLICATIONS, ELSEVIER, AMSTERDAM, NL, vol. 781, no. 1-2, 5 December 2002 (2002-12-05), pages 73-91, XP004394149, ISSN: 1570-0232, DOI: 10.1016/S1570-0232(02)00690-6 * the whole document * * abstract * * page 79, paragraph 2.2.3 - page 86, paragraph 4.2; figures 3,4 * * chapters 6 and 7; page 87 - page 89; table 5 * ----- -/-- | 1-9 | TECHNICAL FIELDS SEARCHED (IPC) G01N G16B G16H |

The present search report has been drawn up for all claims

| Place of search | Date of completion of the search | Examiner |
|---|---|---|
| The Hague | 14 February 2020 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 15 2640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YURIKA MIYOSHI ET AL: "Simultaneous two-dimensional HPLC determination of free-serine and-alanine in the brain and periphery of mutant rats lacking-amino-acid oxidase", JOURNAL OF CHROMATOGRAPHY B: BIOMEDICAL SCIENCES & APPLICATIONS, ELSEVIER, AMSTERDAM, NL, vol. 879, no. 29, 16 August 2010 (2010-08-16), pages 3184-3189, XP028324122, ISSN: 1570-0232, DOI: 10.1016/J.JCHROMB.2010.08.024 [retrieved on 2010-08-22] * the whole document * | 1 | |
| X | FUKUSHIMA T ET AL: "DETERMINATION OF D-AMINO ACIDS IN SERUM FROM PATIENTS WITH RENAL DYSFUNCTION", BIOLOGICAL & PHARMACEUTICAL BULLETIN (OF JAPAN), PHARMACEUTICAL SOCIETY OF JAPAN, TOKYO, JP, vol. 18, no. 8, 1 August 1995 (1995-08-01), pages 1130-1132, XP008073859, ISSN: 0918-6158 * the whole document * | 1-9 | TECHNICAL FIELDS SEARCHED (IPC) |

-/--

The present search report has been drawn up for all claims

| Place of search | Date of completion of the search | Examiner |
|---|---|---|
| The Hague | 14 February 2020 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt
European
Patent Office
Office européen
des brevets

**EUROPEAN SEARCH REPORT**

Application Number

EP 20 15 2640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HAMASE ET AL: "Comprehensive analysis of branched aliphatic d-amino acids in mammals using an integrated multi-loop two-dimensional column-switching high-performance liquid chromatographic system combining reversed-phase and enantioselective columns", JOURNAL OF CHROMATOGRAPHY, ELSEVIER SCIENCE PUBLISHERS B.V, NL, vol. 1143, no. 1-2, 8 February 2007 (2007-02-08), pages 105-111, XP005879718, ISSN: 0021-9673, DOI: 10.1016/J.CHROMA.2006.12.078 * the whole document * * abstract * * chapter 2, especially souchapter 2.4; pages 106-107; figure 2 * * figures 1,3,4; tables 1-3 * | 1-9 | |
| Y | WO 2004/081527 A2 (SIONEX CORP [US]; DRAPER LAB CHARLES S [US]; MILLER RAANAN A [US]; ZAP) 23 September 2004 (2004-09-23) * the whole document * * page 3, line 20 - page 6, line 8; figures 29,30 * * page 32, paragraph 26 - page 34, line 22; claims 1-51; example 8 * | 1-9 | TECHNICAL FIELDS SEARCHED (IPC) |

-/--

The present search report has been drawn up for all claims

| Place of search | Date of completion of the search | Examiner |
|---|---|---|
| The Hague | 14 February 2020 | Boiangiu, Clara |

Europäisches
Patentamt

European
Patent Office

Office européen
des brevets

# EUROPEAN SEARCH REPORT

Application Number

EP 20 15 2640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANTIMO D?ANIELLO ET AL: "Occurrence of D-aspartic acid and N-methyl-D-aspartic acid in rat neuroendocrine tissues and their role in the modulation of luteinizing hormone and growth hormone release", THE FASEB JOURNAL, vol. 14, no. 5, 1 January 2000 (2000-01-01), pages 699-714, XP055121214, ISSN: 0892-6638 * the whole document * * abstract; figures 1-7; tables 1-5 * | 1-9 | |
| Y | Toru Nishikawa: "Metabolism and Functional Roles of Endogenous D-Serine in Mammalian Brains", Biol Pharm Bull, 9 May 2005 (2005-05-09), pages 1561-1565, XP055222788, Retrieved from the Internet: URL:https://www.jstage.jst.go.jp/article/bpb/28/9/28_9_1561/_pdf [retrieved on 2015-10-21] * the whole document * | 1-9 | |
| Y | US 2009/075284 A1 (CHINNAIYAN ARUL M [US] ET AL) 19 March 2009 (2009-03-19) * the whole document * | 1-9 | |
| Y | EP 2 249 161 A1 (BIOCRATES LIFE SCIENCES AG [AT]) 10 November 2010 (2010-11-10) * the whole document * | 1-9 | |
| Y | WO 00/70329 A1 (UNIV BRANDEIS [US]; STANTON MARTIN [US]; WENSINK PIETER [US]; STEWART) 23 November 2000 (2000-11-23) * the whole document * | 1-9 | |

TECHNICAL FIELDS
SEARCHED       (IPC)

The present search report has been drawn up for all claims

| Place of search | Date of completion of the search | Examiner |
|---|---|---|
| The Hague | 14 February 2020 | Boiangiu, Clara |

EPO FORM 1503 03.82 (P04C01)

## ANNEX TO THE EUROPEAN SEARCH REPORT
## ON EUROPEAN PATENT APPLICATION NO.

EP 20 15 2640

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2020

| Patent document cited in search report | | Publication date | Patent family member(s) | | Publication date |
|---|---|---|---|---|---|
| WO 2004081527 | A2 | 23-09-2004 | CA | 2518703 A1 | 23-09-2004 |
| | | | EP | 1601948 A2 | 07-12-2005 |
| | | | WO | 2004081527 A2 | 23-09-2004 |
| US 2009075284 | A1 | 19-03-2009 | US | 2009075284 A1 | 19-03-2009 |
| | | | US | 2014051601 A1 | 20-02-2014 |
| EP 2249161 | A1 | 10-11-2010 | EP | 2249161 A1 | 10-11-2010 |
| | | | EP | 2427773 A1 | 14-03-2012 |
| | | | ES | 2595205 T3 | 28-12-2016 |
| | | | US | 2012136581 A1 | 31-05-2012 |
| | | | US | 2018011111 A1 | 11-01-2018 |
| | | | WO | 2010128054 A1 | 11-11-2010 |
| WO 0070329 | A1 | 23-11-2000 | AU | 775563 B2 | 05-08-2004 |
| | | | AU | 2004210601 A1 | 07-10-2004 |
| | | | CA | 2373314 A1 | 23-11-2000 |
| | | | EP | 1183521 A1 | 06-03-2002 |
| | | | ES | 2459745 T3 | 12-05-2014 |
| | | | JP | 4949560 B2 | 13-06-2012 |
| | | | JP | 2003508729 A | 04-03-2003 |
| | | | WO | 0070329 A1 | 23-11-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4291628 B

**Non-patent literature cited in the description**

- **CORRIGAN J.J.** *Science,* 1969, vol. 164, 142
- **HAMASE K ; MORIKAWA A ; ZAITSU K.** *J Chromatogr.,* 2002, vol. B 781, 73
- **D'ANIELLO A.** *FASEB J,* 2000, vol. 14, 699
- **NAGATA Y.** *FEBS Lett.,* 1999, vol. 444, 160
- **NISHIKAWA T.** *Biol. Pharm. Bull.,* 2005, vol. 28, 1561
- **SASABE, J.** *Proc. Natl. Acad. Sci.,* 2012, vol. 109, 627
- **HAMASE K.** *J. Chromatogr. A,* 2007, vol. 1143, 105
- **HAMASE K.** *J. Chromatogr. A,* 2010, vol. 1217, 1056
- **MIYOSHI Y.** *J. Chromatogr. B,* 2011, vol. 879, 3184
- **ARBER, S.** *Cell,* 1997, vol. 88, 393
- **HSIAO, K.** *Science,* 1996, vol. 274, 99
- **KONNO, R.** *Genetics,* 1983, vol. 103, 277
- **HUANG, A.S.** *J. Neurosci.,* 2006, vol. 26, 2814
- **SHEDLOVSKY, A.** *Genetics,* 1993, vol. 134, 1205
- **HOMANICS GE.** *BMC Med Genet,* 2006, vol. 7, 33